(19) Europäisches Patentamt
European Patent Office
Office européen des brevets

(11) EP 3 853 208 B1

(12) FASCICULE DE BREVET EUROPEEN

(45) Date de publication et mention de la délivrance du brevet:
**07.06.2023 Bulletin 2023/23**

(21) Numéro de dépôt: **19783974.9**

(22) Date de dépôt: **17.09.2019**

(51) Classification Internationale des Brevets (IPC):
***C07D 215/42*** (2006.01) ***A61P 35/00*** (2006.01)
***A61K 31/4706*** (2006.01)

(52) Classification Coopérative des Brevets (CPC):
**C07D 215/42; A61P 35/00**

(86) Numéro de dépôt international:
**PCT/EP2019/074909**

(87) Numéro de publication internationale:
**WO 2020/058290 (26.03.2020 Gazette 2020/13)**

# (54) MÉDICAMENT-CONJUGUÉ COMPRENANT DES DÉRIVÉS DE QUINOLINE

ARZNEIMITTELKONJUGAT MIT CHINOLINDERIVATEN

DRUG CONJUGATE COMPRISING QUINOLINE DERIVATIVES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.09.2018 FR 1858366**

(43) Date de publication de la demande:
**28.07.2021 Bulletin 2021/30**

(73) Titulaires:
- **Centre national de la recherche scientifique 75016 Paris (FR)**
- **Université Paris-Saclay 91190 Gif-sur-Yvette (FR)**

(72) Inventeurs:
- **ALAMI, Mouad 77660 BUSSY SAINT GEORGES (FR)**
- **HAMZE, Abdallah 91300 MASSY (FR)**
- **PROVOT, Olivier 78500 SARTROUVILLE (FR)**
- **KHELIFI, Ilhem 93340 LE RAINCY (FR)**
- **BLANCHARD, Vincent 51490 PONTFAVERGER MORONVILLIERS (FR)**
- **MAKKY-IBRAHIM, Nada 91300 MASSY (FR)**

(74) Mandataire: **Lavoix
2, place d'Estienne d'Orves
75441 Paris Cedex 09 (FR)**

(56) Documents cités:
**EP-A1- 3 129 368**

- **ZHOU YUANYUAN ET AL: "Design, synthesis and biological evaluation of 4-anilinoquinoline derivatives as novel potent tubulin depolymerization agents", EUROPEAN JOURNAL OF MEDICINAL CHEMISTRY, EDITIONS SCIENTIFIQUE ELSEVIER, PARIS, FR, vol. 138, 22 juillet 2017 (2017-07-22), pages 1114-1125, XP085163802, ISSN: 0223-5234, DOI: 10.1016/J.EJMECH.2017.07.040 cité dans la demande**
- **KHELIFI ILHEM ET AL: "Design, synthesis and anticancer properties ofIsoCombretaQuinolines as potent tubulin assembly inhibitors", EUROPEAN JOURNAL OF MEDICINAL CHEMISTRY, EDITIONS SCIENTIFIQUE ELSEVIER, PARIS, FR, vol. 127, 9 novembre 2016 (2016-11-09), pages 1025-1034, XP029907398, ISSN: 0223-5234, DOI: 10.1016/J.EJMECH.2016.11.012 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 853 208 B1

## Description

La présente invention concerne des nouveaux composés à base de combretastatine dérivés de produits naturels utiles comme payloads (appelées aussi charges ou toxines) notamment dans des médicaments-conjugués. La présente invention concerne en outre de nouvelles compositions de combretaquinoléines, y compris utiles comme payloads, liaisons payloads-linker, et médicaments-conjugués, et sur les méthodes utilisant ces payloads, liaisons payloads-linker, et médicaments-conjugués, pour traiter des pathologies telles que par exemple un cancer, une maladie inflammatoire ou infectieuse.

ETAT DE LA TECHNIQUE

Pour de nombreux types de cancer, la chimiothérapie classique reste la seule forme de traitement efficace. La chimiothérapie fonctionne sur la base d'un effet cytotoxique : une toxine tue les cellules cancéreuses, arrêtant ainsi la croissance tumorale. Les agents chimiothérapeutiques endommagent et détruisent principalement les cellules ayant un niveau élevé d'activité de division cellulaire. Cependant, ces traitements doivent être améliorés pour cibler et détruire les cellules malignes tout en minimisant la toxicité collatérale indésirable pour les tissus normaux. En effet, encore aujourd'hui, comme ces médicaments endommagent également les cellules saines, les patients souffrent de graves effets secondaires. De nombreux médicaments hautement cytotoxiques ont une utilité clinique limitée parce qu'ils sont tout aussi agressifs contre les cellules tumorales normales et malignes. Les tissus sains peuvent être fortement affectés par les cytotoxiques. Comme ces médicaments ne font pas spécifiquement la distinction entre les cellules tumorales et normales, entraînant des effets secondaires, les médicaments sont souvent dosés à des niveaux minimaux, ce qui diminue l'efficacité. C'est la raison pour laquelle il est important de trouver un moyen de cibler spécifiquement les tumeurs cellulaires.

L'amélioration de l'administration de médicaments et d'autres agents aux cellules, tissus et tumeurs cibles afin d'obtenir une efficacité maximale et une toxicité minimale a fait l'objet d'une recherche considérable pendant de nombreuses années. Les thérapies anticancéreuses modernes ciblent maintenant le cancer avec plus de précision en utilisant de grosses molécules comme les anticorps. Les anticorps sont une partie importante et naturelle du système immunitaire, de grosses molécules qui peuvent se lier spécifiquement à la surface cellulaire d'un " intrus " (p. ex. un virus) et ainsi l'éliminer. Cependant, souvent non curatifs, les anticorps doivent être associés à des agents chimiothérapeutiques.

Bien que de nombreuses tentatives aient été faites pour mettre au point des méthodes efficaces d'importation de molécules biologiquement actives dans les cellules, tant in vivo qu'in vitro, aucune ne s'est avérée entièrement satisfaisante. Il est souvent difficile ou inefficace d'optimiser l'association du médicament avec sa cible intracellulaire tout en minimisant la redistribution intercellulaire du médicament, p. ex. aux cellules voisines.

Les concepts de conjugués d'anticorps et de drogues (« Antibody Drug Conjugates » dit ADC) cherchent à surmonter ces limites. Ils sont composés de 3 éléments clés : un anticorps (conçu pour cibler sélectivement la tumeur d'intérêt), une charge toxique utile (un composé cytotoxique qui tue la tumeur, appelé « payload » dans le métier) et le lieur (utilisé pour conjuguer la charge utile toxique à l'anticorps). Le principal avantage de ces constructions réside dans l'amélioration significative de la fenêtre thérapeutique : augmentation de la demi-vie et de la spécificité de la charge toxique utile, réduisant les effets et la toxicité hors cible. L'utilisation des ADC a fait l'objet d'études approfondies au cours des trois dernières décennies (Moolten et al. (1972), J Natl Cancer Inst. 49(4):1057-62, Chari et al, (2014) Angew. Chimie. Int. Éd. 53:3796-3827 ; Jackson, (2016) Org. Rés. processus Dev. 2016, 20:852-866). Plusieurs sont déjà approuvés et commercialisés (Kadcyla de Roche et Adcetris de Seattle Genetics).

Les payloads (ou toxines) utilisées dans les ADC comprennent les toxines bactériennes comme la toxine diphtérique (Levy et al. (1975) Cancer Res. 35(5):1182-6), les toxines végétales comme la ricine, les toxines de petites molécules comme les maytansinoids (EP 1391213 ; Liu et al, Proc. 1996). Acad. nat. États-Unis d'Amérique 93:8618-8623), calicéamicine (Lode et al. 1998), Cancer Res. 58:2925-2928 ; Upeslacis et al. 1993 (Cancer Res. 53, 3336-3342), auristatins (Sanderson et al. 2005) Clin. Cancer Res. 11:843-52), SN-38 ou analogues de l'irinotécan (Govidan et al (2013) Mol. Cancer. Ther. 12:968-78, US 2014/0227180A1, Goldenberg et al (2007), Clin. Cancer Res. 13, 5556s-5563s), pyrrolobenzodiazépines (US 2011/0256157A1, Kung Sutherland et al Blood (2013) 122:1455-1463, Chari et al, (2009) Mol. Cancer Ther. 8, B126.) ou de cryptophycines (WO 2011/001052A1, Verma et al (2015) Bioorg. Méd. Chimie. Lett. 25:864-8, US 2012/0225089). Actuellement, plus de 60 % de tous les ADC actuellement en cours d'évaluation clinique contiennent des toxines liées à la monométhyl Auristatine E et F (inhibiteur de tubuline MMAE, MMAF). Le MMAE pourrait être considéré par l'homme du métier comme la charge utile standard (payload standard) de référence pour une comparaison.

La conjugaison de médicaments à des anticorps, soit directement, soit par l'intermédiaire d'agents de liaison (« linker »), implique la prise en compte de divers facteurs, y compris l'identité et l'emplacement du groupe chimique de conjugaison du médicament, le mécanisme de libération du médicament, les éléments structurels qui assurent la libération du médicament et la modification structurelle du médicament sous forme libre. De plus, si le médicament doit être libéré après l'internalisation des anticorps, le mécanisme de libération du médicament doit être compatible avec le trafic intracellulaire du conjugué. Par conséquent, bien qu'un certain nombre de classes de médicaments aient été essayées comme charges utiles, seules quelques classes de médicaments se sont révélées efficaces comme conjugués d'anticorps, en raison de leur efficacité, de leur sélectivité et/ou de leur stabilité limitées (Tolcher et al. (2000) J. Clin. Oncol. 18:4000, Laguzza et al (1989) J. Med. Chimie. 32:548-555, Uadia P, (1984). Cancer Res. 44:4263-4266). Il s'avère que les composés, pour être éligibles en tant que conjugués, doivent présenter un niveau de cytotoxicité inférieur à la CI50 nanomolaire. (Casi et Neri, (2012) J. Control Release. 20;161(2):422-8 ; Wu et Senter (2005) Nat. Biotechnologie. 23(9):1137-46). Les charges utiles idéales devraient échapper au mécanisme de multirésistance (MDR). Dans le cas du MMAE, qui est sujet au MDR, certaines tumeurs peuvent développer un mécanisme de résistance (Chen et al., 2015). Les quelques charges utiles accessibles ne sont pas efficaces contre le large spectre d'indications du cancer. En raison de ces limites, il y a une demande clinique de nouvelles charges utiles ayant un mode d'action différencié, une sélectivité, un profil de toxicité améliorés et non soumises au MDR. Pour ces raisons, les combretastatines ont été considérées comme une potentielle charge toxique utile.

En effet, la Combretastatine a été isolée de l'arbre africain indigène *Combretum caffrum* et similaires dans les années 1980, et son activité inhibitrice de polymérisation de la tubuline a été vérifiée (Pettit GR (1987)[0008]. J Nat Prod 50:119-131). La combretastatine A-4 (ou CA-4) et ses analogues sont cytotoxiques et perturbent sélectivement le système vasculaire tumoral ou préviennent sa néoformation (Dark et al. (1997) Cancer Res 57, 1829- 1834, Grosios K, et al (1999) Br J Cancer 81:1318-1327). Ce stilbène CA-4 structurellement très simple présente plusieurs inconvénients tels qu'une faible solubilité dans l'eau et une instabilité chimique de la double liaison configurée en Z, qui s'isomérise pendant le stockage, l'administration et le métabolisme. Un autre inconvénient du CA-4 est sa cytotoxicité insuffisante. Ces problèmes ont été surmontés par le développement de nouveaux dérivés utilisant des hétérocycles contenant de l'azote comme les quinazolines (voir Figure 1 B) et les quinoléines (voir Figure 1 C & D) (Soussi MA et al (2015) Chem. Med. Chem.10(8):1392-402 ; I. Khelifi, et al, (2017), European Journal of Médicinal Chemistry 127:1025-1034). Avec ces dérivés, le groupe 3,4,5-triméthoxyphényle, considéré comme responsable des effets de neurotoxicité ou de cardiotoxicité, a été supprimé. Mais aucun de ces composés n'a pu être utilisé comme charge utile.

Les combretastatines ont été citées comme étant potentiellement utilisées comme charges utiles dans quelques brevets, mais sans exemplification ni données à l'appui : (EP 1 912 677 B1 "PSMA antibody-drug conjugates") ; WO 2014/164534 ("Site-specific antibody-drug conjugation through glycoengineering"); US 8,535,678 B2 ("Anti-CD70 antibody-drug conjugates and their use for the treatment of cancer and immune disorders"). D'autre part, les combretastatines ont été évaluées comme charges utiles potentielles dans le cadre de quelques études, mais sans aucune application clinique jusqu'à présent en raison de leur activité limitée, de problèmes de stabilité et de profils de métabolisation défavorables reconnus par les auteurs eux-mêmes (Toki et al (2002) J. Org. Chimie. 67, 1866-1872, Bolu et al (2016) Mol. Pharmaceutics, 13, 1482-1490, R. Nani et al (2015) Angew. Chim. Int. Ed. Engl. 9 ; 54(46) : 13635-13638). Le seul exemple d'utilisation de dérivés de la combretastatine comme charges utiles provient de la demande de brevet décrivant les nouvelles isoNH2CombretaQuinolines (PCT/EP2018/058168). Cependant, l'ADC Trastuzumab-VC-PAB-ICQO-1 résultant a donné une CI50 de 27 μg/mL contre la lignée cellulaire SKBR3. Un tel niveau de cytotoxicité est inférieur de 3 logs au ng/mL qui est attendu d'un ADC et n'est pas suffisant pour une utilisation thérapeutique. En d'autres termes, bien que ces dérivés aient montré des propriétés prometteuses en tant que dérivés autonomes, ils semblent perdre leur cytotoxicité une fois conjugués. De plus, le processus de conjugaison de ces dérivés s'est révélé difficile et pourrait faire l'objet d'améliorations innovantes.

En conclusion, la plupart des tentatives de conjugaison ont échoué jusqu'à présent, même avec les nouveaux dérivés de l'isocombretaquinoléine.

L'invention a donc pour but de résoudre un ou plusieurs des problèmes techniques évoqués ci-dessus.

En particulier, la présente invention a pour but de fournir un composés utile comme payload (charge toxique ou toxine) présentant une toxicité, une stabilité et un profil de métabolisation, éligible en tant que payload, notamment utilisable dans un médicament-conjugué.

L'invention a pour but de fournir un nouveau payload présentant un mode d'action différencié, une sélectivité et un profil de toxicité amélioré, et non soumis au phénomène de multi-résistance aux médicaments.

Après de longues recherches et de manière surprenante, les présents inventeurs ont découverts des nouveaux composés possédant une structure permettant leur utilisation dans un conjugué anticorps-médicament.

Ainsi, selon un premier aspect, la présente invention concerne des composés de formule (I) :

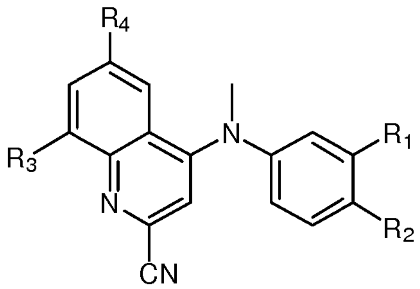

(I)

dans laquelle :

- $R_1$ représente un groupe $-NH_2$, $-NHCOR_a$, $-NHCOOR_b$, $-(C_2-C_6)$alcénylène-CO-NH-OH, $-(C_2-C_6)$alcynylène-CO-NH-OH ou -OH ;
- $R_2$ représente un groupe $-OCH_3$, $-OCH_2CH_3$, $-SCH_3$, $-SCH_2CH_3$ ou $-OCHF_2$ ;
- $R_3$ représente un atome d'hydrogène ou un groupe $-CH_3$, -CN, -F, -Cl ou $-OR_c$ ;
- $R_4$ représente un atome d'hydrogène ou un groupe $-CH_3$, -CN, -F, -Cl ou $-OR_d$ ;
- $R_a$ représente un groupe $-(C_1-C_5)$alkyle ou $-CF_3$ ;
- $R_b$ représente un groupe $-(C_1-C_5)$alkyle ou $-CF_3$ ;
- $R_c$ représente un groupe $-(C_1-C_5)$alkyle ou $-CF_3$ ; et
- $R_d$ représente un groupe $-(C_1-C_5)$alkyle ou $-CF_3$ ;

à l'état de base ou d'acide ou de sels d'acides ou de bases ou sous forme d'hydrate ou de solvate.

Les inventeurs ont découvert que ces composés présentent une efficacité améliorée contre le cancer, en particulier contre les cellules tumorales résistantes. De plus, ils possèdent une stabilité chimique élevée et ne sont sujets à aucune isomérisation donc n'ont aucune tendance à l'inactivation.

Selon un deuxième aspect, la présente invention concerne des composés de formule (II) :

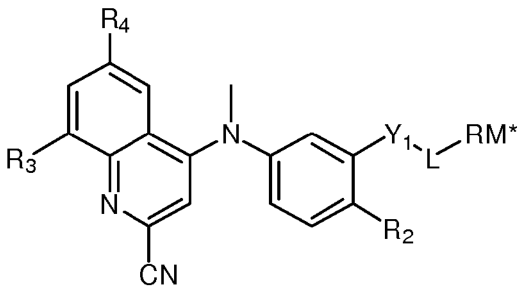

(II)

dans laquelle :

- $Y_1$ représente -O-, -NH-, -NHCO-, $-NHCOR_a-$, -NHCOO-, $-NHCOOR_b-$, $-(C_2-C_6)$alcénylène-CO-NH-O- ou $-(C_2-C_6)$alcynylène-CO-NH-O- ;
- $R_2$ représente un groupe $-OCH_3$, $-OCH_2CH_3$, $-SCH_3$, $-SCH_2CH_3$ ou $-OCHF_2$ ;
- $R_3$ représente un atome d'hydrogène ou un groupe $-CH_3$, -CN, -F, -Cl ou $-OR_c$ ; et de préférence un atome d'hydrogène ;
- $R_4$ représente un atome d'hydrogène ou un groupe $-CH_3$, -CN, -F, -Cl ou $-OR_d$ ; et de préférence un atome d'hydrogène ;
- $R_a$ représente un groupe $-(C_1-C_5)$alkylène- ou $-CF_2-$;
- $R_b$ représente un groupe $-(C_1-C_5)$alkylène- ou $-CF_2-$;
- $R_c$ représente un groupe $-(C_1-C_5)$alkyle ou $-CF_3$ ;
- $R_d$ représente un groupe $-(C_1-C_5)$alkyle ou $-CF_3$ ;
- L représente un agent de liaison ;
- RM* est choisi parmi RM et RM', dans lequel RM est une fonction réactive capable de former une liaison covalente avec une fraction d'un agent de ciblage, en particulier avec une fraction d'un anticorps ou un de ses fragments fonctionnels, et dans lequel RM' est une fraction de RM portant au moins un groupe protecteur ;

à l'état de base ou de sels de bases ou sous forme d'hydrate ou de solvate.

Selon un troisième aspect, la présente invention concerne des conjugués anticorps-médicament de formule (III) :

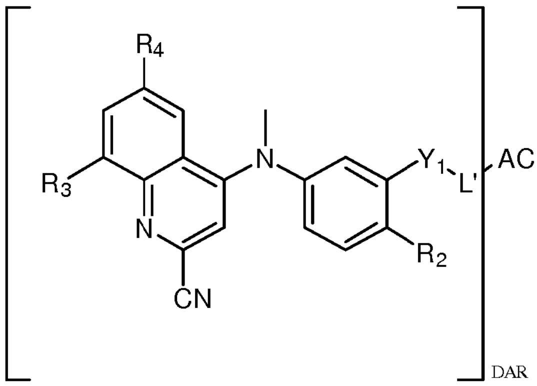

(III)

dans laquelle :

- Yi représente -O-, -NH-, -NHCO-, -NHCOR$_a$-, -NHCOO-, -NHCOOR$_b$-, -($C_2$-$C_6$)alcénylène-CO-NH-O- ou -($C_2$-$C_6$)alcynylène-CO-NH-O- ;
- $R_2$ représente un groupe -$OCH_3$, -$OCH_2CH_3$, -$SCH_3$, -$SCH_2CH_3$ ou -$OCHF_2$ ;
- $R_3$ représente un atome d'hydrogène ou un groupe -$CH_3$, -CN, -F, -Cl ou -OR$_c$ ; et de préférence un atome d'hydrogène ;
- $R_4$ représente un atome d'hydrogène ou un groupe -$CH_3$, -CN, -F, -Cl ou -OR$_d$ ; et de préférence un atome d'hydrogène ;
- $R_a$ représente un groupe -($C_1$-$C_5$)alkylène- ou -$CF_2$-;
- $R_b$ représente un groupe -($C_1$-$C_5$)alkylène- ou -$CF_2$-;
- $R_c$ représente un groupe -($C_1$-$C_5$)alkyle ou -$CF_3$ ;
- $R_d$ représente un groupe -($C_1$-$C_5$)alkyle ou -$CF_3$ ;
- L' représente un agent de liaison ;
- AC représente une fraction d'un agent de ciblage, en particulier une fraction d'un anticorps ou un de ses fragments fonctionnels ; et

dans laquelle le DAR (ratio médicament/agent de ciblage) varie entre 1 et 8, et de préférence entre 2 et 4.

Comme illustré dans la partie expérimentale, et de manière inattendue, les inventeurs ont montré que de tels composés sont très efficaces, comparés aux payloads connus, et possèdent de très bonnes activités inhibitrices à l'encontre de nombreuses cellules cancéreuses.

En particulier des composés de formule (I) sont libérés à partir des composés de formule (III) in vivo, de préférence au niveau du site cible.

D'autres caractéristiques, aspects et avantages de l'invention apparaîtront à la lecture de la description détaillée qui va suivre.

## DEFINITIONS

Dans le cadre de la présente invention, les définitions suivantes sont utilisées :

- « $C_t$-$C_z$ » désigne une chaîne carbonée pouvant avoir de t à z atomes de carbone où t et z peuvent prendre par exemple les valeurs de 1 à 25 ; par exemple $C_1$-$C_3$ est une chaîne carbonée qui peut avoir de 1 à 3 atomes de carbone.
- Un alkyle désigne un groupe aliphatique saturé monovalent linéaire ou ramifié, pouvant par exemple comprendre de 1 à 25 atomes de carbone, et de préférence de 1 à 5 atomes de carbone. A titre d'exemples, on peut citer les groupes méthyle, éthyle, propyle, isopropyle, butyle, isobutyle, tertbutyle, etc.
- Un alkylène désigne un groupe alkyle divalent saturé linéaire ou ramifié pouvant par exemple comprendre de 1 à 25 atomes de carbone.
- Un alcénylène désigne un groupe hydrocarboné aliphatique comprenant une double liaison carbone-carbone, linéaire ou ramifié, les autres liaisons pouvant être des liaisons simples ou d'autres liaisons doubles, et pouvant par exemple comprendre de 1 à 25 atomes de carbone, et de préférence de 2 à 6 atomes de carbone. A titre d'exemples,

on peut citer les groupes éthénylène (-CH=CH-), 1-propénylène, 2-propénylène, 1-buténylène, 2-buténylène, 3-buténylène, etc.

- Un alcynylène désigne un groupe hydrocarboné aliphatique comprenant une triple liaison carbone-carbone, linéaire ou ramifié, les autres liaisons pouvant être des liaisons simples, des liaisons doubles ou d'autres liaisons triples, et pouvant par exemple comprendre de 1 à 25 atomes de carbone, et de préférence de 2 à 6 atomes de carbone. A titre d'exemples, on peut citer les groupes éthynylène, 1-propynylène, 2-propynylène, etc.
- Un groupe $-CF_2-$ désigne un groupe alkyle divalent dont les atomes d'hydrogène ont été substitués par deux atomes de fluor.
- Un cycloalkylène désigne un cycle non aromatique bivalent, monocyclique ou polycyclique, pouvant comprendre de 3 à 12 atomes de carbones. A titre d'exemples, on peut citer les groupes cyclopropylène, cyclobutylène, cyclopentylène, cyclohexylène, cycloheptylène, etc.
- Un cycloalcénylène désigne un cycle non aromatique bivalent, monocyclique ou polycyclique, pouvant comprendre de 3 à 12 atomes de carbones, et comprenant une ou plusieurs insaturations. A titre d'exemples, on peut citer les cyclopenténylène et cyclohéxenylène.
- Un hétérocycloalkylène désigne un cycle non aromatique bivalent, monocyclique ou polycyclique, pouvant comprendre de 3 à 12 atomes de carbones et au moins un hétéroatome choisi parmi O, N ou S.
- Un hétérocycloalcénylène désigne un cycle non aromatique bivalent, monocyclique ou polycyclique, pouvant comprendre de 3 à 12 atomes de carbones et au moins un hétéroatome choisi parmi O, N ou S, et comprenant une ou plusieurs insaturations.
- Un arylène désigne un cycle bivalent, totalement ou partiellement aromatique, pouvant comprendre de 3 à 20 atomes de carbones, par exemple le phenylène.
- Un hétéroarylène désigne un cycle bivalent, totalement ou partiellement aromatique, pouvant comprendre de 3 à 20 atomes de carbones, et au moins un hétéroatome choisi parmi O, N ou S.
- Selon l'invention, le terme « substitué » désigne le fait qu'un ou plusieurs hydrogène est remplacé par un ou plusieurs groupes, par exemple choisis parmi les groupes alkyle, alcényle, alcynyle, cycloalkyle, hétérocycloalkyle, aryle, hétéroaryle, aralkyle, hétéroaralkyle, acyle, aroyle, hétéroaroyle, carboxyle, alkoxy, aryloxy, acyloxy, aroyloxy, hétéroaroyloxy, alkoxycarbonyle, halogène, (thio)ester, cyano, phosphoryle, amino, imino, (thio)amido, sulfhydryle, alkylthio, acylthio, sulfonyl, sulfate, sulfonate, sulfamoyle, sulfonamido, nitro, azido, haloalkyle, notamment perfluoroalkyle (tel que trifluorométhyle), haloalkoxy, alkylsulfanyl, alkylsulfinyl, alkylsulfonyl, alkylsulfonylamino, arylsulfonoamino, phosphate, phosphonate, phosphinate, alkylcarboxy, alkylcarboxyamide, oxo, hydroxy, mercapto, amino (éventuellement mono- ou di-substitué, par exemple par un alkyle, aryle, ou hétéroaryle), imino, carboxamide, carbamoyle (éventuellement mono- ou di-substitué, par exemple par un alkyle, aryle, ou hétéroaryle), amidino, aminosulfonyl, acylamino, aroylamino, (thio)uréido, (arylthio)uréido, alkyl(thio)uréido, cycloalkyl(thio)uréido, aryloxy, aralkoxy, ou $-O(CH_2)_n-OH$, $-O(CH_2)_n-NH_2$, $-O(CH_2)_nCOOH$, $-(CH_2)_nCOOH$, $-C(O)O(CH_2)_nR$, $-(CH_2)_nN(H)C(O)OR$, ou $-N(R)S(O)_2R$, dans lesquels n est un nombre entre 1 et 4 et R est indépendamment choisi par un hydrogène ou un groupe choisi parmi -alkyle, -alcényle, -alcynyle, -cycloalkyle, -cycloalcényl, -hétérocycloalkyle, -hétérocycloalcényle, -aryle et -hétéroaryle, le cas échéant le ou les groupes pouvant être eux-mêmes substitués.
- On entend par « une fonction réactive », une fonction capable de former une liaison covalente avec un autre composé.
- Une fraction désigne une partie d'un composé.
- On entend par « fragment fonctionnel », une partie d'un composé comprenant une fonction réactive, éventuellement protégé.
- On entend par « groupe protecteur », un groupe fonctionnel introduit dans la molécule à partir d'une fonction chimique pour masquer tout ou partie de sa réactivité. Par exemple, il peut s'agir d'un imide, amide, carbamate, imine, énamine, dérivé sulfonylé, dérivé N-sulfénylé, N-alkylé ou N-silylé, diol, dérivé carbonylé, acétal, éther, éther benzylique, éther silylé, ester, ect.
- L'expression « pharmaceutiquement acceptable » désigne ce qui est utile dans la préparation d'une composition pharmaceutique, qui est généralement sûr, non toxique et ni biologiquement ni autrement non souhaitable et qui est acceptable pour une utilisation vétérinaire et/ou pharmaceutique humaine.
- On entend par « sels d'acides ou de bases », des acides ou bases salifiés, par exemple un sel d'addition d'acide peut être formé avec des acides inorganiques tels que l'acide chlorhydrique, sulfurique ou phosphorique, ou avec des acides organiques tels que l'acide acétique ou l'acide benzoïque. Les sels de bases peuvent être formés avec une base inorganique telle que l'hydroxyde d'aluminium, l'hydroxyde de calcium, l'hydroxyde de potassium, le carbonate de sodium et l'hydroxyde de sodium, ou une base organique comme la diéthanolamine, l'éthanolamine, le N-méthylglucamine, la triéthanolamine ou la trométhamine.
- On entend par « hydrate ou solvate » des formes d'associations ou de combinaisons avec une ou plusieurs molécules d'eau ou avec un solvant.

**COMPOSES DE FORMULE (I)**

Comme indiqué précédemment, la présente invention a pour objet des composés de formule (I) :

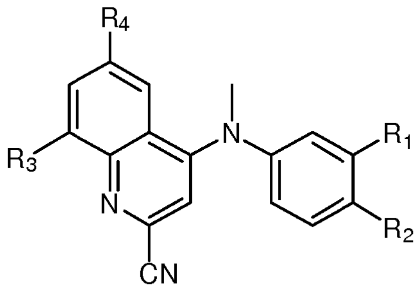

(I)

dans laquelle :

- $R_1$ représente un groupe $-NH_2$, $-NHCOR_a$, $-NHCOOR_b$, $-(C_2-C_6)$alcénylène-CO-NH-OH, $-(C_2-C_6)$alcynylène-CO-NH-OH ou -OH ;
- $R_2$ représente un groupe $-OCH_3$, $-OCH_2CH_3$, $-SCH_3$, $-SCH_2CH_3$ ou $-OCHF_2$ ;
- $R_3$ représente un atome d'hydrogène ou un groupe $-CH_3$, -CN, -F, -Cl ou $-OR_c$ ;
- $R_4$ représente un atome d'hydrogène ou un groupe $-CH_3$, -CN, -F, -Cl ou $-OR_d$ ;
- $R_a$ représente un groupe $-(C_1-C_5)$alkyle ou $-CF_3$ ;
- $R_b$ représente un groupe $-(C_1-C_5)$alkyle ou $-CF_3$ ;
- $R_c$ représente un groupe $-(C_1-C_5)$alkyle ou $-CF_3$ ; et
- $R_d$ représente un groupe $-(C_1-C_5)$alkyle ou $-CF_3$ ;

à l'état de base ou d'acide ou de sels d'acides ou de bases ou sous forme d'hydrate ou de solvate.

De préférence, $R_3$ représente un atome d'hydrogène.

De préférence, $R_4$ représente un atome d'hydrogène.

Selon un mode préféré, le composé de formule (I) est de formule (Ia) :

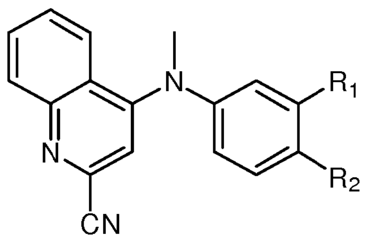

(Ia)

dans laquelle :

- $R_1$ représente un groupe $-NH_2$, $-NHCOR_a$, $-NHCOOR_b$, $-(C_2-C_6)$alcénylène-CO-NH-OH, $-(C_2-C_6)$alcynylène-CO-NH-OH ou -OH ;
- $R_2$ représente un groupe $-OCH_3$, $-OCH_2CH_3$, $-SCH_3$, $-SCH_2CH_3$ ou $-OCHF_2$ ;
- $R_a$ représente un groupe $-(C_1-C_5)$alkyle ou $-CF_3$ ; et
- $R_b$ représente un groupe $-(C_1-C_5)$alkyle ou $-CF_3$ ;

à l'état de base ou d'acide ou de sels d'acides ou de bases ou sous forme d'hydrate ou de solvate.

De préférence, $R_2$ représente un groupe $-OCH_3$, $-OCH_2CH_3$, ou $-OCHF_2$, et de préférence un groupe $-OCH_3$.

De préférence, $R_1$ représente un groupe $-NH_2$, $-NHCOR_a$, $-NHCOOR_b$, ou -OH, en particulier $R_1$ représente un groupe $-NH_2$ ou -OH, et de préférence un groupe $-NH_2$.

Selon un mode encore plus préféré, le composé est choisi parmi :

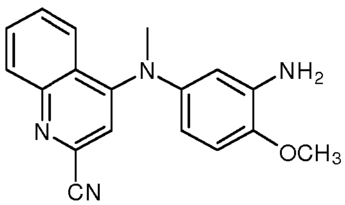
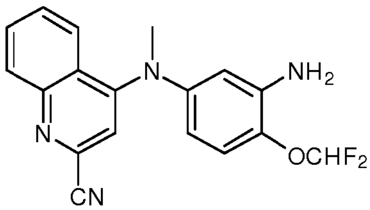
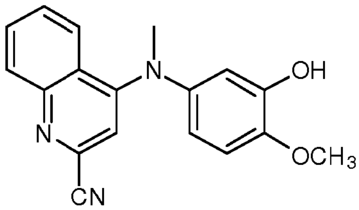

et

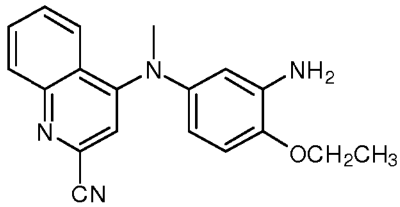

Selon un mode préféré, le composé de formule (I) est de formule (Ib) :

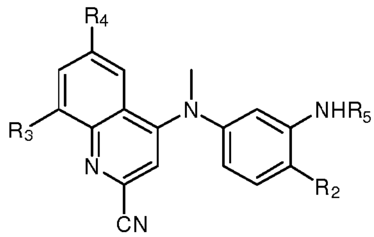

(Ib)

dans laquelle :

- $R_5$ représente un atome d'hydrogène, un groupe $-COR_a$, ou $-COOR_b$ ;
- $R_2$ représente un groupe $-OCH_3$, $-OCH_2CH_3$, $-SCH_3$, $-SCH_2CH_3$ ou $-OCHF_2$ ;
- $R_3$ représente un atome d'hydrogène ou un groupe $-CH_3$, -CN, -F, -Cl ou $-OR_c$, et de préférence un atome d'hydrogène ;
- $R_4$ représente un atome d'hydrogène ou un groupe $-CH_3$, -CN, -F, -Cl ou $-OR_d$, et de préférence un atome d'hydrogène ;
- $R_a$ représente un groupe $-(C_1-C_5)$alkyle ou $-CF_3$ ;
- $R_b$ représente un groupe $-(C_1-C_5)$alkyle ou $-CF_3$ ;
- $R_c$ représente un groupe $-(C_1-C_5)$alkyle ou $-CF_3$ ; et
- $R_d$ représente un groupe $-(C_1-C_5)$alkyle ou $-CF_3$ ;

à l'état de base ou d'acide ou de sels d'acides ou de bases ou sous forme d'hydrate ou de solvate.

De préférence, $R_1$ représente un groupe $-(C_2-C_6)$alcénylène-CO-NH-OH ou $-(C_2-C_6)$alcynylène-CO-NH-OH, et de préférence $R_1$ représente un groupe -CH=CH-CO-NH-OH, $-CH=CH-CH_2-CH_2-$CO-NH-OH ou $-C=C-CH_2-CH_2-$CO-NH-OH.

Selon un mode préféré, le composé de formule (I) est de formule (Ic) :

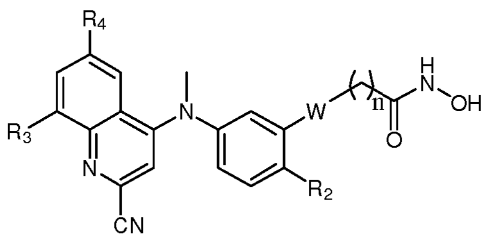

(Ic)

dans laquelle :

- W représente -CH=CH- ou -C=C- ;

- n est 0, 1, 2, 3 ou 4, et de préférence n est 0 ou 2 ;
- $R_2$ représente un groupe $-OCH_3$, $-OCH_2CH_3$, $-SCH_3$, $-SCH_2CH_3$ ou $-OCHF_2$ ;
- $R_3$ représente un atome d'hydrogène ou un groupe $-CH_3$, -CN, -F, -Cl ou $-OR_C$, et de préférence un atome d'hydrogène ;
- $R_4$ représente un atome d'hydrogène ou un groupe $-CH_3$, -CN, -F, -Cl ou $-OR_d$, et de préférence un atome d'hydrogène ;
- $R_c$ représente un groupe $-(C_1-C_5)$alkyle ; et
- $R_d$ représente un groupe $-(C_1-C_5)$alkyle ou $-CF_3$ ;

à l'état de base ou d'acide ou de sels de bases ou sous forme d'hydrate ou de solvate.

Selon un mode encore plus préféré, le composé est choisi parmi :

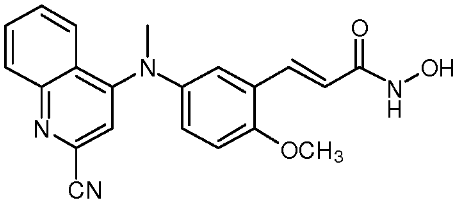

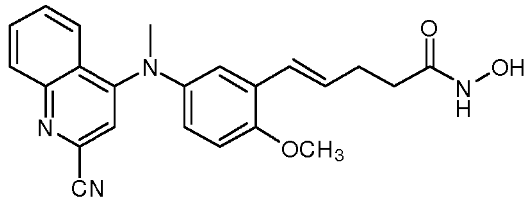

et

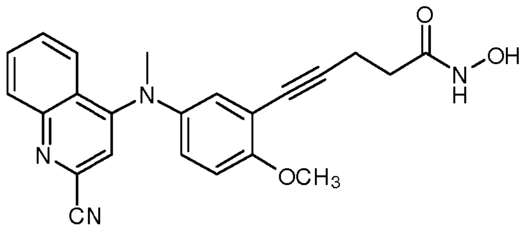

## PROCEDE DE PREPARATION DES COMPOSES DE FORMULE (I)

La présente invention concerne un procédé de préparation d'un composé de formule (I) selon l'invention, dans lequel $R_1$ représente un groupe $-NH_2$ ou -OH, caractérisé en ce que :

- on soumet un composé de formule (A) :

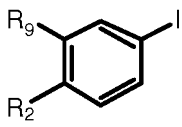

(A)

dans lequel $R_2$ est tel que défini dans la formule (I) décrite précédemment et $R_9$ représente un groupe $-NO_2$ ou -O-Benzoyle, à une réaction d'amination pour former un composé de formule (B) :

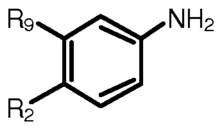

(B)

dans lequel $R_2$ est tel que défini dans la formule (I) décrite précédemment et $R_9$ représente un groupe $-NO_2$ ou -O-Benzoyle ;

- on soumet un composé de formule (C) :

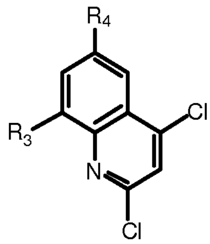

(C)

dans lequel $R_3$ et $R_4$ sont tels que définis dans la formule (I) décrite précédemment, à une réaction de cyanuration pour former un composé de formule (D) :

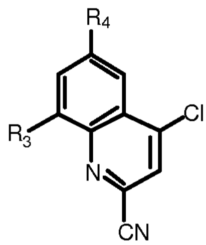

(D)

dans lequel $R_3$ et $R_4$ sont tels que définis dans la formule (I) décrite précédemment ;

- on met en présence le composé de formule (B) avec le composé de formule (D), pour former par une réaction de substitution nucléophile aromatique le composé de formule (E) :

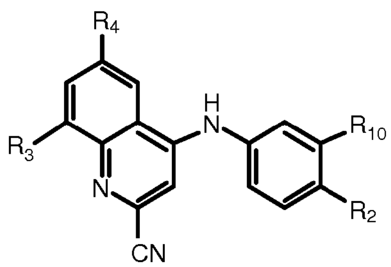

(E)

dans lequel $R_3$, $R_4$ et $R_2$ sont tels que définis dans la formule (I) décrite précédemment, et $R_{10}$ représente un groupe $-NO_2$ ou -O-Benzyle ;

- on soumet le composé de formule (E), à une réaction de méthylation puis à une réaction de réduction ou de déprotection pour former le composé de formule (I) :

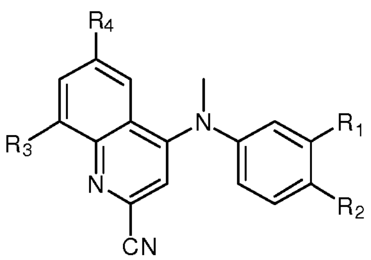

(I)

dans lequel $R_3$, $R_4$ et $R_2$ sont tels que définis dans la formule (I) décrite précédemment, et $R_1$ représente un groupe $-NH_2$ ou -OH, $R_a$ et $R_b$ étant tels que définis dans la formule (I) décrite précédemment.

La présente invention concerne également un procédé de préparation d'un composé de formule (I) selon l'invention, dans lequel $R_1$ représente un groupe $-NH_2$, caractérisé en ce que :

- on soumet un composé de formule (F) :

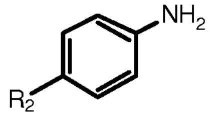

(F)

dans lequel $R_2$ est tel que défini dans la formule (I) décrite précédemment, à une réaction de nitration pour former un composé de formule (G) :

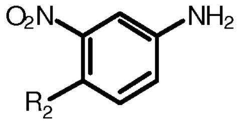

(G)

dans lequel $R_2$ est tel que défini dans la formule (I) décrite précédemment ;

- on soumet un composé de formule (H) :

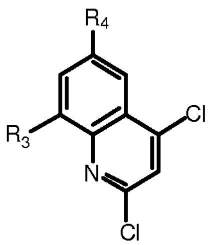

(H)

dans lequel $R_3$ et $R_4$ sont tels que définis dans la formule (I) décrite précédemment, à une réaction de cyanuration pour former un composé de formule (D) :

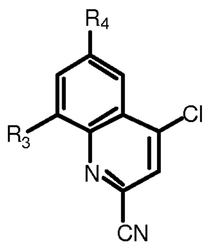

(D)

dans lequel $R_3$ et $R_4$ sont tels que définis dans la formule (I) décrite précédemment ;

- on met en présence le composé de formule (G) avec le composé de formule (J), pour former, par une réaction de couplage en présence de palladium, le composé de formule (K) :

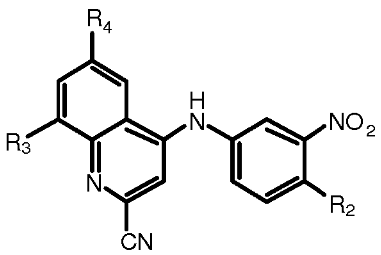

(K)

dans lequel $R_3$, $R_4$ et $R_2$ sont tels que définis dans la formule (I) décrite précédemment ;

- on soumet le composé de formule (K), à une réaction de méthylation puis à une réaction de réduction pour former le composé de formule (L) :

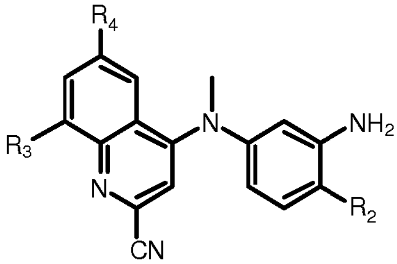
(L)

dans lequel $R_3$, $R_4$ et $R_2$ sont tels que définis dans la formule (I) décrite précédemment.

La présente invention concerne également un procédé de préparation d'un composé selon l'invention, dans lequel $R_1$ représente un groupe -$C_2$-$C_6$-alcénylène-CO-NH-OH ou -$C_2$-$C_6$-alcynylène-CO-NH-OH, caractérisé en ce que :

- on soumet un composé de formule (M) :

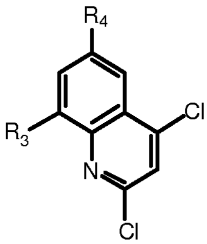
(M)

dans lequel $R_3$ et $R_4$ sont tels que définis dans la formule (I) décrite précédemment, à une réaction de cyanuration pour former un composé de formule (N) :

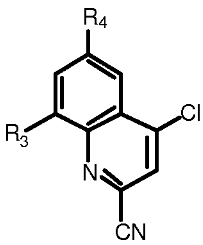
(N)

dans lequel $R_3$ et $R_4$ sont tels que définis dans la formule (I) décrite précédemment ;

- on met en présence le composé de formule (N) avec un composé de formule (O) :

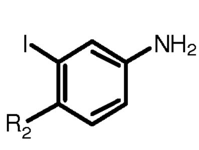
(O)

dans lequel $R_2$ est tel que défini dans la formule (I) décrite précédemment, pour former par une réaction de substitution nucléophile aromatique, suivie d'une réaction de méthylation, le composé de formule (P) :

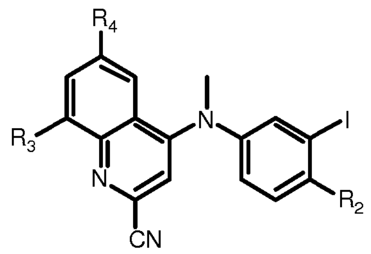

(P)

dans lequel $R_3$, $R_4$ et $R_2$ sont tels que définis dans la formule (I) décrite précédemment ;

- on soumet le composé de formule (P), à une réaction de couplage organométallique avec un groupe $-C_2-C_6$-alcénylène-CO-NH-O-(2-tetrahydropyranyle) ou $-C_2-C_6$-alcynylène-CO-NH-O-(2-tetrahydropyranyle) puis à une réaction de déprotection pour former le composé de formule (I) :

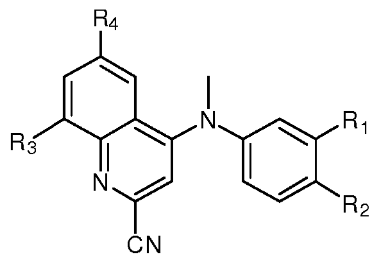

(I)

dans lequel $R_3$, $R_4$ et $R_2$ sont tels que définis dans la formule (I) décrite précédemment, et $R_1$ représente un groupe $-C_2-C_6$-alcénylène-CO-NH-OH ou $-C_2-C_6$-alcynylène-CO-NH-OH.

Selon un mode préféré, la présente invention concerne un procédé de préparation d'un composé selon l'invention, dans lequel $R_1$ représente un groupe $-NH_2$ ou -OH, caractérisé en ce que :

- on met en présence un composé de formule (B) :

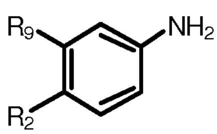

(B)

dans lequel $R_2$ est tel que défini dans la formule (I) décrite précédemment et $R_9$ représente un groupe $-NO_2$ ou -O-Benzoyle ; avec un composé de formule (D) :

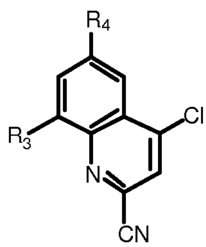

(D)

dans lequel $R_3$ et $R_4$ sont tels que définis dans la formule (I) décrite précédemment ;
pour former par une réaction de substitution nucléophile aromatique, ou par une réaction de couplage en présence d'un catalyseur, en particulier à base de palladium, le composé de formule (E) :

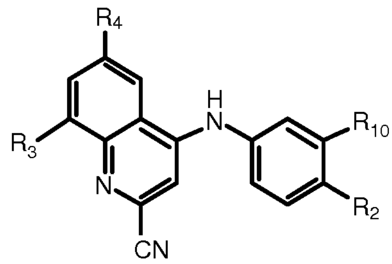

(E)

dans lequel $R_3$, $R_4$ et $R_2$ sont tels que définis dans la formule (I) décrite précédemment, et $R_{10}$ représente un groupe $-NO_2$ ou -O-Benzyle ;

- on soumet le composé de formule (E), à une réaction de méthylation puis à une réaction de réduction ou de déprotection pour former le composé de formule (I) :

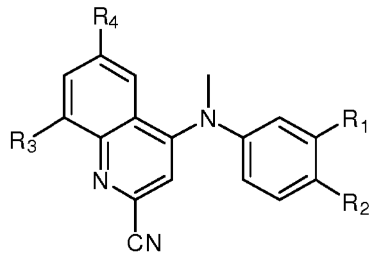

(I)

dans lequel $R_3$, $R_4$ et $R_2$ sont tels que définis dans la formule (I) décrite précédemment, et $R_1$ représente un groupe $-NH_2$ ou -OH, $R_a$ et $R_b$ étant tels que définis dans la formule (I) décrite précédemment.

Selon un mode préféré, la présente invention concerne un procédé de préparation d'un composé (Ic), caractérisé en ce que :

- on met en présence le composé de formule (N) :

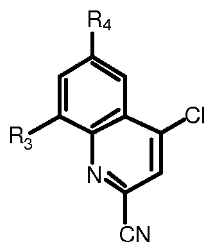

(N)

dans lequel $R_3$ et $R_4$ sont tels que définis dans la formule (Ic) décrite précédemment ; avec un composé de formule (O) :

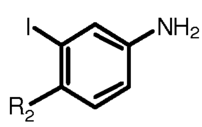

(O)

dans lequel $R_2$ est tel que défini dans la formule (Ic) décrite précédemment, pour former par une réaction de substitution nucléophile aromatique, suivie d'une réaction de méthylation, le composé de formule (P) :

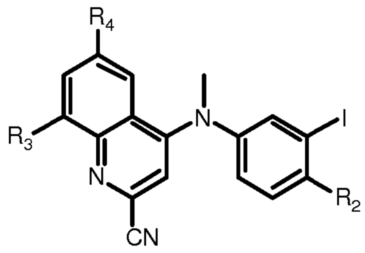

(P)

dans lequel $R_3$, $R_4$ et $R_2$ sont tels que définis dans la formule (Ic) décrite précédemment ;

- on soumet le composé de formule (P), à une réaction de couplage organométallique avec un groupe -($C_2$-$C_6$)alcénylène-CO-NH-O-(2-tetrahydropyranyle) ou -($C_2$-$C_6$)alcynylène-CO-NH-O-(2-tetrahydropyranyle) puis à une réaction de déprotection pour former le composé de formule (Ic) :

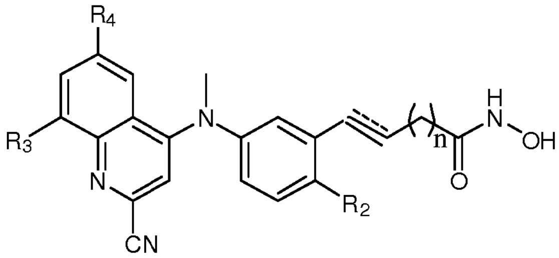

(Ic)

dans laquelle , n, $R_3$, $R_4$ et $R_2$ sont tels que définis dans la formule (Ic) décrite précédemment.

De préférence, les couplages réalisés par substitution nucléophile aromatique, sont effectués en milieu acide, en particulier en présence d'acide chlorhydrique.

En particulier, ils sont réalisés dans un solvant polaire aprotique, plus particulièrement dans du dioxane.

Avantageusement, les couplages sont réalisés à une température comprise entre 120 °C et 160 °C, notamment entre 130 °C et 150 °C, en particulier à reflux du solvant.

Avantageusement, les étapes de couplages sont réalisées pendant une durée comprise entre 10 heures et 14 heures, en particulier entre 11 heures et 13 heures, de préférence pendant 12 heures.

En particulier, les réactions de couplage peuvent être réalisées en présence d'un agent de couplage, tel que le diisopropylcarbodiimide (DIC), le dicyclohexylcarbodiimide (DCC), le chlorhydrate de 1-(3-diméthylaminopropyl)-3-éthylcarbodiimide (EDC), le carbonyldiimidazole (CDI), le 2-(1H-benzotriazole-1-yl)-2,1,3,3-tetraméthyluronium hexafluorophosphate (HBTU), le 2-(1H-benzotriazole-1-yl)-1,1,3,3-tetraméthyluronium tetrafluoroborate (TBTU), le O-(7-azov=benzotriazol-1-yl)-1,1,3,3-tetraméthyluronium hexafluorophosphate (HAUT), le (benzotriazol-1-yloxy)tripyrrolodinophosphonium hexafluorophosphate (PyBOP) ou l'anhydride propylphosphonique, éventuellement associé à un auxiliaire de couplage tel que le N-hydroxy succinimide (NHS), le N-hydroxydbenzotriazole (HOBt), le 3,4-dihydro-3-hydroxy-4-oxo-1,2,3-benzotriazole (HOBt), le I-hydroxy-7-azabenzotriazole (HOAt), le N-hydroxysylfosuccinimide (sulfo NHS), la diméthylaminopyridine (DMAP), la diisopropyléthylamine (DIEA) ou la N-méthylmorpholine (NMM).

Les étapes d'alkylation, de préférence de méthylation, en particulier d'une amine, sont des réactions bien connues de l'homme du métier qui possède toutes les connaissances requises à leur exécution. Par exemple, elles peuvent être réalisées par réaction de l'amine avec un halogénure de -($C_1$-$C_5$)alkyle en présence d'une base, en particulier un carbonate. L'halogénure de -($C_1$-$C_5$)alkyle peut être de préférence un iodure de -($C_1$-$C_5$)alkyle, un bromure de -($C_1$-$C_5$)alkyle ou un chlorure de -($C_1$-$C_5$)alkyle. Plus particulièrement, les étapes d'alkylation sont réalisées dans un solvant polaire aprotique, plus préférentiellement dans du diméthylformamide. De préférence, les étapes d'alkylation sont effectuées à température ambiante.

Les réactions d'amination, de cyanuration, de réduction ou de déprotection, ou encore de nitration sont des réactions bien connues de l'homme du métier qui possède toutes les connaissances requises à leur exécution.

En particulier, l'étape d'amination peut être réalisée à une température comprise entre 60 °C et 100 °C, notamment entre 70 °C et 90 °C, en particulier en présence de fer solide et d'acide chlorhydrique.

Les procédés de préparation selon l'invention peuvent en outre éventuellement comprendre une étape de salification du composé formé, pour donner un sel pharmaceutiquement acceptable, notamment en présence d'un acide ou d'une base pharmaceutiquement acceptable.

La présente invention concerne également des composés intermédiaires, choisis parmi :

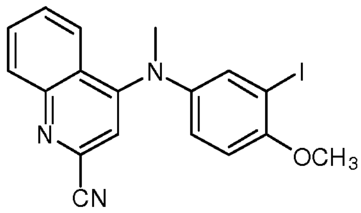

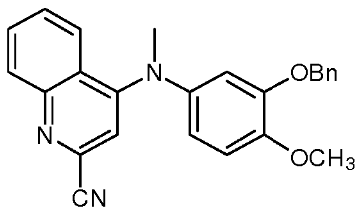

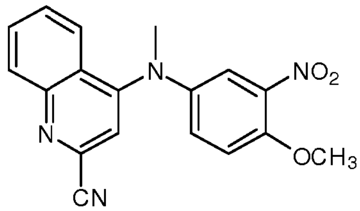

et

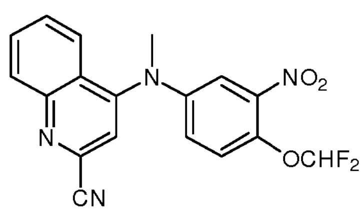

La présente invention concerne également l'utilisation de tels composés intermédiaires, pour la préparation de composés de formule (I) ci-dessus.

**COMPOSES DE FORMULE (II)**

Selon un autre de ses aspects, la présente invention concerne des composés de formule (II) :

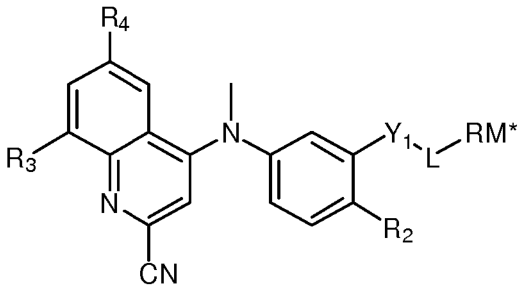

(II)

dans laquelle :

- Yi représente -O-, -NH-, -NHCO-, $-NHCOR_a-$, -NHCOO-, $-NHCOOR_b-$, $-(C_2-C_6)$alcénylène-CO-NH-O- ou $-(C_2-C_6)$alcynylène-CO-NH-O- ;
- $R_2$ représente un groupe $-OCH_3$, $-OCH_2CH_3$, $-SCH_3$, $-SCH_2CH_3$ ou $-OCHF_2$ ;
- $R_3$ représente un atome d'hydrogène ou un groupe $-CH_3$, -CN, -F, -Cl ou $-OR_c$ ; et de préférence un atome d'hydrogène ;
- $R_4$ représente un atome d'hydrogène ou un groupe $-CH_3$, -CN, -F, -Cl ou $-OR_d$ ; et de préférence un atome d'hydrogène ;
- $R_a$ représente un groupe $-(C_1-C_5)$alkylène- ou $-CF_2-$;
- $R_b$ représente un groupe $-(C_1-C_5)$alkylène- ou $-CF_2-$;
- $R_c$ représente un groupe $-(C_1-C_5)$alkyle ;
- $R_d$ représente un groupe $-(C_1-C_5)$alkyle ou $-CF_3$ ;
- L représente un agent de liaison ;
- RM* est choisi parmi RM et RM', dans lequel RM est une fonction réactive capable de former une liaison covalente avec une fraction d'un agent de ciblage, en particulier avec une fraction d'un anticorps ou un de ses fragments fonctionnels, et dans lequel RM' est une fraction de RM portant au moins un groupe protecteur ;

à l'état de base ou de sels de bases ou sous forme d'hydrate ou de solvate.

Selon un premier mode préféré, le groupe L-RM* est choisi parmi :

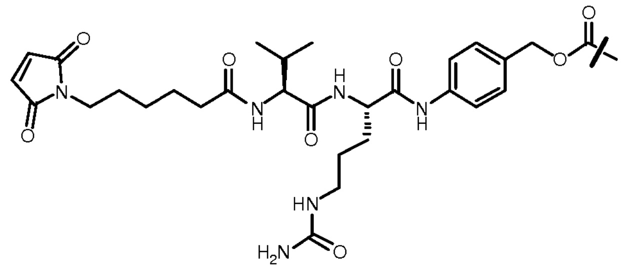

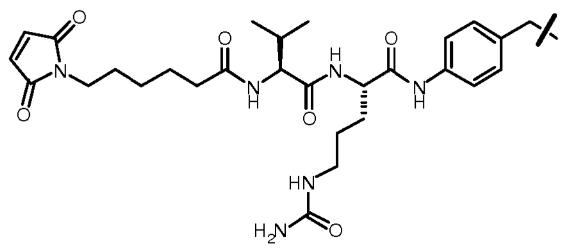

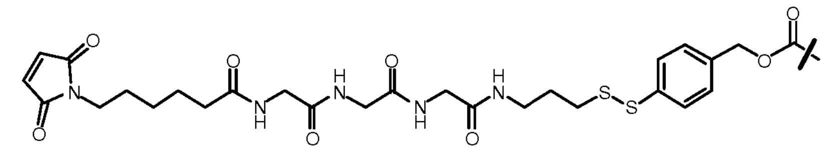

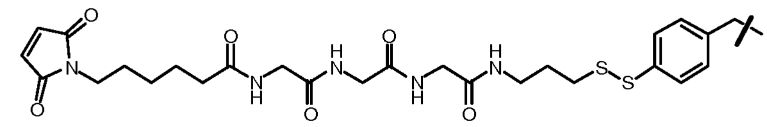

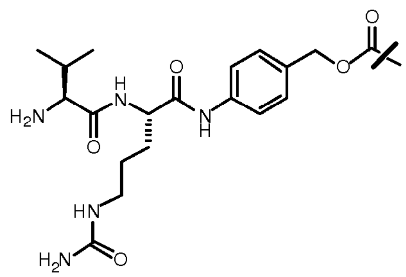

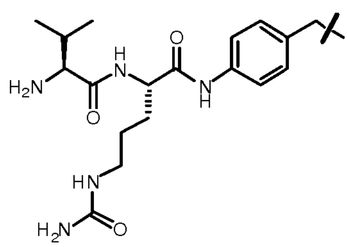

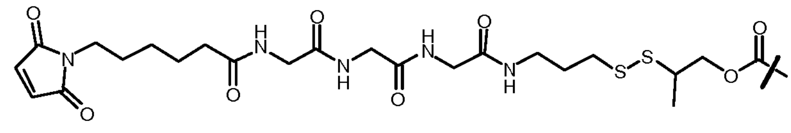

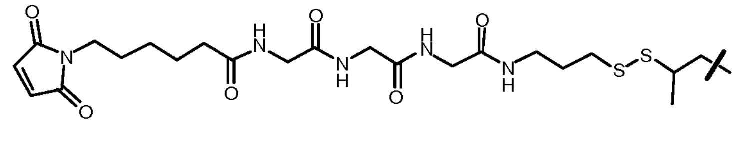

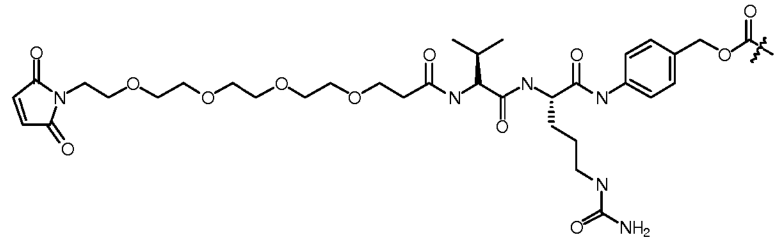

et

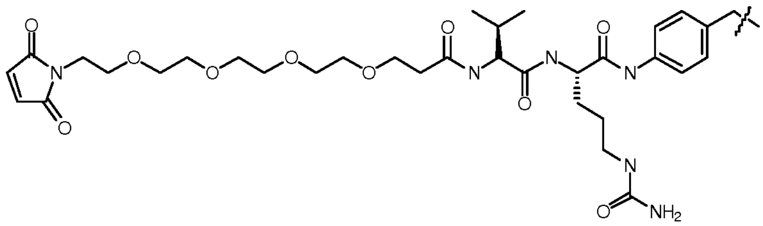

Selon un autre mode préféré, le composé est choisi parmi :

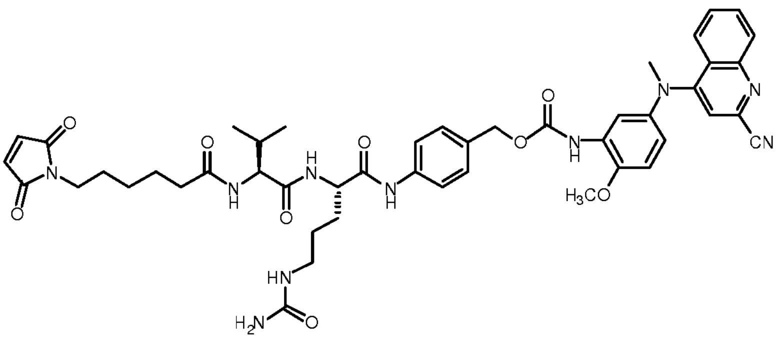

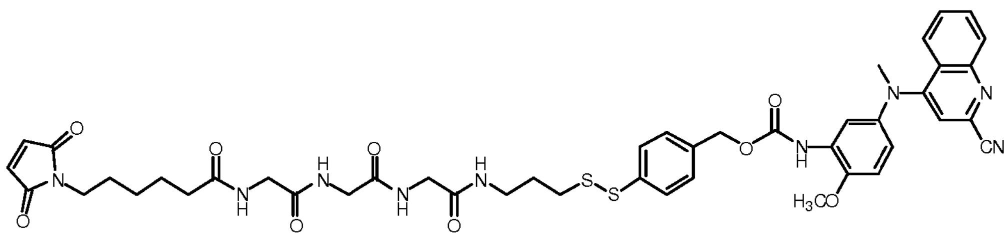

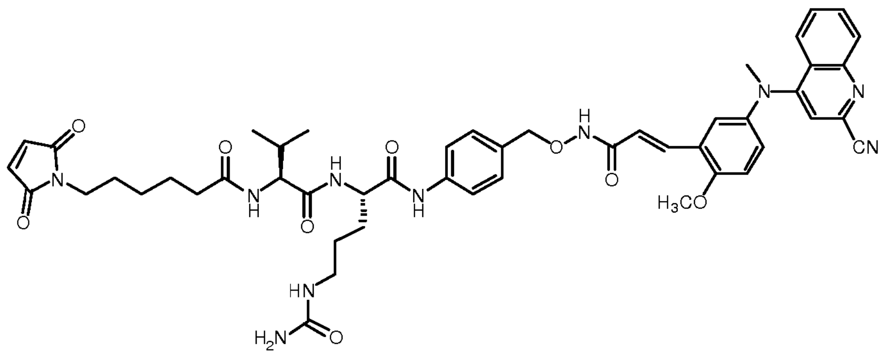

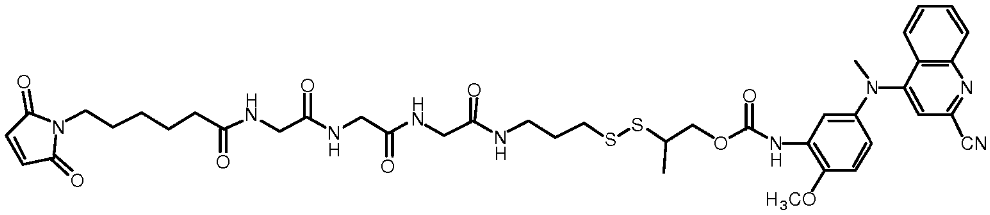

et

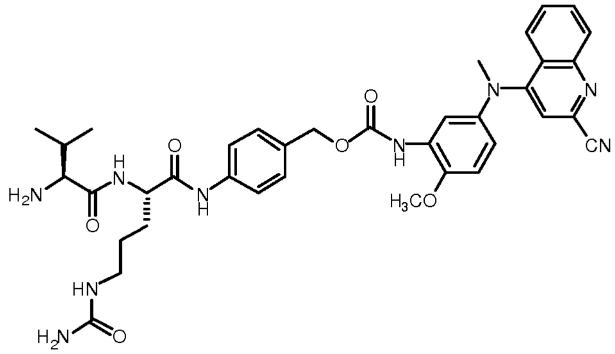

Agent de liaison (Linker)

Un agent de liaison ou « linker » selon la présente invention est un composé reliant deux composants, chacun étant attaché à une extrémité de l'agent de liaison.

Dans le cas où l'agent de liaison est une liaison covalente, une liaison directe du composé toxique (« payload ») à l'agent de ciblage, tel qu'un anticorps, peut diminuer la capacité du payload à interagir avec la molécule ciblée à l'intérieur de la cellule.

Selon des modes de réalisations préférés, le linker augmente la distance entre les deux composants, en particulier entre le payload et l'agent de ciblage, et atténue ainsi les interférences stériques entre eux.

En particulier, le linker possède une chaîne continue comprenant de 1 à 30 atomes dans son squelette, la longueur du linker étant définie comme le nombre d'atomes ou de liaison le plus faible entre le payload et l'agent de ciblage.

Selon un mode de réalisation particulier, le linker est choisi parmi les groupes $C_1$-$C_{20}$-alkylène, $C_1$-$C_{20}$-hétéroalkylène, $C_2$-$C_{20}$-alcénylène, $C_2$-$C_{20}$-hétéroalcénylène, $C_2$-$C_{20}$-alcynylène, $C_2$-$C_{20}$-hétéroalcynylène, cycloalkylène, hétérocycloalkylène, arylène, hétéroarylène, aralkylène, ou hétéroaralkylène, éventuellement substitué. Le linker peut contenir un ou plusieurs éléments tels qu'un carboxamide, un ester, un éther, un thioéther, un disulfure, une urée, une thiourée, une fraction hydrocarbonée, ou un de leurs dérivés. Le linker peut contenir un ou plusieurs de ces éléments structuraux. Chacun de ces éléments peut être présent dans le linker une ou plusieurs, par exemple deux, trois, quatre, cinq voire six fois.

Selon un mode de réalisation particulier, le linker peut comprendre une liaison disulfure.

Il est entendu que le linker doit être attaché en une seule étape ou en plusieurs, par exemple deux, étapes successives, au payload et à l'agent de ciblage. A cette fin, le linker possède deux groupes, en particulier aux extrémités proximale et distale, qui peuvent (i) former une liaison covalente avec une groupe présent dans l'un des composants à lier, en particulier un groupe activé sur la payload ou sur l'agent de ciblage, ou (ii) qui est ou peut être activé pour former une liaison covalente sur un groupe du payload.

Ainsi, selon un mode préféré, les groupes chimiques présents aux extrémités du linker, résultant de la réaction de couplage, sont choisis parmi les esters, les éthers, les uréthanes, les liaisons peptidiques...

Selon un mode de réalisation particulier, le linker L est une chaine linéaire comprenant de 1 à 20 atomes, choisis indépendamment les uns des autres parmi C, O, N et S, en particulier comprenant de 2 à 18 atomes, plus particulièrement entre 5 et 16 atomes, et de préférence entre 6 et 15 atomes.

Selon un mode de réalisation particulier, au moins 60 % des atomes de la chaîne linéaire sont des atomes de carbone. En particulier, les atomes de la chaîne linaire sont liés par des liaisons simples.

Selon un mode de réalisation particulier, le linker L est un groupe choisi parmi les alkylène, hétéroalkylène, alcénylène, hétéroalcénylène, alcynylène, hétéroalcynylène, cycloalkylène, hétérocycloalkylène, arylène, hétéroarylène, aralkylène, ou hétéroaralkylène, comprenant de 1 à 4 hétéroatomes choisis parmi N, O et S, ledit linker étant éventuellement substitué.

Selon un mode de réalisation particulier, le linker L comprend au moins un des groupements suivants : un disulfure (-S-S-), un éther (-O-), un thioéther (-S-), une amine (-NH-), un ester (-O-C(=O)- ou -C(=O)-O-), un carboxamide (-NH-C(=O)- ou C(=O)-NH-), un uréthane (-NH-C(=O)-O- ou -O-C(=O)-NH-), ou une fraction urée (-NH-C(=O)-NH-).

Selon un mode de réalisation particulier de la présente invention, le linker L comprend un nombre m de groupes choisis parmi les alkylène, alcénylène, alcynylène, cycloalkylène, hétéroalkylène, hétéroalcénylène, hétéroalcynylène, hétérocycloalkylène, arylène, hétéroarylène, aralkylène, et hétéroaralkylène, chacun de ces groupes pouvant être éventuellement substitués, et un nombre n de groupes choisis indépendamment les uns des autres parmi au moins un des groupements suivants : un disulfure (-S-S-), un éther (-O-), un thioéther (-S-), une amine (-NH-), un ester (-O-C(=O)- ou -C(=O)-O-), un carboxamide (-NH-C(=O)- ou C(=O)-NH-), un uréthane (-NH-C(=O)-O- ou -O-C(=O)-NH-), ou une fraction urée (-NH-C(=O)-NH-), m étant égal à n+1. En particulier, m est 2 et n est 1, ou m est 3 et n est 2. Plus particulièrement, le linker comprend 2 ou 3 groupes alkylène non substitué, et 1 ou 2, respectivement, disulfure, éther, thioéther, amine, ester, carboxamide, uréthane ou fraction urée liés aux groupes alkylène non substitué.

Selon un mode de réalisation particulier, les atomes de carbone dans la chaîne linéaire font indépendamment partis de groupes méthylène éventuellement substitués ($-CH_2-$). En particulier, les substituants optionnels sont indépendamment choisis parmi les halogènes et les groupes $-(C_1-C_6)$alkyle, en particulier méthyle.

En particulier, le linker L est un agent de liaison stable, c'est-à-dire qu'il est stable (i) en présence d'enzymes, et (ii) dans un environnement réducteur intracellulaire.

Selon un mode de réalisation particulier, le linker stable ne contient pas (i) une sous-structure enzymatique clivable et/ou (ii) un groupe disulfure. En particulier, le linker possède une longueur allant jusqu'à 12 atomes, en particulier une longueur allant de 2 à 10 atomes, plus particulièrement de 4 à 9, et encore plus particulièrement de 6 à 8.

Selon un autre mode de réalisation particulier, le linker est un agent de liaison clivable, c'est-à-dire (i) qu'il est clivable par clivage chimique, ou (ii) qu'il s'agit d'un agent de liaison réductible.

Selon un mode de réalisation, l'agent de liaison est clivable par réduction, c'est-à-dire qu'il peut être clivé dans un environnement réducteur intracellulaire. En particulier, il peut s'agir d'un linker contenant des groupes disulfures, résultant en la libération intracellulaire du payload conjugué à l'agent de ciblage après internalisation par l'environnement réducteur intracellulaire (Shen et al., (1985), J. Biol. Chem. 260:10905-10908).

Selon un mode de réalisation particulier, l'agent de liaison comprend une liaison disulfure, en particulier une fraction $-CMe_2-S-S-CMe_2-$. Selon d'autres modes de réalisation, le linker est attaché à un groupe thiol d'une fraction d'un agent de ciblage par une liaison disulfure.

Selon d'autres modes de réalisation, le linker est clivable par clivage chimique, en particulier par hydrolyse ou protéolyse. En particulier, le clivage chimique est catalysé par une enzyme, c'est-à-dire que le linker peut être clivé par une enzyme, en particulier une peptidase lysosomale, telle que la Cathepsin B, résultant en une libération intracellulaire du payload conjugué à l'agent de ciblage après internalisation (Dubowchik et al., (2002) Bioconjug Chem. 13:855-69). Selon un mode de réalisation particulier, le linker clivable comprend un dipeptide choisi parmi Phe-Lys, Val-Lys, Phe-Ala, Val-Ala, Phe-Cit et Val-Cit. En particulier, le linker clivable comprend en outre un espaceur p-aminobenzyl (PAB) entre les dipeptides et le payload.

Selon un mode de réalisation, le linker comprend un groupe hydrazone. Dans ce mode de réalisation particulier, le clivage intervient par hydrolyse dans le lysosome.

Selon un mode de réalisation, le linker est un agent de liaison auto-sacrificiel, c'est-à-dire qu'il comprend une liaison clivable. En particulier, après clivage, une fragmentation intervient qui retire une partie du linker toujours attaché au payload après ledit clivage. En particulier, le linker peut comprend un groupe -(liaison clivable)-X-phényle-$CH_2$-O-C(=O)-, dans lequel le groupe carbonyle est attaché au groupe amino attaché au cycle phénylé dans les composés selon l'invention, le composé selon l'invention comprenant un groupe amino libre étant libéré.

Selon un mode de réalisation préféré, l'agent de liaison est choisi parmi les composés suivants :

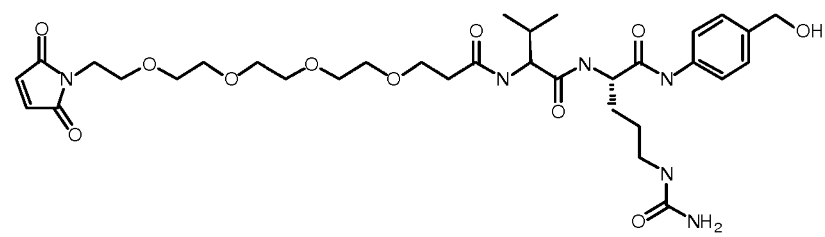

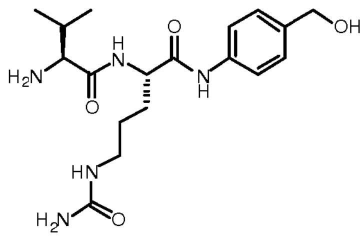

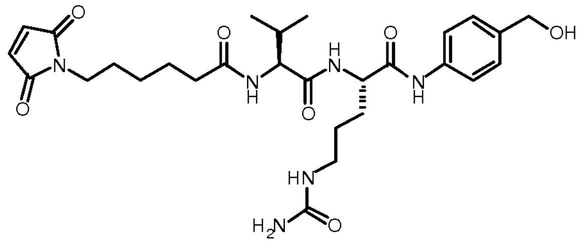

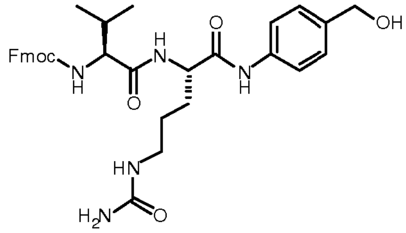

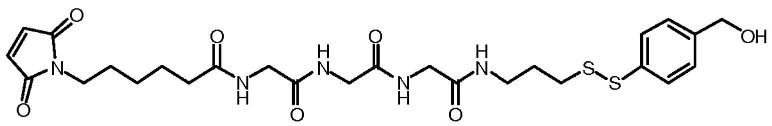

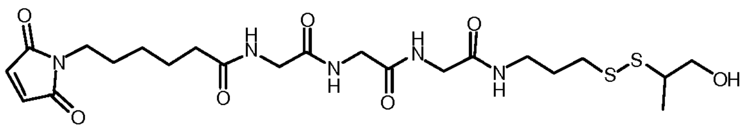

A titre d'agents de liaison convenant à la présente invention, peuvent également être cités ceux décrits dans Jun Lu et al. (« Linker shaving a crucial role in antibody-drug conjugates », Int. J. Mol. Sci. 2016, 17, 561), Jessica R. McCombs et Shawn C. Owen (« Antibody drug conjugates : Design and sélection of linker, payload and conjugation chemistry », The AAPS Journal, vol. 17, No. 2, March 2015, 339), Laurent Ducry et Bernhard Strump (« Antibody-drug conjugates : linking cytotoxic payloads to monoclonal antibodies », Bioconjugate Chem. 2010, 21, 5-13*),* et Nareshkumar Jain et al. (« Current ADS linker chemistry, Pharm. Res. 2015, 32:3526-3540).

**CONJUGUES ANTICORPS-MEDICAMENT**

La présente invention concerne également des conjugués anticorps-médicament comprenant des composés tels que définis précédemment.

Selon un autre de ses aspects, la présente invention concerne des conjugués anticorps-médicament de formule (III) :

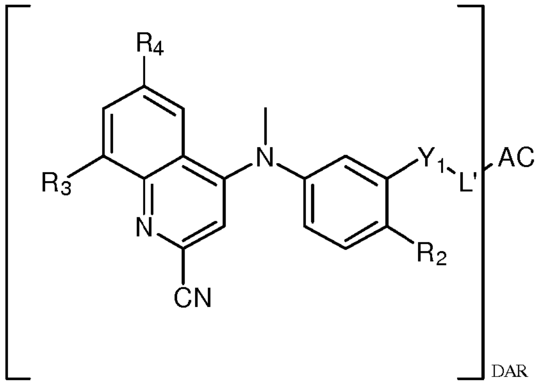

(III)

dans laquelle :

- Yi représente -O-, -NH-, -NHCO-, $-NHCOR_a-$, -NHCOO-, $-NHCOOR_b-$, $-(C_2-C_6)$alcénylène-CO-NH-O- ou $-(C_2-C_6)$alcynylène-CO-NH-O- ;
- $R_2$ représente un groupe $-OCH_3$, $-OCH_2CH_3$, $-SCH_3$, $-SCH_2CH_3$ ou $-OCHF_2$ ;
- $R_3$ représente un atome d'hydrogène ou un groupe $-CH_3$, -CN, -F, -Cl ou $-OR_c$ ; et de préférence un atome d'hydrogène ;
- $R_4$ représente un atome d'hydrogène ou un groupe $-CH_3$, -CN, -F, -Cl ou $-OR_d$ ; et de préférence un atome d'hydrogène ;
- $R_a$ représente un groupe $-(C_1-C_5)$alkylène- ou $-CF_2-$;
- $R_b$ représente un groupe $-(C_1-C_5)$alkylène- ou $-CF_2-$;
- $R_c$ représente un groupe $-(C_1-C_5)$alkyle ;
- $R_d$ représente un groupe $-(C_1-C_5)$alkyle ou $-CF_3$ ;
- L' représente un agent de liaison ;
- AC représente une fraction d'un agent de ciblage, en particulier une fraction d'un anticorps ou un de ses fragments fonctionnels ; et

dans laquelle le DAR (ratio médicament/agent de ciblage) varie entre 1 et 8, de préférence entre 2 et 4.

Agent de ciblage

On entend par agent de ciblage une molécule présentant une affinité pour une cible biologique. L'agent de ciblage a pour fonction de diriger le composé cytotoxique vers la cible biologique.

On entend par cible biologique un antigène, ou un groupe d'antigènes, de préférence localisé à la surface de cellules cancéreuses. Ces antigènes peuvent par exemple être un récepteur de facteur de croissance, un gène ou un produit oncogène « suppresseur de tumeur » muté, une molécule apparentée à l'angiogénèse ou une molécule d'adhésion.

En particulier, l'agent de ciblage est choisi parmi un ligand, une protéine, un anticorps, par exemple un anticorps monoclonal, un fragment de protéine ou d'anticorps, un peptide, un oligonucléotide ou un oligosaccharide.

De préférence, il s'agit d'un anticorps (ou immunoglobuline) ou d'un fragment d'anticorps.

A titre d'anticorps convenant à la présente invention, on peut notamment citer les immunoglobulines IgA, IgD, IgE, IgG et IgM.

Les anticorps peuvent être des anticorps monoclonaux, des anticorps polyclonaux, des anticorps recombinants, des anticorps chimériques, des anticorps humanisés ou des anticorps optimisés, par exemple des anticorps avec une glycosylation modifiée ou des anticorps ayant une région Fc variante présentant une affinité de liaison optimisée avec un ou plusieurs récepteurs Fc. En particulier, il s'agit d'un anticorps monoclonal.

Plus particulièrement, l'anticorps peut être choisi parmi :

- les anticorps antinucléaires, comprenant les auto-anticorps anti-SSA/Ro, les auto-anticorps anti-La/SS-B, les anticorps anti-centromères, l'anticorps-2 nucléaire anti-neuronal, l'anti-ADNdb, l'anti-RNP, l'anti-Smith, l'anticorps anti-topoisomérase, les anticorps anti-histones, les anticorps anti-p62, et les anticorps anti-sp100 ;
- les anticorps anti-glycoprotéine 210 ;
- les anticorps anti-transglutaminase, comprenant les anticorps anti-tTG et les anticorps anti-eTG ;
- les anticorps anti-gangliosides ;

- les anticorps anti-actine ;
- les anticorps anti-CCP, les anticorps de type 1 microsomal de foie et de rein ;
- les anticorps anti-thrombine ;
- les anticorps cytoplasmique anti-neutrophiles (ANCA) comprenant l'anti-myéloperoxydase (MPO), l'anti-protéinase 3 (PR3), l'anti-lactoferrine, l'anti-élastase, la protéine inductrice bactérienne (BPI), l'anti-cathepsine G ;
- la membrane basale anti-glomérulaire (chaîne 3 alpha du collagène 4), le récepteur A2 anti-phospholipase (PLA2R) ;
- les anticorps anti-facteur rhumatoïde ;
- l'anticorps anti-muscle lisse, comprenant les anticorps anti-actine, les anticorps anti-troponine et les anticorps anti-tropomyosine ;
- les anticorps anti-mitochondriaux, comprenant les anticorps anti-cardiolipine, les anticorps anti-sulfite oxydase, les anticorps anti-sarcosine déshydrogénase et les anticorps anti-glycogène phosphorylase ;
- les anticorps anti-SRP ;
- les anticorps anti-VGCC (canal calcique voltage-dépendant) ;
- les anticorps anti-VGKC (canal potassium voltage-dépendant) ;
- les anticorps anti-synthétase comprenant les anticorps anti-PL7, -PL12, -JO1, -EJ, et -OJ ;
- et les anticorps anti-complément terminal, comprenant les auto-anticorps anti-facteur H, l'inhibiteur anti-C1, les anti-C1q, les anti-C3, les anti-facteurs B, les anti-C3bBb (C3 convertase de la voie alternative du complément), les anti-C4b2a (C3 convertase de la voie classique du complément).

A titre d'allo-anticorps, on peut citer les anticorps antigènes plaquettaires humains (HPA) et les anticorps anti-IgA.

A titre d'anticorps thérapeutiques humains, on peut citer ceux choisis dans le groupe comprenant Panitumumab. Actoxumab, Adalimumab, Adecatumumab, Alirocumab, Anifrolumab, Atinumab, Atorolimumab, Belimumab, Bertilimumab, Bezlotoxumab, Bimagrumab, Briakinumab, Brodalumab, Canakinumab, Carlumab, Cixutumumab, Conatumumab, Daratumumab, Denosumab, Drozitumab, Duligotumab, Dupilumab, Dusigitumab, Efungumab, Eldelumab, Enoticumab, Evolocumab, Exbivirumab, Fasinumab, Fezakinumab, Figitumumab, Flanvotumab, Foralumab, Foravirumab, Fresolimumab, Fulranumab, Ganitumab, Gantenerumab, Glembatumumab vedotin, Golimumab, Guselkumab, Icrucumab, Inclacumab, Intetumumab, Ipilimumab, Iratumumab, Lerdelimumab, Lexatumumab, Libivirumab, Lirilumab, Lucatumumab, Mapatumumab, Mavrilimumab, Metelimumab, Morolimumab, Namilumab, Namatumab, Nebacumab, Necitumumab, Nesvacumab, Nivolumab, Ofatumumab, Olaratumab, Orticumab, Oxelumab, Panitumumab, Panobacumab, Parsatuzumab, Patritumab, Placulumab, Pritumumab, Radretumab, Rafivirumab, Ramucirumab, Raxibacumab, Regavirumab, Rilotumumab, Robatumumab, Roledumab, Sarilumab, Secukinumab, Seribantumab, Sevirumab, Sirukumab, Stamulumab, Tabalumab, Teprotumumab, Ticilimumab (= tremelimumab), Tovetumab, Tralokinumab, Tremelimumab, Tuvirumab, Urelumab, Ustekinumab, Vantictumab, Vesencumab, Votumumab, Zalutumumab, Zanolimumab, Ziralimumab.

A titre d'anticorps thérapeutiques murins, on peut citer ceux choisis dans le groupe comprenant Abagovomab, Afelimomab, Anatumomab mafenatox, Blinatumomab, Detumomab, Dorlimomab aritox, Edobacomab, Edrecolomab, Elsilimomab, Enlimomab pegol, Epitumomab cituxetan, Faralimomab, Gavilimomab, Ibritumomab tiuxetan, Imciromab, Inolimomab, Lemalesomab, Maslimomab, Minretumomab, Mitumomab, Moxetumomab pasudotox, Muromonab-CD3, Nacolomab tafenatox, Naptumomab estafenatox, Nerelimomab, Odulimomab, Oregovomab, Pemtumomab, Racotumomab, Solitomab, Taplitumomab paptox, Telimomab aritox, Tenatumomab, Tositumomab, Vepalimomab et Zolimomab aritox.

A titre d'anticorps thérapeutiques chimériques, on peut citer ceux choisis dans le groupe comprenant Abciximab, Amatuximab, Basiliximab, Bavituximab, Brentuximab vedotin, Cetuximab, Clenoliximab, Ecromeximab, Ensituximab, Futuximab, Galiximab, Girentuximab, Gomiliximab, Indatuximab ravtansine, Infliximab, Keliximab, Lumiliximab, Pagibaximab, Priliximab, Pritoxaximab, Rituximab, Setoxaximab, Siltuximab, Teneliximab, Ublituximab, Vapaliximab, Volociximab et Zatuximab.

A titre d'anticorps thérapeutiques humanisés, on peut citer ceux choisis dans le groupe comprenant Afutuzumab, Alacizumab pegol, Alemtuzumab, Anrukinzumab, Apolizumab, Aselizumab, Atlizumab (= tocilizumab), Bapineuzumab, Benralizumab, Bevacizumab, Bivatuzumab mertansine, Blosozumab, Cantuzumab mertansine, Cantuzumab ravtansine, Caplacizumab, Cedelizumab, Certolizumab pegol, Citatuzumab bogatox, Clazakizumab, Clivatuzumab tetraxetan, Concizumab, Crenezumab, Dacetuzumab, Daclizumab, Dalotuzumab, Demcizumab, Eculizumab, Efalizumab, Elotuzumab, Enavatuzumab, Enokizumab, Epratuzumab, Erlizumab, Etaracizumab, Etrolizumab, Farletuzumab, Felvizumab, Ficlatuzumab, Fontolizumab, Gemtuzumab ozogamicin, Gevokizumab, Ibalizumab, Imgatuzumab, Inotuzumab ozogamicin, Itolizumab, Ixekizumab, Labetuzumab, Lambrolizumab, Lampalizumab, Lebrikizumab, Ligelizumab, Lintuzumab, Lodelcizumab, Lorvotuzumab mertansine, Margetuximab, Matuzumab, Mepolizumab, Milatuzumab, Mogamulizumab, Motavizumab, Natalizumab, Nimotuzumab, Ocaratuzumab, Ocrelizumab, Olokizumab, Omalizumab, Onartuzumab, Oportuzumab monatox, Ozanezumab, Ozoralizumab, Palivizumab, Pascolizumab, Pateclizumab, Perakizumab, Pertuzumab, Pexelizumab, Pidilizumab, Pinatuzumab vedotin, Polatuzumab vedotin, Ponezumab, Quilizumab, Ranibizumab, Reslizumab, Romosozumab, Rontalizumab, Rovelizumab, Ruplizumab, Samalizumab, Sibrotuzumab, Sifalimumab, Simtuzumab, Siplizumab, Solanezumab, Sonepcizumab, Sontuzumab, Suvizumab, Tacatuzumab tetraxetan, Tadocizumab, Talizumab, Tanezumab, Tefibazumab, Teplizumab, Tildrakizumab, Tigatuzumab, Tocilizumab (= atlizumab), Toralizumab, Trastuzumab, Tregalizumab, Tucotuzumab celmoleukin, Urtoxazumab, Vatelizumab, Vedolizumab, Veltuzumab, Visilizumab et Vorsetuzumab mafodotin.

De préférence, il s'agit d'un anticorps thérapeutique humanisé, et plus préférentiellement du Trastuzumab.

DAR

Un conjugué comprend généralement un nombre moyen de composés cytotoxiques (payload) compris entre 1 et 8, de préférence entre 2 et 4, liés à l'agent de ciblage (il d'agit du degré de greffage ou « ratio médicament/agent de ciblage » (ou « DAR »)).

Ce nombre peut notamment varier en fonction de la nature de l'agent de ciblage, du payload, ou des conditions utilisées lors de la conjugaison.

Dans le cas où l'agent de ciblage est un anticorps, le DAR peut être déterminé par exemple par spectroscopie UV ou par déconvolution du spectre HRMS du conjugué.

Le DAR évalué par spectroscopie UV est appelé DAR(UV), comme indiqué dans la méthode présentée par Antony S. Dimitrov ((ed), LLC, 2009, "Therapeutic Antibodies and Protocols", vol. 525, 445, Springer Science). Cette méthode consiste à mesurer l'absorbance d'une solution du conjugué après l'étape de séparation à deux longueurs d'onde WL1 et WL2. Les coefficients d'extinction molaire obtenus de l'anticorps nu et du payload avant la conjugaison sont utilisés. Les absorbances de la solution de conjugué à WL1 et WL2, ($A_{WL1}$) et ($A_{WL2}$) sont mesurées soit sur le pic UV correspondant du spectre SEC soit en utilisant un spectrophotomètre UV standard. Les absorbances peuvent être exprimées sous la forme :

$$A_{WL1} = (c_D \text{ x } e_{D\,WL1}) + (c_A \text{ x } e_{A\,WL1})$$

$$A_{WL2} = (c_D \text{ x } e_{D\,WL2}) + (c_A \text{ x } e_{A\,WL2})$$

dans lesquelles:

- $c_D$ et $c_A$ représentent, respectivement, les concentrations dans la solution de la partie du conjugué relative au payload et de la partie du conjugué relative à l'anticorps ;
- $e_{D\,WL1}$ et $e_{D\,WL2}$ représentent, respectivement, les coefficients d'extinction molaire du payload avant conjugaison aux longueurs d'onde WL1 et WL2 ;
- $e_{A\,WL1}$ et $e_{A\,WL2}$ représentent, respectivement, les coefficients d'extinction molaire de l'anticorps nu aux longueurs d'onde WL1 et WL2.

L'expression « anticorps nu » désigne l'anticorps sur lequel aucun payload n'est attaché, c'est-à-dire l'anticorps avant conjugaison.

La résolution de ces deux équations conduit à :

$$c_D = [(e_{A\,WL1} \text{ x } A_{WL2}) - (e_{A\,WL2} \text{ x } A_{WL1})] / [(e_{D\,WL2} \text{ x } e_{A\,WL1}) - (e_{A\,WL2} \text{ x } e_{D\,WL1})]$$

$$c_A = [A_{WL1} - (c_D \text{ x } e_{D\,WL1})] / e_{A\,WL1}$$

Le DAR(UV) correspond alors à $c_D/c_A$.

Alternativement, le DAR peut être calculé par déconvolution du spectre HRMS du conjugué et est alors nommé DAR (HRMS).

**PROCEDE DE PREPARATION ET CONJUGAISON**

La présente invention concerne également un procédé de préparation d'un composé de formule (II) tel que défini précédemment, caractérisé en ce qu'il comprend une étape de réaction d'un composé de formule (I) tel que défini précédemment, avec un composé de formule X-L"-RM* dans laquelle :

- X représente un groupe capable de réagir avec un groupe $R_1$ tel que défini précédemment ;
- L" représente un agent de liaison ;
- RM* est choisi parmi RM et RM', dans lequel RM est une fonction réactive capable de former une liaison covalente avec une fraction d'un agent de ciblage, en particulier avec une fraction d'un anticorps ou un de ses fragments fonctionnels, et dans lequel RM' est une fraction de RM portant au moins un groupe protecteur ;

dans lequel la réaction entre la fraction $-R_1$ du composé de formule (I) et le composé de formule X-L"-RM* résulte en la formation d'une fraction $-Y_1$-L-RM*.

Selon un mode préféré, dans lequel RM* est RM, le procédé comprend en outre une étape de déprotection d'une fraction RM' résultant en un groupe RM.

De préférence, le procédé comprend une étape de réaction d'un composé de formule (II) tel que défini précédemment avec une fraction d'un agent de ciblage.

La conjugaison de la fraction d'un agent de ciblage peut être réalisée par couplage d'une construction composé de formule(I)-L-RM avec des groupes aminos libres présents dans la fraction de l'agent de ciblage. Selon un tel mode de réalisation, le groupe RM peut être choisi parmi un dérivé d'acide carboxylique activé, tel qu'un ester N-hydroxy succimide, ou un dérivé d'acide carbonique activé, tel qu'un isothiocyanate.

La conjugaison de la fraction d'un agent de ciblage peut également être réalisée par couplage d'une construction composé de formule(I)-L-RM avec des groupes thiols libres présents dans la fraction de l'agent de ciblage. Selon un tel mode de réalisation, le groupe RM peut être choisi parmi un groupe haloacétyl, un groupe RM comprenant un alkène substitué accepteur (système Mickael), en particulier un groupe maléimide ou un groupe propenoyl (Badescu et al., 2014, Bioconjugate Chem., 25:460-469), un groupe maléimide substitué en position 3 ou disubstitué en positions 3 et 4 par des groupes partants X, en particulier choisis parmi Cl, Br, et aryl-S-, en particulier phényle-S-.

Selon un mode de réalisation particulier, le groupe thiol fait partie d'un résidu cystéine simple non couplé présent dans la fraction de l'agent de ciblage sauvage. Selon un autre mode de réalisation particulier, le groupe thiol fait partie d'un résidu cystéine simple non couplé généré à partir d'une fraction de l'agent de ciblage sauvage, en particulier par génie génétique recombinant, par exemple par insertion dans la séquence sauvage, par élimination d'une seconde cystéine qui forme un pont disulfure avec le premier résidu de cystéine dans la fraction de l'agent de ciblage sauvage, ou par remplacement d'un résidu non-cystéine. Selon un autre mode de réalisation, le groupe thiol est généré par réduction d'une liaison disulfure entre deux cystéines présentes dans la fraction de l'agent de ciblage sauvage.

La conjugaison de la fraction d'un agent de ciblage peut également être réalisée par couplage d'une construction composé de formule(I)-L-RM avec deux groupes thiols libres présents dans la fraction de l'agent de ciblage. Selon un tel mode de réalisation, le groupe RM peut être choisi parmi un groupe maléimide disubstitué en positions 3 et 4 avec des groupes partants X, en particulier choisis parmi Cl, Br, et aryl-S-, en particulier phényle-S-.

Selon un mode de réalisation, les deux groupes thiol font chacun partie d'un résidu cystéine simple non couplé présent dans la fraction de l'agent de ciblage sauvage. Selon un autre mode de réalisation particulier, les groupes thiols font partie de deux résidus cystéine simples non couplés générés à partir d'une fraction de l'agent de ciblage sauvage, en particulier par génie génétique recombinant, par exemple par insertion dans la séquence sauvage, par élimination d'une seconde cystéine qui forme un pont disulfure avec le premier résidu de cystéine dans la fraction de l'agent de ciblage sauvage, ou par remplacement d'un résidu non-cystéine. Selon un autre mode de réalisation, les deux groupes thiol sont générés par réduction d'une liaison disulfure entre deux cystéines présentes dans la fraction de l'agent de ciblage sauvage. Par réaction de tels deux groupes thiols avec un maléimide disubstitué, les groupes thiols sont pontés, imitant ainsi un pont disulfure présent originellement.

Selon un mode de réalisation particulier, un groupe thiol libre peut être généré par thiolation de groupes amino libres présent dans la fraction de l'agent de ciblage, en particulier par réaction de tels groupes amino libres avec un agent de thiolation choisi parmi les 2-iminothiolane (Still et al., 1984, Can. J. Org. Chem. 62:586) et un dérivé d'acide acylthioacétique (X-C(=O)-$CH_2$-SAcyl), tel qu'un ester N-hydroxy succinimide d'acide acétylthioacétique.

La conjugaison de la fraction d'un agent de ciblage peut être réalisée par couplage d'acides aminés non naturels introduits par génie génétique, par exemple par introduction de p-acétylphénylalanine et de ligature d'oxime subséquente (Kazane et al., (2012) Proc. Natl. Acad. Sci. U.S.A, 109:3731-3736*)*.

La conjugaison de la fraction d'un agent de ciblage peut être réalisée par couplage de diazodicarboxamides cyclique au cycle phénylé de résidus tyrosine dans la fraction de l'agent de ciblage (Ban et al., (2010) J Am. Chem. Soc. 13:1523-5).

La conjugaison de la fraction d'un agent de ciblage peut être réalisée via cycloaddition 1,3-dipolaire (« click chemistry »).

Selon un mode de réalisation, la fraction de l'agent de ciblage comprend une double ou une triple liaison et la construction composé de formule(I)-L-RM comprend un 1,3-dipôle, en particulier un groupe azide. En particulier, la fraction de l'agent de ciblage est mise à réagir en premier avec un ester dibenzocyclooctyne-N-hydroxysuccinimide ou un ester azadibenzocyclooctyne-Nhydroxysuccinimide (Zhou et al., (2013) J Am Chem Soc. 135:12994-7).

Selon un autre mode de réalisation, la fraction de l'agent de ciblage comprend un 1,3-dipôle, en particulier un groupe azide, et la construction composé de formule(I)-L-RM comprend une double ou une triple liaison. En particulier, la fraction de l'agent de ciblage est un anticorps glycosylé qui est mis à réagir en premier avec une molécule comprenant un azide par réaction enzymatique catalysée (*SiteClick;* Zeglis et al., (2013) Bioconjug Chem. 24:1057-67). Selon un autre mode de réalisation, le groupe azido est incorporé via l'acide aminé p-azidophénylalamine non naturel (Kazane et al., (2012) Proc. Natl. Acad. Sci. U.S.A, 109:3731-3736).

## UTILISATION PHARMACEUTIQUE

La présente invention concerne également une composition pharmaceutique comprenant un conjugué anticorps-médicament tel que défini précédemment, en association avec un support pharmaceutiquement acceptable.
En particulier, une composition pharmaceutique selon l'invention peut comprendre au moins un excipient pharmaceutiquement acceptable.
La composition pharmaceutique peut comprendre un ou plusieurs diluants pharmaceutiquement acceptable, supports, excipients, charges, liants, lubrifiants, agent de glissement, désintégrant, absorbants et/ou conservateurs.
L'invention concerne également les compositions pharmaceutiques selon l'invention pour leur utilisation en tant que médicament, notamment destiné à traiter le cancer.
La présente invention concerne également un conjugué anticorps-médicament tel que défini précédemment ou une composition pharmaceutique telle que définie ci-dessus, pour son utilisation en tant que médicament.
La présente invention concerne également un conjugué anticorps-médicament tel que défini précédemment ou une composition pharmaceutique telle que définie ci-dessus, pour son utilisation pour tuer ou inhiber la croissance des cellules.
La présente invention concerne également un conjugué anticorps-médicament tel que défini précédemment ou une composition pharmaceutique telle que définie ci-dessus, pour son utilisation dans le traitement du cancer.
Parmi les cancers, on peut citer de manière non limitative les leucémies, par exemple la leucémie myéloïde chronique, les lymphomes, les sarcomes, le mélanome, le cancer du foie, le cancer du pancréas, le cancer du poumon, le cancer de l'estomac, le cancer de l'oesophage, le cancer du rein, le cancer de la plèvre, le cancer de la thyroïde, le cancer de la peau, le cancer du col de l'utérus, le cancer du sein, le cancer des ovaires, le cancer colorectal, le cancer des testicules, le cancer de la prostate, le cancer de la vessie, le cancer du cerveau, le cancer du rectum ou le cancer des os.
En particulier, on peut citer le cancer colorectal, le cancer du poumon, notamment non à petites cellules, le cancer de l'estomac, le cancer du pancréas, la leucémie myéloïde chronique, le cancer du sein, et le cancer des ovaires, et plus particulièrement le cancer du sein.
En particulier, le patient ayant besoin d'un traitement est un mammifère, notamment un humain.
La présente invention décrit également une méthode de traitement du cancer, comprenant l'administration d'une quantité efficace d'au moins un conjugué anticorps-médicament selon l'invention, en particulier pour un patient en ayant besoin.
Selon un mode de réalisation particulier, les compositions pharmaceutiques selon l'invention sont utilisées sous forme de médicament administré par voie systémique.
Ainsi, les compositions pharmaceutiques selon l'invention peuvent être destinées à une administration par voie parentérale. Par parentéraux, on inclut les injectables et les perfusions.
Les injectables peuvent être formulés soit sous forme d'ampoules, soit sous forme d'injectables prêts à l'emploi, par exemple des seringues prêtes à l'emploi ou des seringues à usage unique, ou encore dans des flacons perforables pour une opération multiple. L'administration d'injectables peut se faire sous la forme d'une application sous-cutanée (s.c.), intramusculaire (i.m.), intraveineuse (i.v.) ou intracutanée (i.c.). En particulier, il est possible de produire des formulations injectables appropriées sous la forme d'une suspension de cristaux, de solutions, de nanoparticules ou de systèmes colloïdaux dispersés, tels que par exemple les hydrosols.
Des compositions injectables peuvent en outre être produites sous forme de concentrés, qui peuvent être dissous ou dispersés avec des diluants aqueux isotoniques. Les perfusions peuvent également être préparées sous forme de solutions isotoniques, d'émulsions grasses, de formulations liposomales et de micro-émulsions. De même que pour les injectables, les préparations pour perfusion peuvent également être préparées sous forme de concentrés pour dilution. Des formulations injectables peuvent également être appliquées sous la forme de perfusions permanentes à la fois dans la thérapie hospitalière et ambulatoire, par exemple au moyen de mini-pompes.
Il est possible d'ajouter aux compositions médicamenteuses parentérales, par exemple de l'albumine, du plasma, des diluants, des substances tensioactives, des diluants organiques, des substances influençant le pH, des substances complexantes ou des substances polymériques, notamment comme substances influençant l'adsorption des conjugués selon l'invention à des protéines ou des polymères, ou ils peuvent également être ajoutés dans le but de réduire l'adsorption des conjugués de l'invention à des matériaux tels que des instruments d'injection ou des matériaux

d'emballage, par exemple en plastique ou en verre.

Les composés selon l'invention comprenant une fraction d'un agent de ciblage peuvent être liés à des microporteurs ou à des nanoparticules parentérales, par exemple à des particules finement dispersées à base de poly(méth)acrylates, polylactates, polyglycolates, polyaminoacides ou polyéther uréthanes. Les compositions parentérales peuvent également être modifiées comme préparations retard, par exemple sur la base du « principe d'unité multiple », si les conjugués de la présente invention sont introduits sous forme finement dispersée, ou en suspension, respectivement, ou sous forme de suspension de cristaux dans le médicament ou sur la base du « principe unitaire » si le conjugué de l'invention est enfermé dans une formulation, par exemple dans un comprimé ou une tige qui est ensuite implantée. Ces implants ou dépôt de médicaments dans des formulations à unité unique et à unités multiples consistent souvent en ce qu'on appelle des polymères biodégradables, par exemple des polyesters d'acide lactique et d'acide glycolique, des polyétheruréthannes, des polyaminoacides, des poly(méth)acrylates ou des polysaccharides.

Les adjuvants et les véhicules ajoutés pendant la préparation des compositions pharmaceutiques selon la présente invention formulés en tant que produits parentéraux sont en particulier choisis parmi l'eau stérilisée (*aqua sterilisata*), des substances influençant la valeur du pH telles que les acides ou bases organiques ou inorganiques ainsi que leurs sels, les substances tampons pour ajuster les valeurs de pH, les substances pour l'isotonisation telles que le chlorure de sodium, l'hydrogénocarbonate de sodium, le glucose et le fructose, les tensioactifs et les émulsifiants, tels que les esters partiels d'acides gras de polyoxyéthylène sorbitanes (par exemple, Tween®) ou les esters d'acides gras de polyoxyéthylènes (par exemple, Cremophor®), les huiles grasses telles que l'huile d'arachide, l'huile de soja ou l'huile de ricin, des esters synthétiques d'acides gras tels que l'oléate d'éthyle, le myristate d'isopropyle et l'huile neutre (par exemple, Miglyol®) ainsi que des adjuvants polymériques tels que la gélatine, le dextrane, la polyvinylpyrrolidone, des additifs qui augmentent la solubilité de solvants organiques, tels que le propylèneglycol, l'éthanol, le N,N-diméthylacétamide, le propylène glycol ou des substances complexantes telles que le citrate et l'urée, des conservateurs tels que l'ester hydroxypropylique de l'acide benzoïque et l'ester méthylique, l'alcool benzylique, les antioxydants tels que le sulfite de sodium et les stabilisants tel que l'EDTA.

Selon un mode de réalisation préféré, lors de la formulation des compositions pharmaceutiques selon la présente invention, sont ajoutés des agents épaississants empêchant le durcissement des conjugués de l'invention, ou des tensioactifs et polyélectrolytes pour assurer la remise en suspension des sédiments et/ou des agents complexants tels que l'EDTA. Il est également possible de réaliser des complexes de l'ingrédient actif avec divers polymères. Des exemples de tels polymères sont le polyéthylèneglycol, le polystyrène, la carboxyméthylcellulose, les Pluronics® ou un ester d'acide gras et de sorbitol de polyéthylène glycol. Les conjugués de l'invention peuvent également être incorporés dans des formulations liquides sous la forme de composés d'inclusion, par exemple avec des cyclodextrines. Selon un mode de réalisation particulier, des agents dispersants peuvent être ajoutés en tant qu'adjuvants supplémentaires. Pour la production de lyophilisats, des agents tels que la mannite, le dextrane, le saccharose, l'albumine humaine, le lactose, la PVP ou des variétés de gélatine peuvent être utilisés.

Pour l'administration parentérale, la composition peut se présenter sous la forme d'une suspension aqueuse ou d'une solution qui peut contenir des agents de suspension et/ou des agents mouillants. La composition est avantageusement stérile. Elle peut se présenter sous la forme d'une solution isotonique (en particulier par rapport au sang).

Les composés selon l'invention peuvent être utilisés dans une composition pharmaceutique à une dose allant de 0,01 mg à 1000 mg par jour, administrés en une seule dose une fois par jour ou en plusieurs doses durant la journée.

La dose administrée quotidiennement est avantageusement comprise entre 5 mg et 500 mg, et plus avantageusement entre 10 mg et 200 mg.

Cependant, il peut être nécessaire d'utiliser des doses en dehors de ces plages, ce dont pourra se rendre compte l'homme du métier.

Selon un mode particulier de l'invention, les composés pour leur utilisation selon l'invention sont administrés en association avec un autre principe actif, notamment un composé anticancéreux, cytotoxique ou non. Ainsi, la composition pharmaceutique selon la présente invention peut comprendre en outre un autre principe actif.

Ainsi, la composition pharmaceutique selon l'invention comprend au moins un conjugué anticorps-médicament tel que défini précédemment et au moins un autre principe actif en tant que produit de combinaison pour une utilisation simultanée, séparée ou étalée dans le temps, qui peut notamment être utilisée pour le traitement du cancer.

Dans toute la description, y compris les revendications, l'expression « comportant un » doit être comprise comme étant synonyme de « comportant au moins un », sauf si le contraire est spécifié. Les expressions « compris entre ... et ... » et « allant de ... à ... » doivent se comprendre bornes incluses, sauf si le contraire est spécifié.

Dans la description et les exemples, la température est exprimée en degré Celsius sauf indication contraire, et la pression est la pression atmosphérique, sauf indication contraire.

L'invention est illustrée plus en détail par les exemples non limitatifs présentés ci-après.

## EXEMPLES

### Exemple 1: Préparation du 4-((3-amino-4-methoxyohenyl)(methyl)amino) ouinoline-2-carbonitrile

1.1 Schéma général

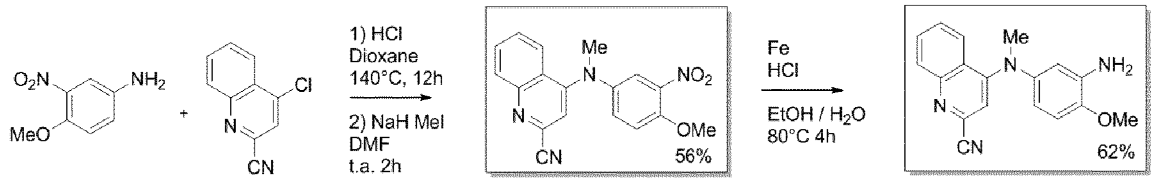


1.2 Préparation du 4-((4-methoxy-3-nitrophenyl)(methyl)amino)quinoline-2-carbonitrile

Dans un tube scellé sont ajoutés successivement le composé hétérocyclique chloré et le dérivé aromatique aminé dans 2 mL de dioxane.

Une goutte d'HCl est ensuite ajoutée au mélange et le milieu réactionnel est chauffé à 140 °C, sous agitation, pendant 12 heures.

Le mélange est refroidi, puis neutralisé avec $NaOH_{aq.}$ (5N) et la mixture est extraite à l'acétate d'éthyle (3 × 10 mL).

Les phases organiques réunies sont séchées sur $Na_2SO_4$ et concentrées sous vide. Le brut réactionnel est dissout dans une solution de DMF (5 mL) contenant du NaH à 0 °C. À ce mélange est ajouté au goutte-à-goutte $CH_3I$ et le milieu réactionnel est placé à température ambiante, sous agitation, pendant 2 heures.

Le brut réactionnel est concentré et purifié par chromatographie sur colonne de silice.

On obtient le 4-((4-methoxy-3-nitrophenyl)(methyl)amino) quinoline-2-carbonitrile sous forme solide avec un rendement de 56 %.

La température de fusion mesurée est de 219,9 - 220,5 °C.

Caractérisation $^1H$ NMR (300 MHz, $CDCl_3$) $\delta$ : 8,11 (d, J = 8,5 Hz, 1H) ; 7,71 (t, J = 8,5 Hz, 1H) ; 7,61 (d, J = 8,5 Hz, 1H) ; 7,58 (d, J = 2,7 Hz, 1H) ; 7,42 (t, J = 8,5 Hz, 1H) ; 7,34 (s, 1H) ; 7,05 (dd, J = 9,1 Hz, J = 2,8 Hz, 1H) ; 6,98 (d, J = 9,1 Hz, 1H) ; 3,94 (s, 3H) ; 3,52 (s, 3H).

Caractérisation $^{13}C$ NMR (75 MHz, $CDCl_3$) $\delta$ : 153,9 ; 150,0 ; 149,3 ; 142,1 ; 139,9 ; 134,3 ; 130,9 ; 130,8 ; 128,2 ; 127,4 ; 124,5 ; 123,6 ; 118,4 ; 117,6 ; 114,9 ; 114,5 ; 56,9 ; 42,9.

Caractérisation HRMS $C_{18}H_{15}N_4O_3$ : 335,1144 (calculé) ; 335,1150 (observé).

Caractérisation IR neat $v_{max}/cm^{-1}$ : 2363, 2340, 1679, 1625, 1603, 1460, 1275.

1.3 Préparation du 4-((3-amino-4-methoxyphenyl)(methyl)amino) quinoline-2-carbonitrile

Dans un bicol de 25 mL est ajouté le composé nitro à un mélange éthanol/eau [8/2]. Le milieu réactionnel est chauffé à 80 °C et du fer solide (10 équivalents) et 3 gouttes d'HCl sont ajoutées au milieu réactionnel. L'ensemble est chauffé à 80 °C, sous agitation jusqu'à réduction complète du composé nitro.

Le brut réactionnel est refroidi à température ambiante et filtré sur papier filtre. Le filtrat est concentré et purifié par chromatographie sur colonne de silice et le produit réduit est obtenu.

On obtient le 4-((3-amino-4-methoxyphenyl)(methyl)amino)quinoline-2-carbonitrile sous forme solide avec un rendement de 62 %.

La température de fusion mesurée est de 84,9 - 92,6 °C.

Caractérisation $^1H$ NMR (300 MHz, $CDCl_3$) $\delta$ : 7,98 (d, J = 8,4 Hz, 1H) ; 7,59 (t, J = 9,2 Hz, 2H) ; 7,26 (d, J = 8,3 Hz, 1H) ; 6,68 (d, J = 8,5 Hz, 1H) ; 6,46 (d, J = 2,5 Hz, 1H) ; 6,36 (dd, J = 8,5 Hz, J = 2,5 Hz, 1H) ; 3,88 (s, 2H) ; 3,84 (s, 3H) ; 3,42 (s, 3H).

Caractérisation $^{13}C$ NMR (75 MHz, $CDCl_3$) $\delta$ : 154,5 ; 149,7 ; 144,9 ; 143,5 ; 137,5 ; 134,0 ; 130,1 ; 129,9 ; 126,7 ; 125,6 ; 123,0 ; 118,2 ; 113,6 ; 111,0 ; 110,9 ; 110,7 ; 55,7 ; 43,8.

Caractérisation HRMS $C_{18}H_{17}N_4O$ : 305,1402 (calculé) ; 305,1399 (observé).

Caractérisation IR neat $v_{max}/cm^{-1}$ : 3475, 3377, 2960, 2837, 2236, 1570, 1502, 1264.

Caractérisation LC-MS : t.r. 15,36.

Pureté : 95,47 %.

**Exemple 2: Préparation du 4-((3-iodo-4-methoxyohenyl)(methyl)amino) quinoline-2-carbonitrile**

2.1 Préparation du 4-(3-iodo-4-methoxyphenyl)amino)guinoline-2-carbonitrile

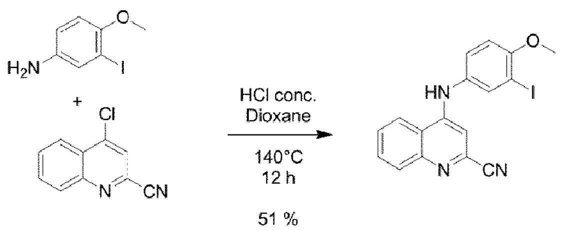


Dans un tube scellé sont mélangés le 4-chloroquinoline-2-carbonitrile (376 mg, 2,00 mmoles, 1 équivalent), et le 3-iodo-4-methoxyaniline (500 mg, 2,00 mmoles), du dioxane (10 mL) et du HCl concentré (5 gouttes).

Le mélange est chauffé une nuit à 140 °C.

Après refroidissement du milieu réactionnel, le mélange est neutralisé jusqu'à pH neutre et extrait avec de l'acétate d'éthyle. Les phases organiques sont séchées sur $MgSO_4$.

Le résidu brut est purifié par chromatographie sur gel de silice (cyclohexane : acétate d'éthyle 0 $\rightarrow$ 30 %).

Le 4-(3-iodo-4-methoxyphenyl)amino)quinoline-2-carbonitrile est isolé sous forme d'un solide jaune avec un rendement de 51 %.

Caractérisation $^1H$ RMN (300 MHz, $CDCl_3$) $\delta$ : 8,11 (d, J = 8,5 Hz, 1H) ; 7,92 (d, J = 7,7 Hz, 1H) ; 7,86 - 7,74 (m, 2H) ; 7,71 - 7,60 (m, 1H) ; 7,33 (dd, J = 8,3 ; 2,7 Hz, 1H) ; 7,00 - 6,88 (m, 2H) ; 6,73 (s, 1H) ; 3,98 (s, 3H).

Caractérisation HRMS ($ESI^+$): m/z pour $C_{17}H_{13}N_3OI$ $[M+H]^+$ 402,0103 (calculé) ; 402,0113 (trouvé).

2.2 Préparation du 4-((3-iodo-4-methoxyphenyl)(methyl)amino)quinoline-2-carbonitrile

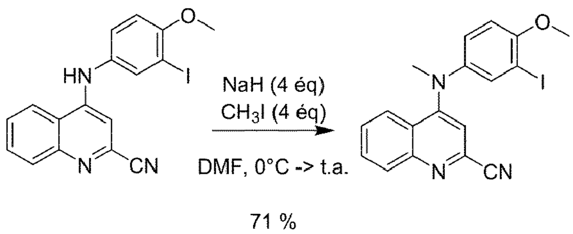


NaH (70,0 mg, 2,17 mmoles) est ajouté sur une solution de 4-((3-iodo-4-methoxyphenyl)amino)quinoline-2-carbonitrile (219 mg, 0,54 mmoles) dans du DMF (5 mL) à 0 °C. $CH_3I$ (305 mg, 2,17 mmoles) est alors rajouté goutte à goutte. Le mélange est agité pendant 30 minutes à 0 °C.

Le mélange est ramené à température ambiante, dilué avec de l'eau et ensuite extrait avec de l'acétate d'éthyle et séché sur $MgSO_4$.

Le résidu brut est purifié par chromatographie sur gel de silice (cyclohexane : acétate d'éthyle 0 $\rightarrow$ 40 %).

Le composé 4-((3-iodo-4-methoxyphenyl)(methyl)amino)quinoline-2-carbonitrile est isolé sous frome d'un solide jaune avec un rendement de 71 %.

Caractérisation $^1H$ RMN (300 MHz, $CDCl_3$) $\delta$ : 8,08 (d, J = 8.4 Hz, 1H) ; 7,79 - 7,51 (m, 3H) ; 7,48 - 7,21 (m, 2H) ; 6,92 (dd, J = 8,7 ; 2,7 Hz, 1H) ; 6,73 (d, J = 8,8 Hz, 1H) ; 3,88 (s, 3H) ; 3,48 (s, 3H).

Caractérisation $^{13}C$ RMN (75 MHz, $CDCl_3$) $\delta$ : 155,49 ; 154,17 ; 149,84 ; 144,02 ; 134,25 ; 134,19 ; 130,56 ; 130,29 ; 127,41 ; 125,07 ; 124,27 ; 123,06 ; 117,91 ; 112,35 ; 111,28 ; 86,64 ; 56,66 ; 43,64.

Caractérisation HRMS ($ESI^+$) : m/z $C_{18}H_{15}N_3OI$ $[M+H]^+$ 416,0260 (calculée) ; 416,0267 (trouvée).

Caractérisation IR (neat) : 1571, 1562, 1482, 1430, 1279, 1109, 808, 766, 713, 603 $cm^{-1}$.

Point de fusion = 175 - 180 °C.

**Exemple 3 : Préparation du (E)-5-(5-((2-cyanoquinolin-4-yl)(methyl) amino)-2-methoxyphenyt)-N-hydroxypent-4-enamide**

3.1 Préparation du N-((tetrahydro-2H-pyran-2-yl)oxy)pent-4-enamide

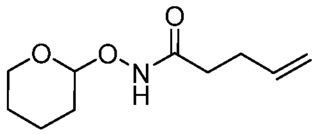

Un équivalent de l'acide carboxilique est mélangé avec un équivalent de NHTHP dans du DCM. Un équivalent de DCC est ensuite rajouté. Le mélange est agité une nuit à température ambiante et puis lavé avec une solution saturée de bicarbonate et de l'eau. Le résidu est purifié par chromatographie sur gel de silice (cyclohexane : acétate d'éthyle 1:1).

Le N-((tetrahydro-2H-pyran-2-yl)oxy)pent-4-enamide sous forme de solide blanc est obtenu avec un rendement de 86 %.

Caractérisation $^{1}$H RMN (300 MHz, DMSO) δ : 10,93 (s, 1H) ; 5,78 (dq, J = 10,6 ; 6,6 Hz, 1H) ; 5,00 (dd, J = 21,1 ; 13,7 Hz, 2H) ; 4,80 (s, 1H) ; 3,92 (m, 1H) ; 3,50 (m, 1H) ; 2,24 (m, 2H) ; 2,08 (m, 2H) ; 1,69 - 1,51 (m, 6H).

3.2 Préparation du (E)-5-(5-((2-cyanoquinolin-4-yl)(methyl)amino)-2-methoxyphenyl)-N-hydroxypent-4-enamide

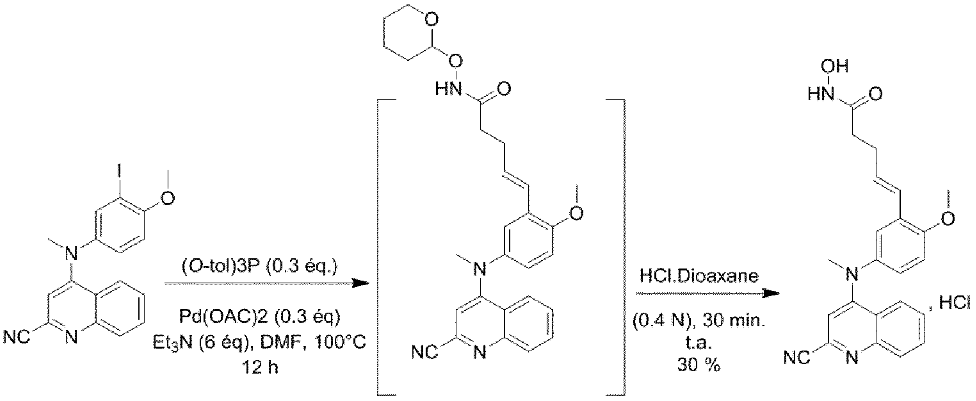


Dans un tube scellé et purgé à l'argon, sont rajoutés le 4-((3-iodo-4-methoxyphenyl)(methyl)amino)quinoline-2-carbonitrile tel que préparé dans l'exemple 2 ci-dessus (30,0 mg, 0,072 mmoles), le N-((tetrahydro-2H-pyran-2-yl)oxy)pent-4-enamide (42,0 mg, 0,20 mmoles), du tri(o-tolyl)phosphine (7,20 mg, 0,023 mmoles), du $Pd(OAC)_2$ (6,0 mg, 0,026 mmoles), du $EtN_3$ (49,0 mg, 0,48 mmoles) et du DMF (1 mL), le mélange est dégazé et chauffé une nuit à 100 °C.

Le milieu réactionnel est ramené à température ambiante dilué avec du DCM et ensuite filtré sur célite, le filtrat est évaporé. La formation du composé intermédiaire (cf. schéma ci-dessus) est confirmée par LC/MS, le composé est utilisé dans l'étape suivante sans purification.

Sur une solution du composé intermédiaire (cf. schéma ci-dessus) dans du dioxane anhydre (1 mL), est rajoutée une solution de HCl dans du dioxane 4N (0,5 mL). Le mélange est agité pendant 30 minutes à température ambiante ensuite évaporé à température ambiante et purifié par HPLC en utilisant un gradient d'acétonitrile dans l'eau. Après lyophilisation, le composé (E)-5-(5-((2-cyanoquinolin-4-yl)(methyl)amino)-2-methoxyphenyl)-N-hydroxypent-4-enamide est obtenu sous forme d'un solide jaune avec un rendement de 30 % .

Caractérisation HRMS ($ESI^+$) : m/z $C_{23}H_{23}N_4O_3$ $[M+H]^+$: 403,1770 (calculée) 403,1768 (trouvée).

Caractérisation IR (neat) : 2947, 1641, 1569, 1491,1235, 1031, 970, 758 $cm^1$.

Pureté (HPLC) : 100 %.

Caractérisation $^{1}$H RMN (400 MHz, MeOD) δ : 7,89 (d, J = 8,3 Hz, 1H) ; 7,61 (t, J = 7,4 Hz, 1H) ; 7,53 - 4,45 (m, 1H) ; 7,34 (m, 1H) ; 7,24 - 7,23 (m, 3H) ; 6,87 (m, 3H) ; 6,67 (d, J = 15,9 Hz, 1H) ; 6,17 - 6,04 (m, 1H) ; 3,81 (s, 3H) ; 3,48 (s, 4H) ; 2,46 (dd, J = 13,5 ; 6,7 Hz, 2H) ; 2,28 - 2,16 (m, 2H).

Caractérisation $^{13}$C RMN (101 MHz, MeOD) δ : 172,04 ; 156,07 ; 155,65 ; 150,60 ; 144,35 ; 134,94 ; 131,31 ;

131,22 ; 130,31 ; 129,22 ; 127,66 ; 126,91 ; 126,25 ; 125,32 ; 123,70 ; 123,63 ; 118,85 ; 113,20 ; 111,66 ; 56,24 ; 44,45 ; 33,52 ; 30,33.

Point de fusion = 177 - 182 °C.

## Exemple 4 : Préparation du 5-(5-((2-cyanoquinolin-4-yl)(methyl)amino)-2-methoxvphenyl)-N-hydroxypent-4-ynamide

### 4.1 Préparation du N-((tetrahydro-2H-pyran-2-yl)oxy)pent-4-ynamide

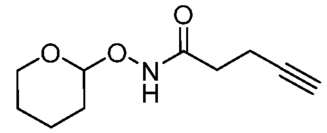

Un équivalent de l'acide carboxilique est mélangé avec un équivalent de NHTHP dans du DCM. Un équivalent de DCC est ensuite rajouté. Le mélange est agité une nuit à température ambiante et puis lavé avec une solution saturée de bicarbonate et de l'eau. Le résidu est purifié par chromatographie sur gel de silice (cyclohexane : acétate d'éthyle 1:1).

Du N-((tetrahydro-2H-pyran-2-yl)oxy)pent-4-ynamide sous forme de solide blanc est obtenu avec un rendement de 76 %.

Caractérisation $^1$H RMN (300 MHz, DMSO) $\delta$ : 11,04 (s, 1H) ; 4,80 (s, 1H) ; 3,91 (s, 1H) ; 3,50 (d, J = 11,6 Hz, 1H) ; 2,80 (s, 1H) ; 2,35 (d, J = 6,2 Hz, 2H) ; 2,18 (t, J = 7,0 Hz, 2H) ; 1,58 (m, 6H).

### 4.2 Préparation du 5-(5-((2-cyanoguinolin-4-yl)(methyl)amino)-2-methoxyphenyl)-N-hydroxypent-4-ynamide

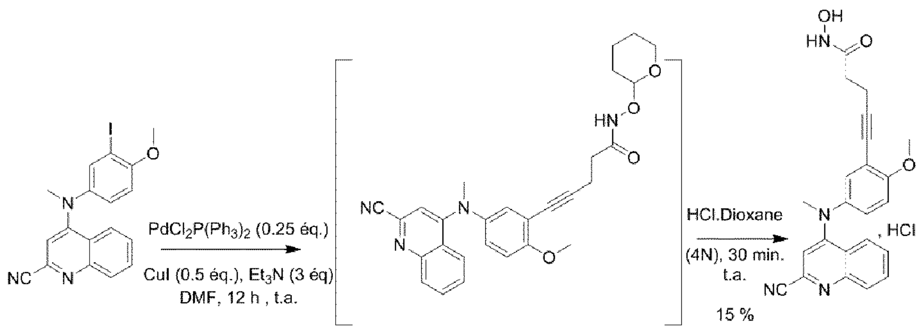


Dans un tube scellé et purgé à l'argon, sont rajoutés le 4-((3-iodo-4-methoxyphenyl)(methyl)amino)quinoline-2-carbonitrile tel que préparé dans l'exemple 2 ci-dessus (30,0 mg, 0,072 mmoles), du N-((tetrahydro-2H-pyran-2-yl)oxy)pent-4-ynamide (28,0 mg, 0,144 mmoles), du $PdCl_2P(Ph_3)_2$ (12 mg, 0,017 mmoles), du CuI (7 mg, 0,036 mmoles), du $Et_3N$ (21,0 mg, 0,22 mmoles) et du DMF (1mL).

Le mélange est dégazé et agité une nuit à température ambiante. Le mélange réactionnel est dilué avec du DCM et ensuite filtré sur célite, le filtrat est évaporé. La formation du composé intermédiaire (cf. schéma ci-dessus) est confirmée par LC/MS, le composé est utilisé dans l'étape suivante sans purification.

Sur une solution du composé intermédiaire (cf. schéma ci-dessus) (29,0 mg, 0,06 mmoles) dans du dioxane anhydre (1 mL), est rajoutée une solution de HCl dans du dioxane 4N (0,5 mL). Le mélange est agité pendant 30 minutes à température ambiante, puis évaporé à température ambiante et purifié par HPLC en utilisant un gradient d'acétonitrile dans l'eau.

Après lyophilisation, le composé 5-(5-((2-cyanoquinolin-4-yl)(methyl)amino)-2-methoxyphenyl)-N-hydroxypent-4-ynamide est obtenu sous forme d'un solide jaune avec un rendement de 15 %.

Caractérisation $^1$H RMN (400 MHz, MeOD) $\delta$ : 8,54 (s, 1H) ; 7,93 (d, J = 8,3 Hz, 1H) ; 7,72 - 7,49 (m, 2H) ; 7,30 - 7,27 (m, 2H) ; 7,18 - 6,85 (m, 3H) ; 3,83 (s, 3H) ; 3,47 (s, 3H) ; 2,70 (s, 2H) ; 2,34 (s, 2H).

Caractérisation $^{13}$C RMN (101 MHz, DMSO) $\delta$ : 167,20 ; 156,84 ; 153,86 ; 149,09 ; 142,69 ; 133,65 ; 130,27 ; 129,92 ; 128,30 ; 127,21 ; 125,14 ; 125,05 ; 122,31 ; 118,06 ; 113,16 ; 112,43 ; 112,11 ; 94,05 ; 76,58 ; 55,84 ; 43,65 ;

40,15 ; 39,94 ; 39,73 ; 39,52 ; 39,31 ; 39,10 ; 38,89 ; 31,49 ; 15,41.

Caractérisation HRMS ($ESI^+$): m/z $C_{23}H_{21}N_4O_3$ $[M+H]^+$ 403,1614 (calculée) ; 403,1621 (trouvée).

Caractérisation IR (neat) : 2987, 1644, 1496, 1066, 766 $cm^1$.

Pureté (HPLC) : 100 %.

Point de fusion = 170 - 175°C.

**Exemple 5 : Préparation du (E)-3-(5-((2-cyanoauinolin-4-yl)(methyl) amino)-2-methoxyphenyl)-N-hydroxyacrylamide**

5.1 Préparation du N-((tetrahydro-2H-pyran-2-yl)oxy)acrylamide

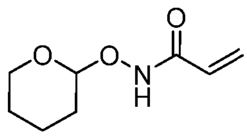

Un équivalent de l'acide carboxilique est mélangé avec un équivalent de NHTHP dans du DCM. Un équivalent de DCC est ensuite rajouté. Le mélange est agité une nuit à température ambiante, puis lavé avec une solution saturée de bicarbonate et de l'eau. Le résidu est purifié par chromatographie sur gel de silice (cyclohexane : acétate d'éthyle 1:1).

Le N-((tetrahydro-2H-pyran-2-yl)oxy)acrylamide est obtenu sous forme d'un solide blanc avec un rendement de 61 %.

Caractérisation $^1H$ RMN (200 MHz, $CDCl_3$) δ : 9,41 (s, 1H) ; 6,40 (d, J = 16,7 Hz, 1H) ; 6,15 (s, 1H) ; 5,69 (d, J = 10,4 Hz, 1H) ; 4,97 (s, 1H) ; 3,96 (t, J = 8,1 Hz, 1H) ; 3,59 (dd, J = 11,3 ; 4,0 Hz, 1H) ; 1,77 (d, J = 10,9 Hz, 3H) ; 1,58 (s, 3H).

5.2 Préparation du (E)-3-(5-((2-cyanoquinolin-4-yl)(methyl)amino)-2-methoxyphenyl)-N-hydroxyacrylamide

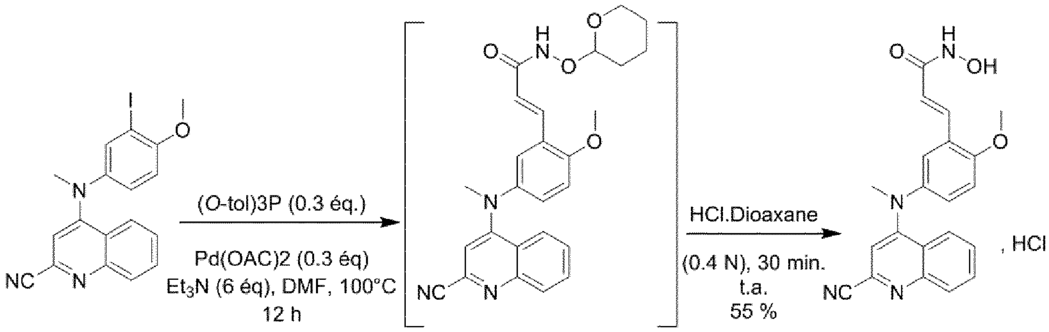


Dans un tube scellé et purgé à l'argon, sont rajoutés le 4-((3-iodo-4-methoxyphenyl)(methyl)amino)quinoline-2-carbonitrile tel que préparé dans l'exemple 2 ci-dessus (30,0 mg, 0,072 mmoles), le N-((tetrahydro-2H-pyran-2-yl)oxy)acrylamide (34,0 mg, 0,20 mmoles), du tri(o-tolyl)phosphine (7,20 mg, 0,023 mmoles), du $Pd(OAC)_2$ (6,0 mg, 0,026 mmoles), du $EtN_3$ (49,0 mg, 0,48 mmoles) et du DMF (1mL). Le mélange est dégazé et chauffé une nuit à 100 °C.

Le milieu réactionnel est ramené à température ambiante dilué avec du DCM et ensuite filtré sur célite, le filtrat est évaporé. La formation du composé intermédiaire (cf. schéma ci-dessus) est confirmée par LC/MS, le composé est utilisé dans l'étape suivante sans purification.

Sur une solution du composé intermédiaire (cf. schéma ci-dessus) dans du dioxane anhydre (1 mL), est rajoutée une solution de HCl dans du dioxane 4N (0,5 mL). Le mélange est agité pendant 30 minutes à température ambiante, puis évaporé à température ambiante et purifié par HPLC en utilisant un gradient d'acétonitrile dans l'eau.

Après lyophilisation, le composé (E)-3-(5-((2-cyanoquinolin-4-yl)(methyl)amino)-2-methoxyphenyl)-N-hydroxyacrylamide est obtenu sous forme d'un solide jaune avec un rendement de 55 %.

Caractérisation $^1H$ RMN (300 MHz, MeOD) δ : 7,94 (d, J = 8,2 Hz, 1H) ; 7,82 - 7,48 (m, 3H) ; 7,44 (s, 1H) ; 7,29 (s, 2H) ; 7,07 (dd, J = 29,0 ; 8,9 Hz, 2H) ; 6,42 (d, J = 17,0 Hz, 1H) ; 4,85 (pic correspondant au sel d'hydrochlorure

recoupé avec l'eau), 3,90 (s, 3H) ; 3,53 (s, 3H).

Caractérisation $^{13}C$ RMN (75 MHz, MeOD) $\delta$ : 165,03 ; 155,74 ; 154,66 ; 149,31 ; 143,14 ; 134,58 ; 133,72 ; 130,06 ; 129,10 ; 126,58 ; 125,39 ; 124,73 ; 123,80 ; 122,40 ; 118,50 ; 117,41 ; 112,46 ; 110,98 ; 55,02 ; 42,86.

Caractérisation HRMS ($ESI^+$): m/z $C_{21}H_{19}N_4O_3$ $[M+H]^+$ 375,1457 (calculée) ; 375,1455 (trouvée).

Caractérisation IR (neat) : 3213, 1567, 1492, 1427, 1241, 1105, 977, 762 $cm^{-1}$.

Pureté (HPLC) : 100 %.

Point de fusion = 215 - 210 °C.

**Exemple 6: Comparaison des activités antiprolifératives de composés de l'invention (référencés « ICON ») en format libre non conjugués sur des lignées cellulaires cancéreuses**

Les activités $CI_{50}$ (ou « $IC_{50}$ » en anglais) des composés selon l'invention 4-((3-amino-4-methoxyphenyl)(methyl)amino) quinoline-2-carbonitrile (ICQN-1), 5-(5-((2-cyanoquinolin-4-yl)(methyl)amino)-2-methoxyphenyl)-N-hydroxypent-4-ynamide (ICQN-2) et (E)-3-(5-((2-cyanoquinolin-4-yl)(methyl)amino)-2-methoxyphenyl)-N-hydroxyacrylamide (ICQN-3) ont été comparées à des composés de l'art antérieur et à un composé de référence commercial et clinique notamment le MMAE (monométhylauristatine E).

Les activités $CI_{50}$ ont été mesurées sur différentes lignées cellulaires cancéreuses HCT116 (tumeur colorectale), A549 (cancer du poumon non à petites cellules), NCI-N87 (tumeur gastrique), Mia-Paca-2 (tumeur du pancréas), K562R (leucémie myéloïde chronique), SKOV3 (tumeur de l'ovaire), SKBr3 (tumeur du sein, surexpression Her2), MCF7 (tumeur du sein) et MDA-MB231 (tumeur du sein).

Les lignées de cellules cancéreuses ont été obtenues auprès de l'American Culture Collection (ATCC, Rockville, MD) ou de la collection Allemande de microorganismes et de cultures cellulaires de l'Institut Leibniz (DSMZ, Braunschweig-Allemagne) ou de la collection Européenne de cultures cellulaires (ECACC, Angleterre). Les lignées cellulaires cancéreuses ont été cultivées selon les instructions du fournisseur.

Le carcinome colorectal humain HCT-116, les carcinomes du sein SK-BR3 et le carcinome ovarien SK-OV-3 ont été cultivés dans du 5A Gibco McCoy supplémenté avec 10 % de sérum de veau foetal (FCS) et 1 % de glutamine.

Les cellules de carcinome pulmonaire A549, de la leucémie myéloïde K562R, et les cellules de carcinome gastrique NCI-N87 ont été cultivées dans du milieu RPMI 1640 de Gibco supplémenté avec 10 % de sérum de veau foetal (FCS) et 1 % de glutamine. Des cellules de carcinome Mia-Paca2 ont été cultivées dans du milieu DMEM de Gibco additionné de 10 % de sérum de veau foetal (FCS) et de 1 % de glutamine.

Les cellules d'adénocarcinome mammaire MDA-MB231 et MCF7 ont été cultivées dans du milieu de culture Gibco RPMI1640 supplémenté avec 10 % de sérum de veau foetal (FCS) et 1 % de glutamine.

Les cellules ont été comptées en utilisant une Vi-cell XR (Beckman Coulter) et leur viabilité a été évaluée par exclusion de colorant bleu trypan à 0,25 %. Ils ont été testés pour la présence de mycoplasmes avant des expériences avec le kit de détection de PCR Mycoplasma (Applied Biological Materials Inc., Canada) conformément aux instructions du fabricant et seules des cellules exemptes de mycoplasmes ont été utilisées pour l'étude. Les lignées cellulaires ont été maintenues à 37 °C dans une atmosphère humidifiée contenant 5 % de $CO_2$.

Pour la détermination de $CI_{50}$, les cellules ont été ensemencées dans des plaques à 96 puits (3 x 103 cellules / puits) contenant 100 $\mu$l de milieu de croissance. Après 24 heures de culture, les cellules ont été traitées avec les composés testés à 10 concentrations finales différentes. Chaque concentration a été obtenue à partir de dilutions en série dans du milieu de culture à partir de la solution mère. Les cellules témoins ont été traitées avec le véhicule. Les expériences ont été réalisées en trois exemplaires.

Les mesures ont été faites après 72 heures de traitement à l'aide du test CellTiter Glo (Promega) qui permet de mesurer par luminescence (quantification de l'ATP) le nombre de cellules vivantes en utilisant un lecteur de microplaques PolarStar Omega (BMG- Labtech).

Les courbes dose-réponse ont été tracées avec le logiciel Graph Prism et les valeurs CI50 ont été calculées en utilisant le logiciel Graph Prism à partir de courbes polynomiales (équations logistiques à quatre ou cinq paramètres).

Les résultats sont indiqués dans le tableau 1 ci-dessous.

Comme le montrent ces résultats, les composés selon l'invention possèdent des très bonnes activités inhibitrices à l'encontre de nombreuses lignées cellulaires cancéreuses.

Tableau 1

| Lignées cellulaires | HCT116 $CI_{50}$ (nM) | A549 $CI_{50}$ (nM) | NCI-N87 $CI_{50}$ (nM) | Mia-Paca-2 $CI_{50}$ (nM) | K562R $CI_{50}$ (nM) | SKOV3 $CI_{50}$ (nM) | SKBr3 $CI_{50}$ (nM) | MCF7 $CI_{50}$ (nM) | MDA-MB231 $CI_{50}$ (nM) |
|---|---|---|---|---|---|---|---|---|---|
| 4-((3-amino-4-methoxyphenyl)(methyl)amino) quinoline-2-carbonitrile ICQN-1 | 0,23 | 0,72 | 0,30 | 0,49 | 0,47 | 0,44 | 0,36 | 0,17 | 0,34 |
| MMAE (GOLD STANDARD) | 1,52 | 4,12 | 5,58 | 3,02 | 19,70 | 0,08 | 0,06 | 0,58 | 1,25 |
| 4-((3-amino-4-ethoxyphenyl)(methyl)amino) quinoline-2-carbonitrile ICQN-2 | / | / | 0,93 | 0,29 | 0.36 | 0.59 | 0.38 | / | / |
| (E)-3-(5-((2-cyanoquinolin-4-yl)(methyl)amino)-2-methoxyphenyl)-N-hydroxyacrylamide ICQN-3 | / | / | 0,10 | 0,94 | 0,56 | 2.64 | 1.78 | 0.78 | 0,44 |

Les résultats de $CI_{50}$ sur les lignées cellulaires HCT116, A549, NCI-N87, Mia-Paca-2, K562R, MCF7 et MDA-MB231 sont globalement bien meilleurs que ceux obtenus avec le composé de référence commerciale et clinique MMAE. Il est à rappeler que le choix des lignées cellulaires est basé sur un large spectre d'indications de cancer solide et liquide représentant un besoin médical non pourvu au sein desquels des anticorps conjugués seraient pertinents. Cette cytotoxicité observée sur un large panel est aussi un critère de succès commercial pour les payloads.

**Exemple 7: Analyse comparative des activités antiprolifératives de composés de l'invention au composé de référence commercial et clinique MMAE (en format non conjugué)**

Le ratio d'$IC_{50}$ a été établi entre un composé de l'invention (ICQN-1) et le MMAE. Plus le ratio d'$IC_{50}$ est élevé, plus important est le potentiel d'amélioration thérapeutique et le potentiel de succès commercial.

Tableau 2

| Lignées cellulaires | HCT1 16 | A549 | NCI-N87 | Mia-Paca-2 | MC F7 | MDA-MB231 |
|---|---|---|---|---|---|---|
| Ratio d'$IC_{50}$ ICQN1/MMAE | 7 | 6 | 22 | 6 | 3 | 4 |

Ainsi, les composés selon l'invention démontrent un avantage très significatif face au MMAE. A titre d'exemple, dans le cadre de la lignée NCI-N87 (cancer gastrique) ICQN1 est 22 fois plus efficace que le MMAE.

**Exemple 8 : Activités antiprolifératives par rapport aux composés de l'art antérieur ayant un noyau quinoléine et un cycle aromatique liés entre eux par un groupe N-Me ( L. Chen. et al. *Eur. J. Med. Chem.* 2017,*138*,1114).**

Dans le cadre des travaux de Chen et al. ci-dessus, parmi les molécules citées, le composé 13b présente la structure suivante :

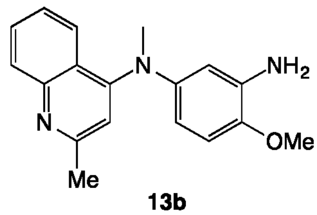

**13b**

$IC_{50}$ (MDA-MB-231) = 3.2 nM

Ce composé 13b montre une $IC_{50}$ de 3,2 nM sur la lignée MDA-MB-231.

Le tableau 3 suivant rassemble des données comparatives sur 7 lignées cellulaires d'un composé selon l'invention (ICQN-1 (4-((3-amino-4-methoxyphenyl) (methyl)amino) quinoline-2-carbonitrile)) et du composé 13b décrit par L. Chen. et al. Eur. J. Med. Chem. 2017, 138, 1114

Tableau 3

| | HCT116 | A549 | NCI-N87 | Mia-Paca2 | K562R | SKOV3 | SKBr3 |
|---|---|---|---|---|---|---|---|
| Composé antérieur 13b selon L. Chen et al. | 2.15 | 0.68 | 4.36 | 0.92 | 3.59 | 1.28 | 2.17 |
| Composé selon l'invention (composé **ICQN1**) | 0.23 | 0.72 | 0.30 | 0.49 | 0.47 | 0.44 | 0.36 |
| Ratio activité 13b vs ICQN-1 | ~10 | 1 | 15 | 2 | 8 | 3 | 6 |

Les composés de l'invention, de manière surprenante, ont une activité cytotoxique globalement largement supérieure à celle du composé 13b. L'activité obtenue suite au remplacement du groupe Me par CN en position 2 de la quinoline est inattendue et ne pouvait être prédite par l'homme de l'art. En effet, selon L. Chen. et al ainsi que l'homme de l'art, le methyl en position 2 de la quinoline était considéré comme nécessaire pour maintenir l'activité d'inhibition. Par ailleurs, selon L. Chen. et al, il n'est fait mention d'aucune conjugaison ou application dans le cadre d'une stratégie ADC.

**Exemple 9 : Activités antiprolifératives par rapport aux composés de l'art antérieur avant une structure de type Dihetéroarylmethylamine (Alami et al.)**

Selon Alami et al. Eur. J. Med. Chem. 2019, 168, 176-188, par comparaison des molécules 1a et 1b (structures ci-dessous), l'homme du métier constatait que remplacer Me par CN en position 2 de la quinoline conduisait à une baisse drastique de l'activité cytotoxique (par un facteur 170 sur la lignée cellulaire HCT116).

**1a**

**0.070 ± 0.001**

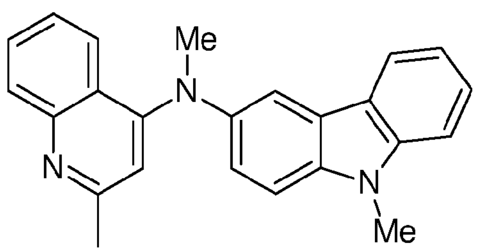

**1b**

**12 ± 0.8**

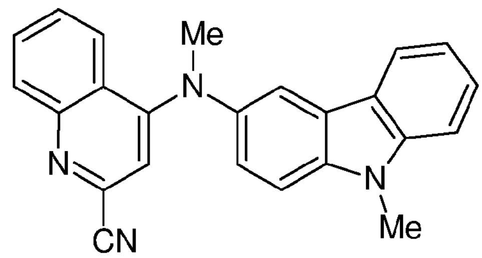

Or, comme supporté par l'exemple 8 ci-dessus, le remplacement d'un Me par CN en position 2 de la quinoline induit une amélioration surprenante de la cytotoxicité (d'un facteur 10). L'homme du métier ne s' attendait pas et n' aurait pu prévoir une telle activité des composés selon la présente invention.

Il convient par ailleurs de noter que le composé **1a** selon Alami et al. ne peut être considéré comme un payload dans le cadre d'une stratégie ADC étant donné qu'il ne possède pas un point d'ancrage pour se lier à un linker.

**Exemple 10 : Activités antiprolifératives par rapport aux composés de l'art antérieur ayant une structure de type Di(hetéroaryl)arylethylène**

Selon Alami et al. J. Med. Chem. 2019, 62, 1902-1916, en comparant deux à deux les molécules **4f/4j** et **4g/4k** portant une double liaison au lieu du groupe N-Me, l'homme du métier aurait constaté que remplacer Me par CN en position 2 de la quinoline ne permettait pas de prédire une amélioration de l'activité cytotoxique $IC_{50}$ en dessous de 1 nM pour la lignée HCT116. L'homme du métier constatait une perte d'activité en passant du composé 4f (Me en position 2) au composé 4j (CN en position 2).

Tableau 4

| | $IC_{50}$ (HCT116) nM |
|---|---|
| **4f** 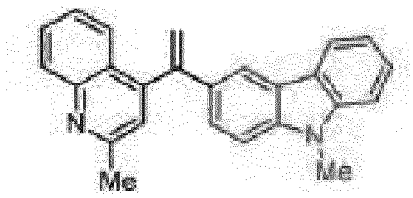 | 2 |
| **4j** 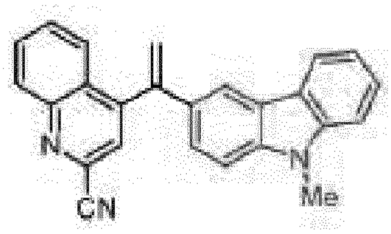 | 12 |
| **4g** 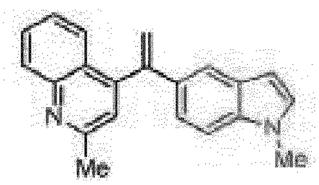 | 5.7 |
| **4k** 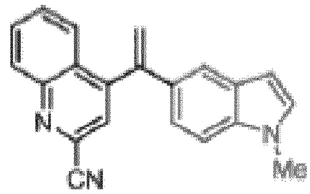 | 3.4 |

**Exemple 11: Analyse comparative des activités antiprolifératives d'un composé d'invention par rapport au payload antérieur ICQO-1**

Sur l'ensemble des composés identifiés au sein de l'art antérieur, ICQO-1 est le seul à présenter la caractéristique d'un payload selon l'homme de l'art. ICQO est un composé de la demande PCT/EP2018/058168 ayant la structure suivante :

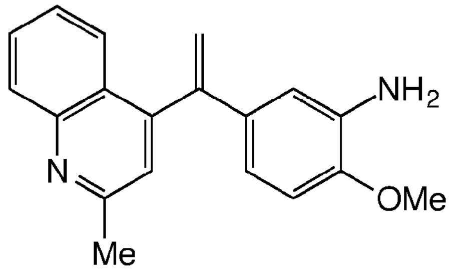

Le ratio d'$IC_{50}$ a été établi entre un composé de l'invention (ICQN-1) et le composé antérieur ICQO-1. Plus le ratio est élevé, plus important est la potentielle activité thérapeutique *in vivo.*

Tableau 5

| Lignées cellulaires | HCT-116 | SKOV-3 |
|---|---|---|
| ICQN-1 vs ICQO-1 | 44 | 3 |

Les ratios indiquent que les composés de l'invention (ICQN) démontrent un avantage très significatif face aux composés de type ICQO-1. A titre d'exemple, dans le cadre de la lignée HCT-116 (cancer colo-rectal) ICQN-1 est 44 fois plus efficace que le composé payload ICQO-1. Cette différence significative était particulièrement imprévisible et inattendue pour l'homme de l'art.

**Exemple 12 - Analyse comparative des activités antiprolifératives de ICON par rapport à des composés antérieurs de type quinoleines** (demande US 2006/074187, exemple 21)

VB118 est un composé quinoléine (demande US 2006/074187, exemple 21) décrit qui a été synthétisé en vue d'être comparé avec des composés selon l'invention.

Tableau 6

| Lignées cellulaires | **ICQN2** | **ICQN1** | **VB118** |
|---|---|---|---|
| NCI-N87 | 0.93 | 0.30 | > 100 nM |
| Mia-Paca-2 | 0.29 | 0.49 | > 100 nM |
| K562R | 0.36 | 0.47 | > 100 nM |
| SKOV-3 | 0.59 | 0.44 | > 100 nM |
| SKBR3 | 0.38 | 0.36 | > 100 nM |

L'addition de la fonction nitrile et de la fonction amine -NH2 en position 2 de la quinoline sur les composés selon l'invention (ICQN), par rapport aux composés antérieur du type VB118, induit une augmentation surprenante, supérieure d'au moins 2 Log d'activité, sur un large panel de lignées cellulaires et d'indications cancéreuses.

**Exemple 13: Analyse comparative des activités antiprolifératives de composés de l'invention (ICQN) par rapport au composé de référence commercial MMAE et au payload ICQO-1 sur les cellules cancéreuses résistantes (profile MDR :Multi Drug Resitance)**

Les cellules K562R (leucémie myéloïde chronique) ont un profile MDR leur permettant de résister à plusieurs composés chimio-thérapeutiques dont le composé de référence MMAE.

Tableau 7

| | K562R (PgP+) | Ratio vs MMAE | Ratio vs ICQO-1 |
|---|---|---|---|
| MMAE | 35 nM | | |
| ICQO-1 | 1.98 nM | 17 | |
| ICQN-1 | 0.47 nM | 74 | 4.2 |

L'homme de l'art dans le domaine des payloads et médicament conjugués constate qu'un payload tel que le MMAE avec un IC50 de 35 nM a moins d'intérêt thérapeutique qu'un composé selon l'invention. Un payload, pour être efficace une fois conjugué a de préférence une activité sub-nanomolaire sur la lignée cancéreuse ciblée. On constate que le composé selon l'invention ICQN-1 présente non seulement une supériorité 74 fois plus importante que le MMAE mais aussi 4.2 fois plus que le composé ICQO-1. Les composés selon l'invention présente une activité particulièrement surprenante mais aussi présente une différenciation thérapeutique pour des cancers possédant un profil de multi-résistance (MDR).

## Exemple 14 - Préparation des Anticorps conjugués

Différents composés « Toxic-Linker » (à base de valine-citruline, clivables par voie enzymatique Cathepsine B) et non clivables ont été obtenus comme illustré dans le tableau 8 suivant :

Tableau 8

| | |
|---|---|
| **Mal-Val-Cit-PABC-ICQN-1 (clivable)** (VB179) | 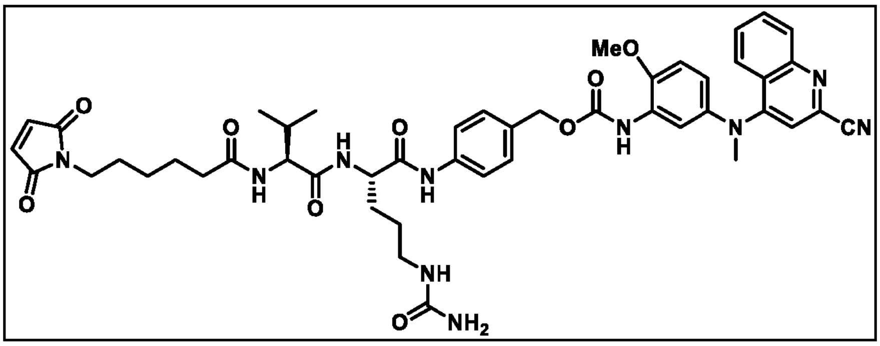 |
| Mal-**Val-Cit-PABC-ICQO-1** (VB185) | 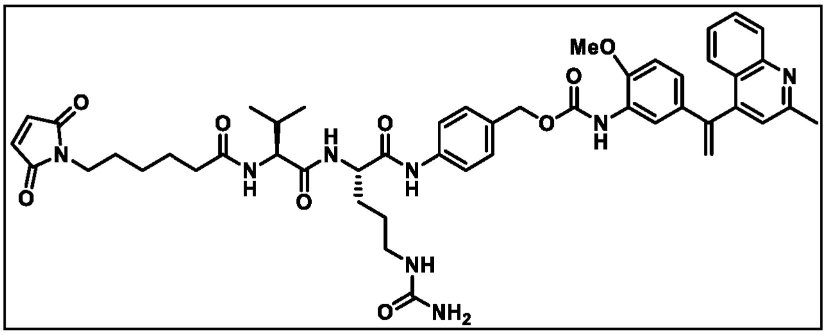 |
| **Mal-PEG4-Val-Cit-PABC-ICQN-1** (VB199) | 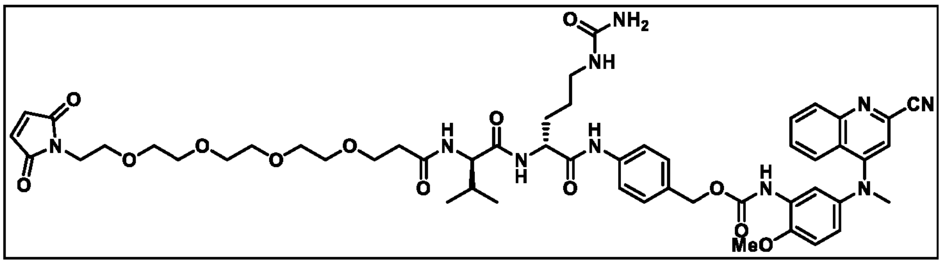 |
| **Mal-PEG4-Val-Cit-PABC-ICQO-1** (VB279 & VB288) | 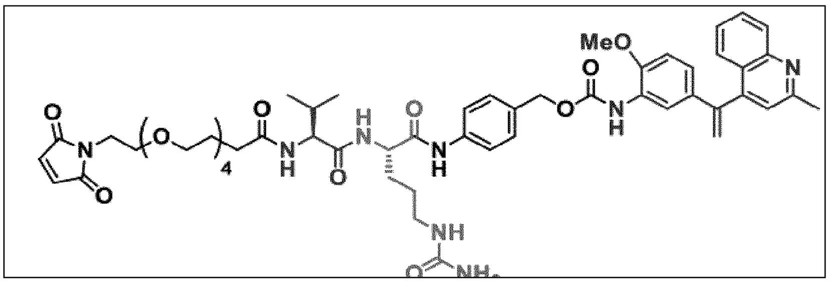 |

(suite)

| | |
|---|---|
| Mal-**Val-Cit-PABC-NI313** (VB277) | 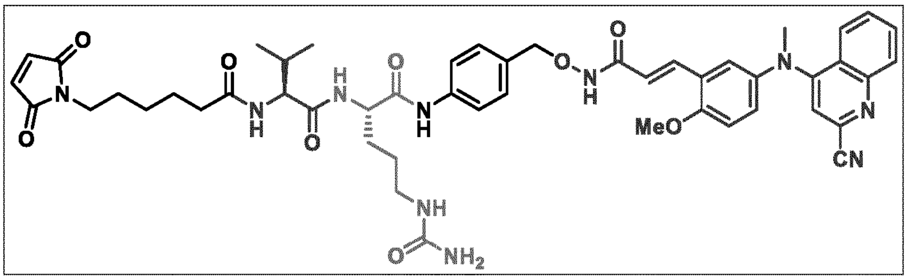 |
| Mal-**ICQN-1** (**non clivable**) (VB284) | 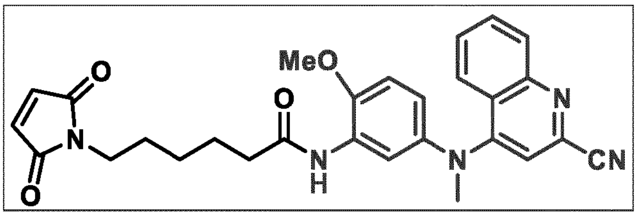 |
| MCC-**ICQN-1** (**non clivable**) (VB289) | 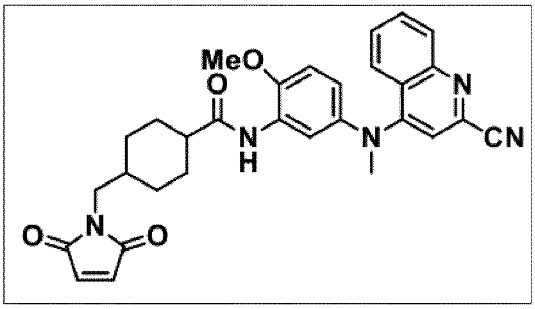 |

Les composés VB179, VB185, VB199 et VB279 ont été obtenus par activation de la fonction NH2 de composés de formule (I) selon l'invention (ICQN-1) par le chloroformiate de paranitrophényle. L'intermédiaire formé réagit ensuite avec la fonction alcool benzylique du linker MC-Val-Cit-PABA.

Le composé VB277 a été préparé par activation de la fonction alcool benzylique du linker MC-Val-Cit-PABA sous forme triflate puis réaction en milieu basique avec le composé NI313

Les composés VB284 et VB289 ont été préparés par couplage peptidique entre ICQN-1 et une fonction acide.

**VB179** :

$^1$H NMR (400 MHz, DMSO-$d_6$) $\delta$: 8.58 (s, 1H), 8.06 (d, $J$ = 7.3 Hz, 1H), 7.96 (d, $J$ = 7.8 Hz, 1H), 7.79 (d, $J$ = 8.4 Hz, 1H), 7.67 (t, $J$ = 7.6 Hz, 1H), 7.60 (s, 1H), 7.58 (s, 2H), 7.56 (s, 1H), 7.49 (dd, $J$ = 8.6, 1.1 Hz, 1H), 7.34 (m, 1H), 7.31 (s, 1H), 6.98 (s, 2H), 6.94 (d, $J$ = 8.8 Hz, 1H), 6.74 (dd, $J$ = 8.7, 2.7 Hz, 1H), 5.97 (t, $J$ = 5.8 Hz, 1H), 5.40 (s, 2H), 5.00 (s, 2H), 4.38 (td, $J$ = 8.2, 5.5 Hz, 1H), 4.18 (dd, $J$ = 8.3, 7.1 Hz, 1H), 3.77 (s, 3H), 3.43 (s, 3H), 2.97 (m, 2H), 2.15 (m, 2H), 1.97 (dq, $J$ = 13.5, 6.7 Hz, 1H), 1.70 (m, 1H), 1.57 (m, 1H), 1.52 - 1.47 (m, 2H), 1.45 (d, $J$ = 7.3 Hz, 2H), 1.42 - 1.30 (m, 2H), 1.23 - 1.14 (m, 2H), 0.85 (d, J = 6.7 Hz, 3H), 0.82 (d, $J$ = 6.7 Hz, 3H). HRMS (ESI): m/z [M + H] calcd. for $C_{47}H_{55}N_{10}O_9$: 903,4153 Found: 903,4156. Purity: 98.2%

**VB 199** :

$^1$H NMR (400 MHz, DMSO-$d_6$) $\delta$: 8.58 (s, 1H), 8.06 (d, $J$ = 7.3 Hz, 1H), 7.96 (d, $J$ = 7.8 Hz, 1H), 7.79 (d, $J$ = 8.4 Hz, 1H), 7.67 (t, $J$ = 7.6 Hz, 1H), 7.60 (s, 1H), 7.58 (s, 2H), 7.56 (s, 1H), 7.49 (dd, $J$ = 8.6, 1.1 Hz, 1H), 7.34 (m, 1H), 7.31 (s, 1H), 6.98 (s, 2H), 6.94 (d, $J$ = 8.8 Hz, 1H), 6.74 (dd, $J$ = 8.7, 2.7 Hz, 1H), 5.97 (t, $J$ = 5.8 Hz, 1H), 5.40 (s, 2H), 5.00 (s, 2H), 4.38 (td, $J$ = 8.2, 5.5 Hz, 1H), 4.18 (dd, $J$ = 8.3, 7.1 Hz, 1H), 3.77 (s, 3H), 3,62 - 3,47 (m, 10 2H), 3.43 (s, 3H), 2.97 (m, 2H), 1.97 (dq, $J$ = 13.5, 6.7 Hz, 1H), 1.70 (m, 1H), 1.57 (m, 1H), 1.42 - 1.30 (m, 2H), 0.85 (d, J = 6.7 Hz, 3H), 0.82 (d, $J$ = 6.7 Hz, 3H). HRMS (ESI): m/z [M + H] calcd. for $C_{52}H_{65}N_{10}O_{13}$: 1037.4733 Found: 1037.4727. Purity:

96.0%

**VB277**

HRMS (ESI): m/z [M + H] calcd. for $C_{49}H_{57}N_{10}O_9$: 929.4310 Found: 929.4312.

**VB284**

$^1$H NMR (300 MHz, $CDCl_3$) $\delta$: 8.48 (d, *J* = 1.9 Hz, 1H), 8.04 (d, *J* = 9.1 Hz, 1H), 7.83 (s, 1H), 7.73 - 7.55 (m, 2H), 7.21 (s, 1H), 6.74 (d, *J* = 8.7 Hz, 1H), 6.71 (s, 2H), 6.53 (d, *J* = 9.6 Hz, 1H), 3.90 (s, 2H), 3.57 (m, 2H), 3.53 (s, 3H), 2.43 (t, *J* = 7.3 Hz, 2H), 1.76 (m, 4H), 1.42 (m, 2H). HRMS (ESI): m/z [M + H] calcd. for $C_{28}H_{28}N_5O_4$: 498.2141 Found:498.2140. Purity: 100%

**VB289**

$^1$H NMR (300 MHz, $CDCl_3$) $\delta$: 8.46 (d, *J* = 2.3 Hz, 1H), 8.02 (d, *J* = 8.8 Hz, 1H), 7.86 (s, 1H), 7.70 - 7.54 (m, 2H), 7.34 - 7.23 (m, 1H), 7.18 (s, 1H), 6.71 (s, 2H), 6.45 (dd, *J* = 8.6, 2.5 Hz, 1H), 3.87 (s, 3H), 3.46 (s, 3H), 3.40 (d, *J* = 6.6 Hz, 2H), 2.30 - 2.16 (m, 1H), 2.10 - 1.97 (m, 2H), 1.87 - 1.76 (m, 2H), 1.54 (ddd, *J* = 25.0, 13.1, 3.0 Hz, 2H), 1.16 - 0.98 (m, 2H), 0.88 (m, 1H). HRMS (ESI): m/z [M + H] calcd. for 524.2298 $C_{30}H_{30}N_5O_4$: Found: 524.2300. Purity: 93%
Les anticorps conjugués sont obtenus selon le protocole décrit exemple 9 de la demande PCT/EP2018/058168, (paragraphe 158 en particulier).

**Exemple 15: Evaluation des activités des anticorps conjugués obtenus**

Un composé selon l'invention (**ICQN-1**) a été couplé à différents linker puis les toxic linker ont été conjugués à l'anticorps Trastuzumab en vue d'obtenir préparer différents ADC. Parmi les ADC préparés, l'ADC suivant **Trastuzumab-Mal-PEG4-Val-Cit-PABC-ICQN-1** en DAR4 a montré in vitro des activités nanomolaires sur la lignée carcinome gastrique NCI-N87 ($IC_{50}$ = 9 nM). A titre de comparaison, l'ADC de référence avec le payload de référence MMAE : **Trastuzumab-Mal-Val-Cit-PABC-MMAE** a montré une activité cytotoxique quasiéquivalente ($IC_{50}$ de 6 nM).

## Revendications

**1.** Composé de formule (I) :

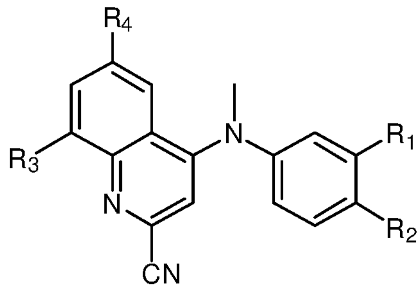

(I)

dans laquelle :

- $R_1$ représente un groupe $-NH_2$, $-NHCOR_a$, $-NHCOOR_b$, $-(C_2-C_6)$alcénylène-CO-NH-OH, $-(C_2-C_6)$alcynylène-CO-NH-OH ou -OH ;
- $R_2$ représente un groupe $-OCH_3$, $-OCH_2CH_3$, $-SCH_3$, $-SCH_2CH_3$ ou $-OCHF_2$ ;
- $R_3$ représente un atome d'hydrogène ou un groupe $-CH_3$, -CN, -F, -Cl ou $-OR_c$ ;
- $R_4$ représente un atome d'hydrogène ou un groupe $-CH_3$, -CN, -F, -Cl ou $-OR_d$ ;
- $R_a$ représente un groupe $-(C_1-C_5)$alkyle ou $-CF_3$ ;
- $R_b$ représente un groupe $-(C_1-C_5)$alkyle ou $-CF_3$ ;
- $R_c$ représente un groupe $-(C_1-C_5)$alkyle ou $-CF_3$ ; et
- $R_d$ représente un groupe $-(C_1-C_5)$alkyle ou $-CF_3$ ;

à l'état de base ou d'acide ou de sels d'acides ou de bases ou sous forme d'hydrate ou de solvate.

**2.** Composé selon la revendication 1, **caractérisé en ce que** $R_1$ représente un groupe $-NH_2$, $-NHCOR_a$, $-NHCOOR_b$, ou -OH, et/ou $R_2$ représente un groupe $-OCH_3$, $-OCH_2CH_3$, ou $-OCHF_2$, et/ou $R_3$ représente un atome d'hydrogène et/ou $R_4$ représente un atome d'hydrogène.

**3.** Composé selon l'une quelconque des revendications 1 à 2, **caractérisé en ce qu'**il est de formule (Ia) :

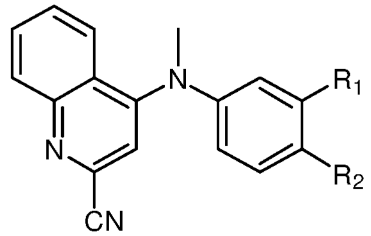

(Ia)

dans laquelle :

- $R_1$ représente un groupe $-NH_2$, $-NHCOR_a$, $-NHCOOR_b$, $-(C_2-C_6)$alcénylène-CO-NH-OH, $-(C_2-C_6)$alcynylène-CO-NH-OH ou -OH ;
- $R_2$ représente un groupe $-OCH_3$, $-OCH_2CH_3$, $-SCH_3$, $-SCH_2CH_3$ ou $-OCHF_2$ ;
- $R_a$ représente un groupe $-(C_1-C_5)$alkyle ou $-CF_3$ ; et
- $R_b$ représente un groupe $-(C_1-C_5)$alkyle ou $-CF_3$ ;

à l'état de base ou d'acide ou de sels d'acides ou de bases ou sous forme d'hydrate ou de solvate.

**4.** Composé selon l'une quelconque des revendications 1 à 3, **caractérisé en ce qu'**il est choisi parmi :

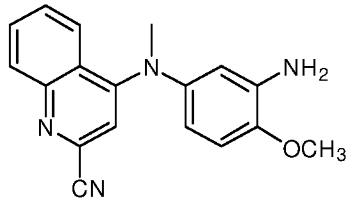

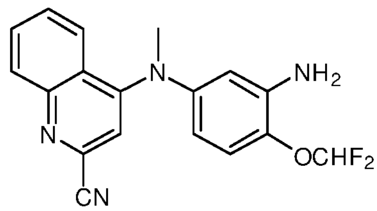

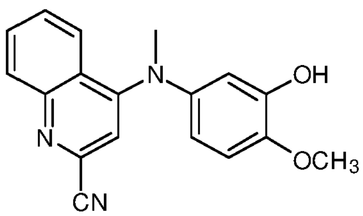

et

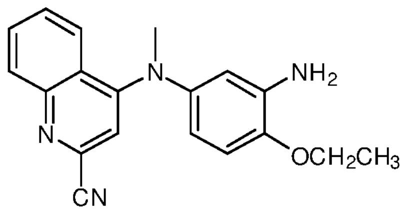

**5.** Composé selon la revendication 1, **caractérisé en ce qu'**il est de formule (Ib) :

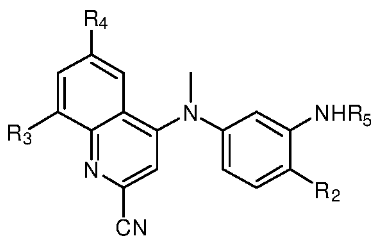

(Ib)

dans laquelle :

- $R_5$ représente un atome d'hydrogène, un groupe $-COR_a$, ou $-COOR_b$ ;
- $R_2$ représente un groupe $-OCH_3$, $-OCH_2CH_3$, $-SCH_3$, $-SCH_2CH_3$ ou $-OCHF_2$ ;
- $R_3$ représente un atome d'hydrogène ou un groupe $-CH_3$, -CN, -F, -Cl ou $-OR_c$, et de préférence un atome d'hydrogène ;
- $R_4$ représente un atome d'hydrogène ou un groupe $-CH_3$, -CN, -F, -Cl ou $-OR_d$, et de préférence un atome d'hydrogène ;
- $R_a$ représente un groupe $-(C_1-C_5)$alkyle ou $-CF_3$ ;
- $R_b$ représente un groupe $-(C_1-C_5)$alkyle ou $-CF_3$ ;
- $R_c$ représente un groupe $-(C_1-C_5)$alkyle ou $-CF_3$ ; et
- $R_d$ représente un groupe $-(C_1-C_5)$alkyle ou $-CF_3$ ;

à l'état de base ou d'acide ou de sels d'acides ou de bases ou sous forme d'hydrate ou de solvate.

6. Composé selon l'une quelconque des revendications 1 à 3, **caractérisé en ce que** $R_1$ représente un groupe $-(C_2-C_6)$alcénylène-CO-NH-OH ou $-(C_2-C_6)$alcynylène-CO-NH-OH, et de préférence $R_1$ représente un groupe -CH=CH-CO-NH-OH, $-CH=CH-CH_2-CH_2-CO-NH-OH$ ou $-C=C-CH_2-CH_2-CO-NH-OH$.

7. Composé selon la revendication 1, **caractérisé en ce qu'**il est de formule (Ic) :

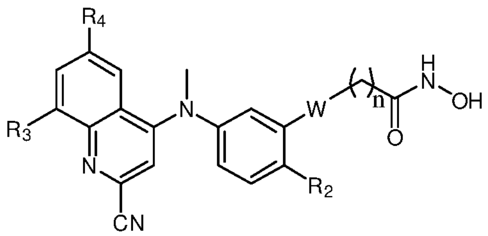

(Ic)

dans laquelle :

- W représente -CH=CH- ou -C=C- ;
- n est 0, 1, 2, 3 ou 4, et de préférence n est 0 ou 2 ;
- $R_2$ représente un groupe $-OCH_3$, $-OCH_2CH_3$, $-SCH_3$, $-SCH_2CH_3$ ou $-OCHF_2$ ;
- $R_3$ représente un atome d'hydrogène ou un groupe $-CH_3$, -CN, -F, -Cl ou $-OR_c$, et de préférence un atome d'hydrogène ;
- $R_4$ représente un atome d'hydrogène ou un groupe $-CH_3$, -CN, -F, -Cl ou $-OR_d$, et de préférence un atome d'hydrogène ;
- $R_c$ représente un groupe $-(C_1-C_5)$alkyle ou $-CF_3$ ; et
- $R_d$ représente un groupe $-(C_1-C_5)$alkyle ou $-CF_3$ ;

à l'état de base ou d'acide ou de sels de bases ou sous forme d'hydrate ou de solvate.

8. Composé selon la revendication 7, **caractérisé en ce qu'**il est choisi parmi :

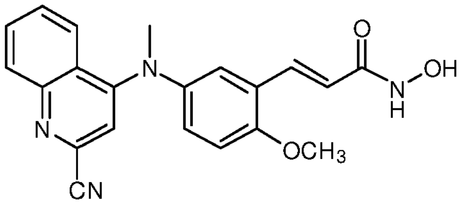

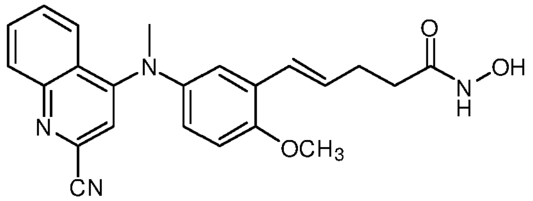

et

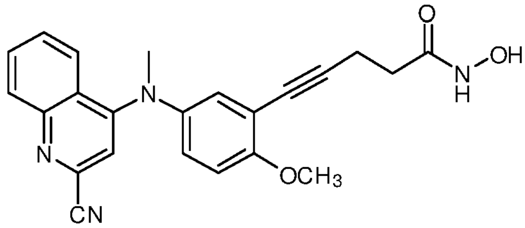

**9.** Procédé de préparation d'un composé selon la revendication 1, dans lequel $R_1$ représente un groupe $-NH_2$ ou -OH, **caractérisé en ce que** :

- on met en présence un composé de formule (B) :

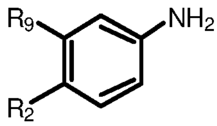

(B)

dans lequel $R_2$ est tel que défini selon la revendication 1 et $R_9$ représente un groupe $-NO_2$ ou -O-Benzoyle ; avec un composé de formule (D) :

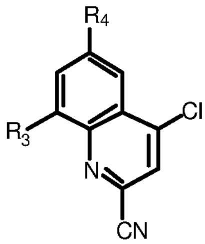

(D)

dans lequel $R_3$ et $R_4$ sont tels que définis selon la revendication 1 ;
pour former par une réaction de substitution nucléophile aromatique, ou par une réaction de couplage en présence d'un catalyseur, en particulier à base de palladium, le composé de formule (E) :

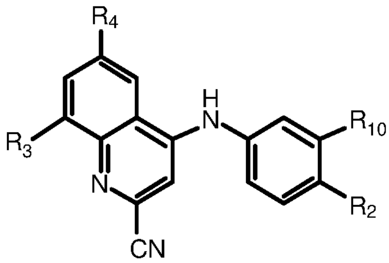

(E)

dans lequel $R_3$, $R_4$ et $R_2$ sont tels que définis selon la revendication 1, et $R_{10}$ représente un groupe $-NO_2$ ou -O-Benzyle ;

- on soumet le composé de formule (E), à une réaction de méthylation puis à une réaction de réduction ou de déprotection pour former le composé de formule (I) :

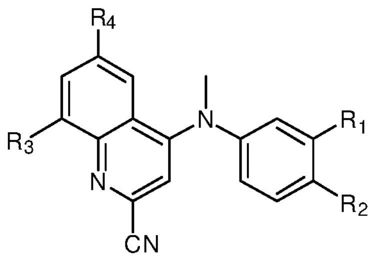

(I)

dans lequel $R_3$, $R_4$ et $R_2$ sont tels que définis selon la revendication 1, et Ri représente un groupe $-NH_2$ ou -OH, $R_a$ et $R_b$ étant tels que définis en revendication 1.

**10.** Procédé de préparation d'un composé (Ic) selon la revendication 7, **caractérisé en ce que** :

- on met en présence le composé de formule (N) :

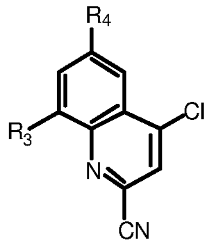

(N)

dans lequel $R_3$ et $R_4$ sont tels que définis selon la revendication 7 ; avec un composé de formule (O):

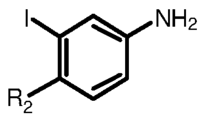

(O)

dans lequel $R_2$ est tel que défini selon la revendication 7, pour former par une réaction de substitution nucléophile aromatique, suivie d'une réaction de méthylation, le composé de formule (P) :

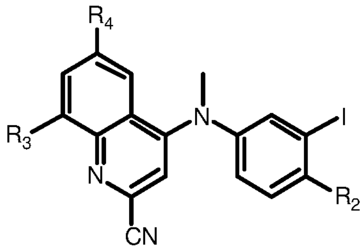

(P)

dans lequel $R_3$, $R_4$ et $R_2$ sont tels que définis selon la revendication 7 ;

- on soumet le composé de formule (P), à une réaction de couplage organométallique avec un groupe -($C_2$-$C_6$)alcénylène-CO-NH-O-(2-tetrahydropyranyle) ou -($C_2$-$C_6$)alcynylène-CO-NH-O-(2-tetrahydropyranyle) puis à une réaction de déprotection pour former le composé de formule (Ic) :

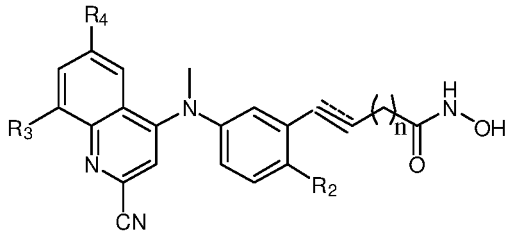

(Ic)

dans laquelle

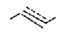

, n, $R_3$, $R_4$ et $R_2$ sont tels que définis selon la revendication 7.

**11.** Composé intermédiaire, **caractérisé en ce qu'**il est choisi parmi :

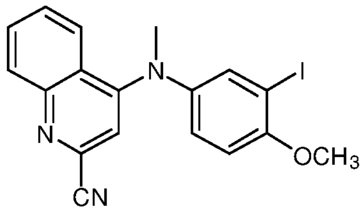

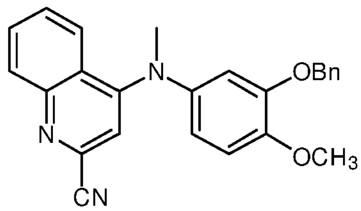

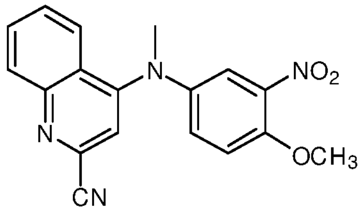

et

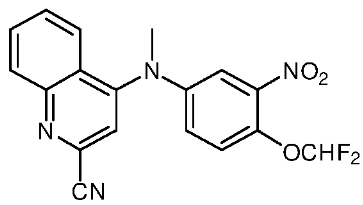

**12.** Utilisation des composés selon la revendication 11, pour la préparation de composés de formule (I) selon la revendication 1.

**13.** Composé de formule (II) :

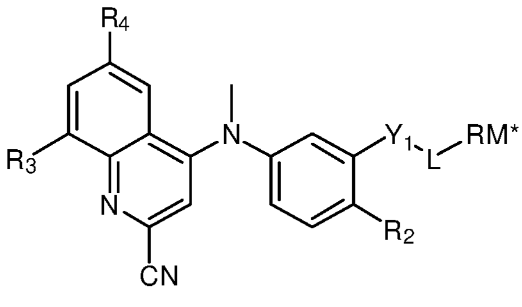

(II)

dans laquelle :

- $Y_1$ représente -O-, -NH-, -NHCO-, -NHCOR$_a$-, -NHCOO-, -NHCOOR$_b$-, -($C_2$-$C_6$)alcénylène-CO-NH-O- ou -($C_2$-$C_6$)alcynylène-CO-NH-O- ;
- $R_2$ représente un groupe $-OCH_3$, $-OCH_2CH_3$, $-SCH_3$, $-SCH_2CH_3$ ou $-OCHF_2$ ;
- $R_3$ représente un atome d'hydrogène ou un groupe $-CH_3$, -CN, -F, -Cl ou $-OR_c$ ; et de préférence un atome d'hydrogène ;
- $R_4$ représente un atome d'hydrogène ou un groupe $-CH_3$, -CN, -F, -Cl ou $-OR_d$ ; et de préférence un atome

d'hydrogène ;

- $R_a$ représente un groupe $-(C_1-C_5)$alkylène- ou $-CF_2-$;
- $R_b$ représente un groupe $-(C_1-C_5)$alkylène- ou $-CF_2-$;
- $R_c$ représente un groupe $-(C_1-C_5)$alkyle ou $-CF_3$ ;
- $R_d$ représente un groupe $-(C_1-C_5)$alkyle ou $-CF_3$ ;
- L représente un agent de liaison ;
- RM* est choisi parmi RM et RM', dans lequel RM est une fonction réactive capable de former une liaison covalente avec une fraction d'un agent de ciblage, en particulier avec une fraction d'un anticorps ou un de ses fragments fonctionnels, et dans lequel RM' est une fraction de RM portant au moins un groupe protecteur ;

à l'état de base ou de sels de bases ou sous forme d'hydrate ou de solvate, et de préférence le groupe L-RM* est choisi parmi :

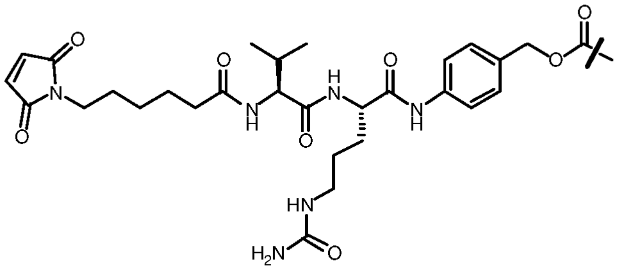

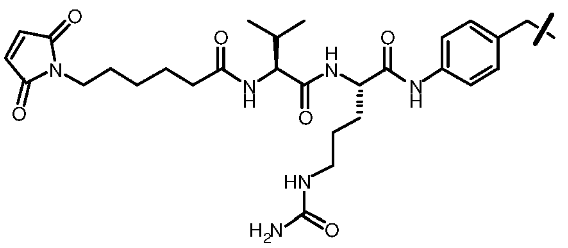

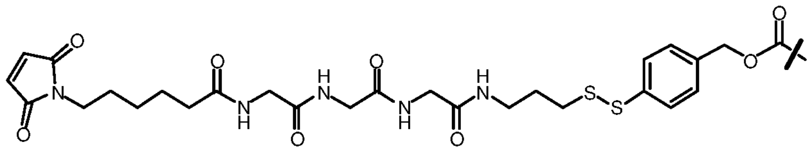

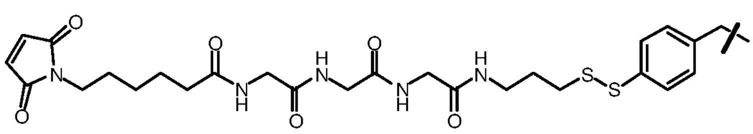

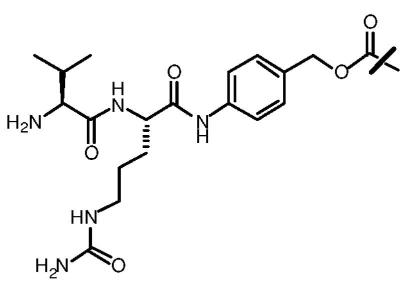

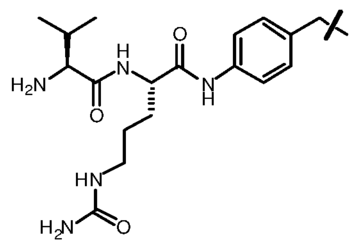

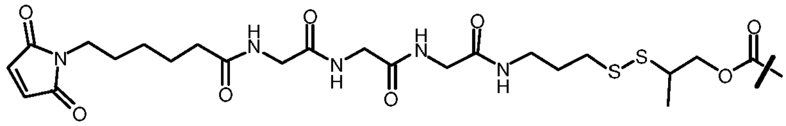

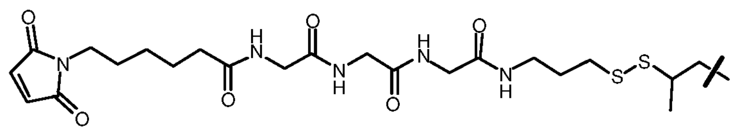

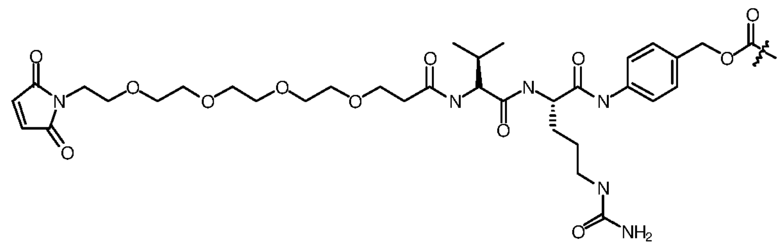

et

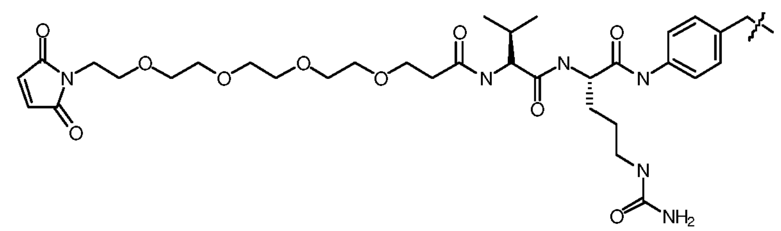

**14.** Composé selon la revendication 13, **caractérisé en ce qu'**il est choisi parmi :

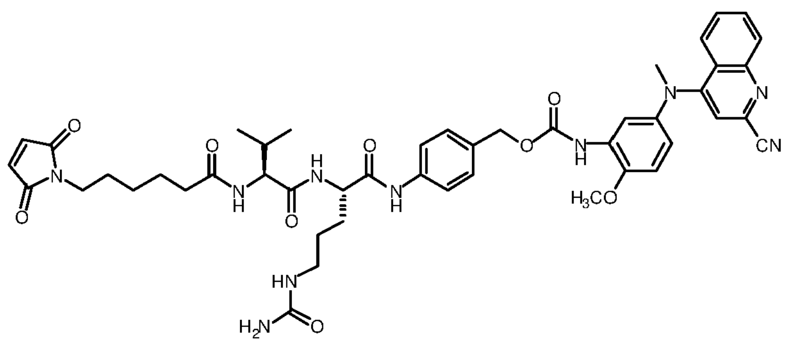

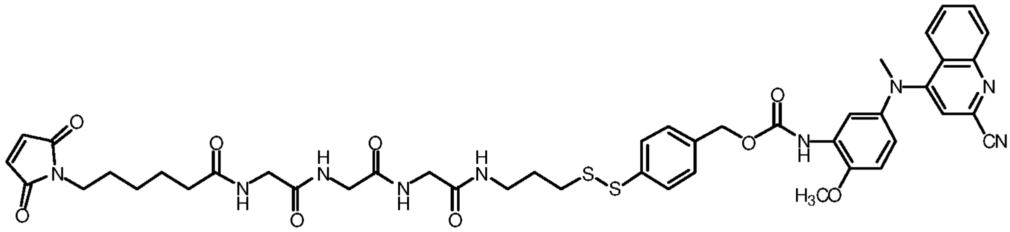

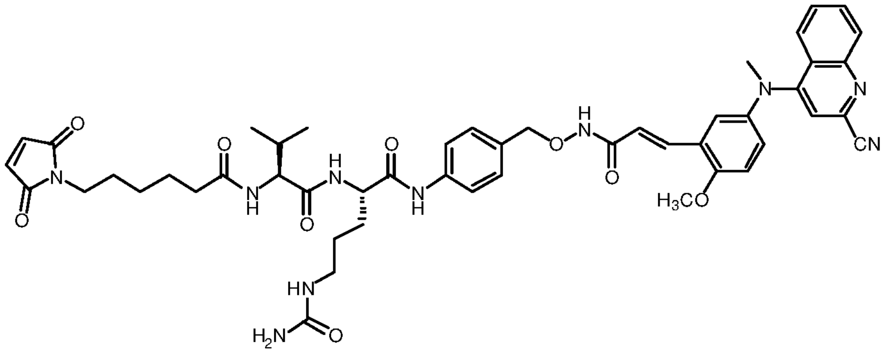

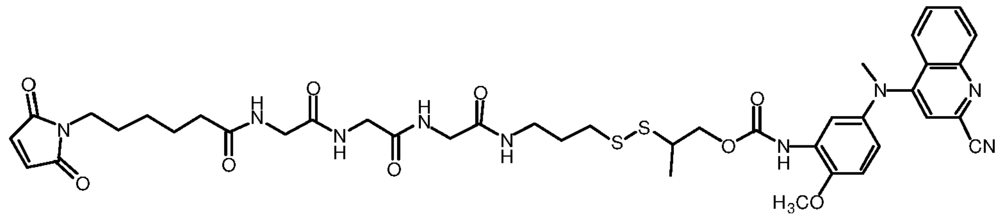

et

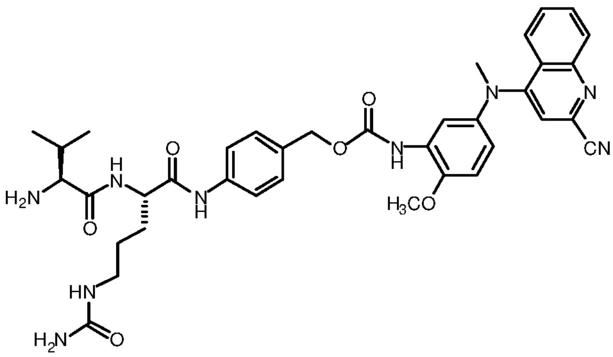

**15.** Conjugué anticorps-médicament de formule (III) :

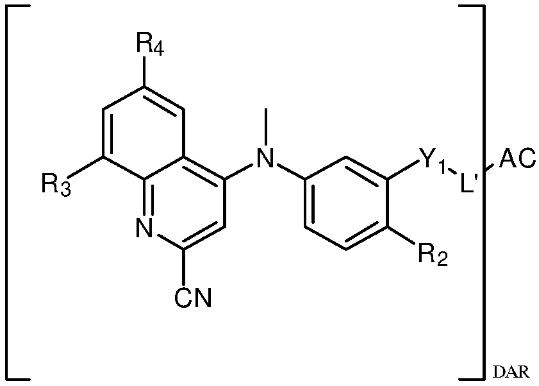

(III)

dans laquelle :

- $Y_1$ représente -O-, -NH-, -NHCO-, -NHCOR$_a$-, -NHCOO-, -NHCOOR$_b$-, -($C_2$-$C_6$)alcénylène-CO-NH-O- ou -($C_2$-$C_6$)alcynylène-CO-NH-O- ;
- $R_2$ représente un groupe $-OCH_3$, $-OCH_2CH_3$, $-SCH_3$, $-SCH_2CH_3$ ou $-OCHF_2$ ;
- $R_3$ représente un atome d'hydrogène ou un groupe $-CH_3$, -CN, -F, -Cl ou -OR$_c$ ; et de préférence un atome d'hydrogène ;
- $R_4$ représente un atome d'hydrogène ou un groupe $-CH_3$, -CN, -F, -Cl ou -OR$_d$ ; et de préférence un atome d'hydrogène ;
- $R_a$ représente un groupe -($C_1$-$C_5$)alkylène- ou $-CF_2$-;
- $R_b$ représente un groupe -($C_1$-$C_5$)alkylène- ou $-CF_2$-;
- $R_c$ représente un groupe -($C_1$-$C_5$)alkyle ou $-CF_3$ ;
- $R_d$ représente un groupe -($C_1$-$C_5$)alkyle ou $-CF_3$ ;
- L' représente un agent de liaison ;
- AC représente une fraction d'un agent de ciblage, en particulier une fraction d'un anticorps ou un de ses fragments fonctionnels ; et

dans laquelle le DAR (ratio médicament/agent de ciblage) varie entre 1 et 8, de préférence entre 2 et 4.

**16.** Procédé de préparation d'un composé de formule (II) selon la revendication 13, **caractérisé en ce qu'**il comprend une étape de réaction d'un composé de formule (I) selon la revendication 1, avec un composé de formule X-L"-RM* dans laquelle :

- X représente un groupe capable de réagir avec un groupe $R_1$ tel que défini en revendication 1 ;
- L" représente un agent de liaison ;
- RM* est choisi parmi RM et RM', dans lequel RM est une fonction réactive capable de former une liaison covalente avec une fraction d'un agent de ciblage, en particulier avec une fraction d'un anticorps ou un de ses fragments fonctionnels, et dans lequel RM' est une fraction de RM portant au moins un groupe protecteur ;

dans lequel la réaction entre la fraction -Ri du composé de formule (I) et le composé de formule X-L"-RM* résulte en la formation d'une fraction -$Y_1$-L-RM*.

**17.** Procédé selon la revendication 16, dans lequel RM* est RM, **caractérisé en ce qu'**il comprend en outre une étape de déprotection d'une fraction RM' résultant en un groupe RM.

**18.** Procédé de préparation d'un composé de formule (III) selon la revendication 15, **caractérisé en ce qu'**il comprend une étape de réaction d'un composé de formule (II) selon la revendication 13 avec une fraction d'un agent de ciblage.

**19.** Composition pharmaceutique comprenant un conjugué anticorps-médicament selon la revendication 15, en association avec un support pharmaceutiquement acceptable.

**20.** Conjugué anticorps-médicament selon la revendication 15 ou une composition pharmaceutique selon la revendication 19, pour son utilisation en tant que médicament, et de préférence pour son utilisation pour tuer ou inhiber la croissance des cellules, ou pour son utilisation dans le traitement du cancer.

**Patentansprüche**

1. Verbindung von Formel (I):

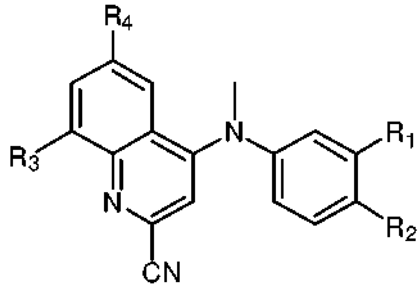

(I)

wobei:

- $R_1$ eine Gruppe $-NH_2$, $-NHCOR_a$, $-NHCOOR_b$, $-(C_2-C_6)$Alkenylen-CO-NH-OH, $-(C_2-C_6)$Alkinylen-CO-NH-OH oder -OH darstellt;
- $R_2$ eine Gruppe $-OCH_3$, $-OCH_2CH_3$, $-SCH_3$, $-SCH_2CH_3$ oder $-OCHF_2$ darstellt;
- $R_3$ ein Wasserstoffatom oder eine Gruppe $-CH_3$, -CN, -F, -Cl oder $-OR_c$ darstellt;
- $R_4$ ein Wasserstoffatom oder eine Gruppe $-CH_3$, -CN, -F, -Cl oder $-OR_d$ darstellt;
- $R_a$ eine Gruppe $-(C_1-C_5)$-Alkyl oder $-CF_3$ darstellt;
- $R_b$ eine Gruppe $-(C_1-C_5)$-Alkyl oder $-CF_3$ darstellt;
- $R_c$ eine Gruppe $-(C_1-C_5)$-Alkyl oder $-CF_3$ darstellt; und
- $R_d$ eine Gruppe $-(C_1-C_5)$-Alkyl oder $-CF_3$ darstellt;

als Base oder Säure oder als Salze von Säuren oder Basen oder in Form eines Hydrats oder Solvats.

2. Verbindung nach Anspruch 1, **dadurch gekennzeichnet, dass** $R_1$ eine Gruppe $-NH_2$, $-NHCOR_a$, $-NHCOOR_b$ oder -OH darstellt, insbesondere $R_1$ eine Gruppe $-NH_2$ oder -OH und vorzugsweise eine Gruppe $-NH_2$ darstellt, und/oder $R_2$ eine Gruppe $-OCH_3$, $-OCH_2CH_3$ oder - $OCHF_2$ und vorzugsweise eine Gruppe $-OCH_3$ darstellt, und/oder $R_3$ ein Wasserstoffatom darstellt und/oder $R_4$ ein Wasserstoffatom darstellt.

3. Verbindung nach einem der Ansprüche 1 bis 2, **dadurch gekennzeichnet, dass** sie von Formel (Ia) ist:

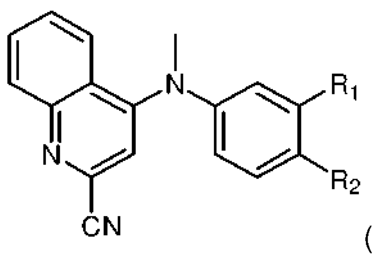

(Ia)

wobei:

- $R_1$ eine Gruppe $-NH_2$, $-NHCOR_a$, $-NHCOOR_b$, $-(C_2-C_6)$Alkenylen-CO-NH-OH, $-(C_2-C_6)$Alkinylen-CO-NH-OH oder -OH darstellt;
- $R_2$ eine Gruppe $-OCH_3$, $-OCH_2CH_3$, $-SCH_3$, $-SCH_2CH_3$ oder $-OCHF_2$ darstellt;
- $R_a$ eine Gruppe $-(C_1-C_5)$-Alkyl oder $-CF_3$ darstellt; und
- $R_b$ eine Gruppe $-(C_1-C_5)$-Alkyl oder $-CF_3$ darstellt;

als Base oder Säure oder als Salze von Säuren oder Basen oder in Form eines Hydrats oder Solvats.

4. Verbindung nach einem der Ansprüche 1 bis 3, **dadurch gekennzeichnet, dass** sie ausgewählt ist aus:

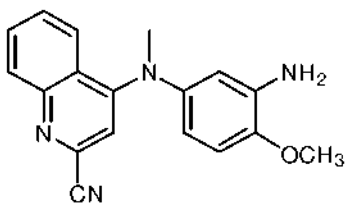
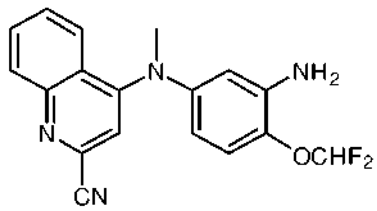

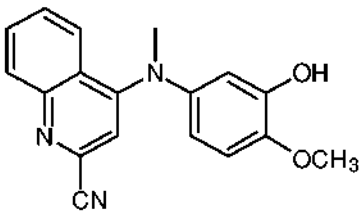

und

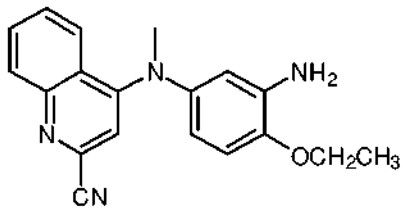

5. Verbindung nach Anspruch 1, **dadurch gekennzeichnet, dass** sie von Formel (Ib) ist:

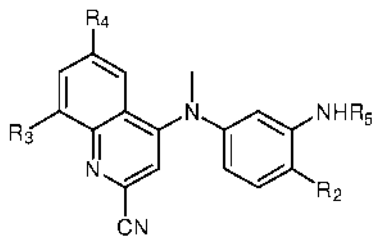

(Ib)

wobei:

- $R_5$ rein Wasserstoffatom, eine Gruppe $-COR_a$ oder $-COOR_b$ darstellt;
- $R_2$ eine Gruppe $-OCH_3$, $-OCH_2CH_3$, $-SCH_3$, $-SCH_2CH_3$ oder $-OCHF_2$ darstellt;
- $R_3$ ein Wasserstoffatom oder eine Gruppe $-CH_3$, -CN, -F, -Cl oder $-OR_c$ und vorzugsweise ein Wasserstoffatom darstellt;
- $R_4$ ein Wasserstoffatom oder eine Gruppe $-CH_3$, -CN, -F, -Cl oder $-OR_d$ und vorzugsweise ein Wasserstoffatom darstellt;
- $R_a$ eine Gruppe $-(C_1-C_5)$-Alkyl oder $-CF_3$ darstellt;
- $R_b$ eine Gruppe $-(C_1-C_5)$-Alkyl oder $-CF_3$ darstellt;
- $R_c$ eine Gruppe $-(C_1-C_5)$-Alkyl oder $-CF_3$ darstellt; und
- $R_d$ eine Gruppe $-(C_1-C_5)$-Alkyl oder $-CF_3$ darstellt;

als Base oder Säure oder als Salze von Säuren oder Basen oder in Form eines Hydrats oder Solvats.

6. Verbindung nach einem der Ansprüche 1 bis 3, **dadurch gekennzeichnet, dass** $R_1$ eine Gruppe $-(C_2-C_6)$-Alkenylen-CO-NH-OH- oder $-(C_2-C_6)$-Alkinylen-CO-NH-OH darstellt, und vorzugsweise $R_1$ eine Gruppe -CH=CH-CO-NH-OH, $-CH=CH-CH_2-CH_2-CO-NH-OH$ ou $-C=C-CH_2-CH_2-CO-NH-OH$ darstellt.

7. Verbindung nach Anspruch 1, **dadurch gekennzeichnet, dass** sie von Formel (Ic) ist:

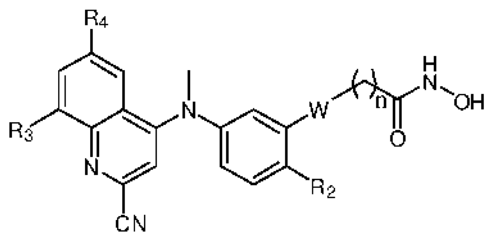

(Ic)

wobei:

- W -CH=CH- oder -C=C- darstellt;
- n 0, 1, 2, 3 oder 4 ist, und vorzugsweise n 0 oder 2 ist;
- $R_2$ eine Gruppe $-OCH_3$, $-OCH_2CH_3$, $-SCH_3$, $-SCH_2CH_3$ oder $-OCHF_2$ darstellt;
- $R_3$ ein Wasserstoffatom oder eine Gruppe $-CH_3$, -CN, -F, -Cl oder $-OR_c$ und vorzugsweise ein Wasserstoffatom darstellt;
- $R_4$ ein Wasserstoffatom oder eine Gruppe $-CH_3$, -CN, -F, -Cl oder $-OR_d$ und vorzugsweise ein Wasserstoffatom darstellt;
- $R_c$ eine Gruppe $-(C_1-C_5)$-Alkyl oder $-CF_3$ darstellt; und
- $R_d$ eine Gruppe $-(C_1-C_5)$-Alkyl oder $-CF_3$ darstellt;

als Base oder Säure oder als Salze von Basen oder in Form eines Hydrats oder Solvats.

**8.** Verbindung nach Anspruch 7, **dadurch gekennzeichnet, dass** sie ausgewählt ist aus:

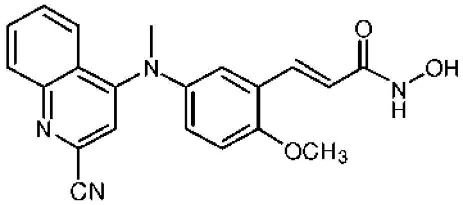

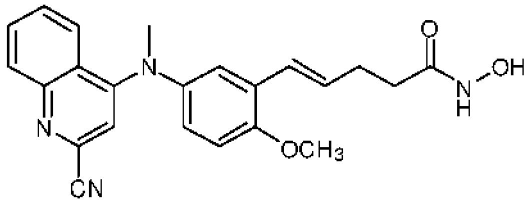

und

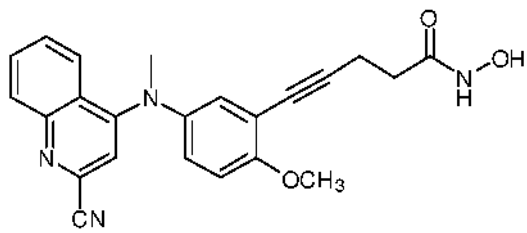

**9.** Verfahren zur Herstellung einer Verbindung nach Anspruch 1, wobei $R_1$ eine Gruppe $-NH_2$ oder -OH darstellt, **dadurch gekennzeichnet, dass**:

- eine Verbindung von Formel (B):

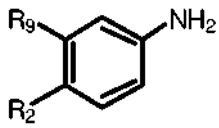

(B)

wobei $R_2$ wie definiert in Anspruch 1 ist und $R_9$ eine Gruppe $-NO_2$ oder -O-Benzoyl darstellt; mit einer Verbindung von Formel (D) zusammengebracht wird:

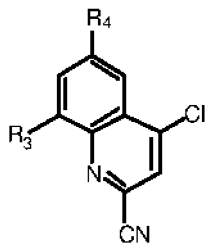

(D)

wobei $R_3$ und $R_4$ wie definiert in Anspruch 1 sind; um durch eine aromatische nucleophile Substitutionsreaktion oder durch eine Kupplungsreaktion in Anwesenheit eines Katalysators, insbesondere auf Palladiumbasis, die Verbindung von Formel (E) zu bilden:

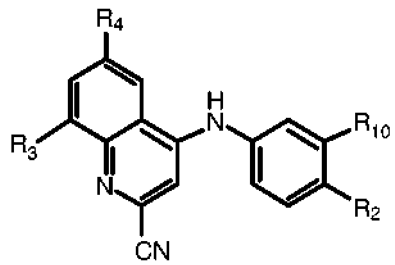

(E)

wobei $R_3$, $R_4$ und $R_2$ wie definiert nach Anspruch 1 sind und $R_{10}$ eine Gruppe $-NO_2$ oder -O-Benzyl darstellt;

- die Verbindung von Formel (E) einer Methylierungsreaktion und anschließend einer Reduktions- oder Entschützungsreaktion unterzogen wird, um die Verbindung von Formel (I) zu bilden:

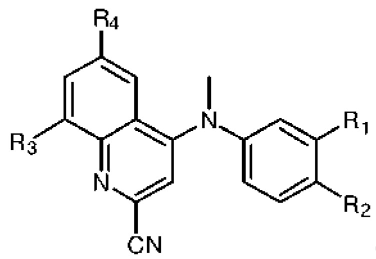

(I)

wobei $R_3$, $R_4$ und $R_2$ wie definiert in Anspruch 1 sind und $R_1$ eine Gruppe $-NH_2$ oder -OH darstellt, wobei $R_a$ und $R_b$ wie definiert in Anspruch 1 sind.

**10.** Verfahren zur Herstellung einer Verbindung nach Anspruch 7, **dadurch gekennzeichnet, dass**:

- die Verbindung von Formel (N):

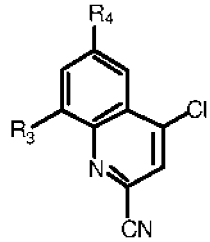

(N)

wobei $R_3$ und $R_4$ wie definiert in Anspruch 7 sind; mit einer Verbindung von Formel (O) zusammengebracht wird:

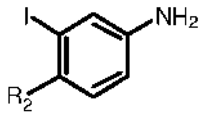

(O)

wobei $R_2$ wie definiert in Anspruch 7 ist, um durch eine aromatische nukleophile Substitutionsreaktion, gefolgt von einer Methylierungsreaktion, die Verbindung von Formel (P) zu bilden:

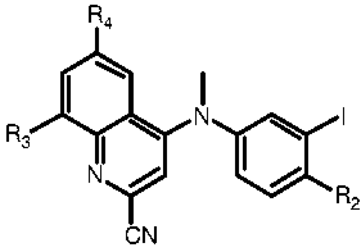

(P)

wobei $R_3$, $R_4$ und $R_2$ wie definiert in Anspruch 7 sind;

- die Verbindung von Formel (P) einer metallorganischen Kupplungsreaktion mit einer Gruppe -($C_2$-$C_6$)-Alkenylen-CO-NH-O-(2-Tetrahydropyranyl) oder -($C_2$-$C_6$)-Alkinylen-CO-NH-O-(2-Tetrahydropyranyl) und anschließend einer Entschützungsreaktion unterzogen wird, um die Verbindung von Formel (Ic) zu bilden:

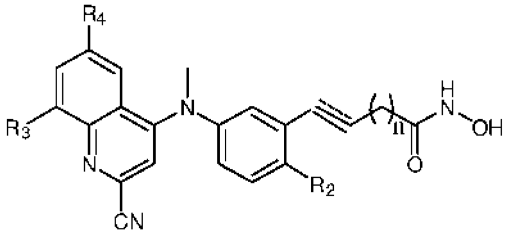

(Ic)

wobei

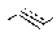

, n, $R_3$, $R_4$ und $R_2$ wie definiert nach Anspruch 7 sind.

**11.** Zwischenverbindung, **dadurch gekennzeichnet, dass** sie ausgewählt ist aus:

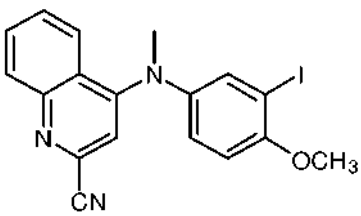

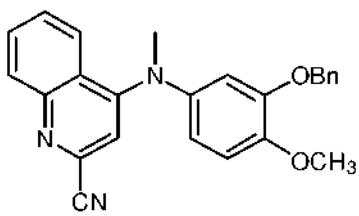

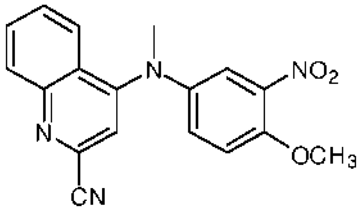

und

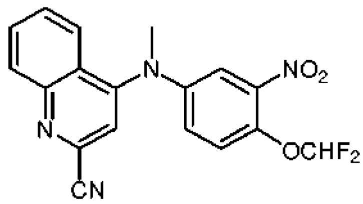

**12.** Verwendung der Verbindungen nach Anspruch 11 zur Herstellung von Verbindungen von Formel (I) nach Anspruch 1.

**13.** Verbindung von Formel (II):

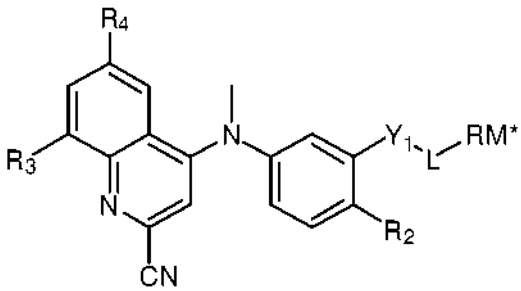

(II)

wobei:

- $Y_1$ -O-, -NH-, -NHCO-, $-NHCOR_a-$, -NHCOO-, $-NHCOOR_b-$, $-(C_2-C_6)$-Alkenylen-CO-NH-O-oder $-(C_2-C_6)$-Alkinylen-CO-NH-O- darstellt;
- $R_2$ eine Gruppe $-OCH_3$, $-OCH_2CH_3$, $-SCH_3$, $-SCH_2CH_3$ oder $-OCHF_2$ darstellt;
- $R_3$ ein Wasserstoffatom oder eine Gruppe $-CH_3$, -CN, -F, -Cl oder $-OR_c$ und vorzugsweise ein Wasserstoffatom darstellt;
- $R_4$ ein Wasserstoffatom oder eine Gruppe $-CH_3$, -CN, -F, -Cl oder $-OR_d$ und vorzugsweise ein Wasserstoffatom darstellt;
- $R_a$ eine Gruppe $-(C_1-C_5)$-Alkylen oder $-CF_2$ darstellt;
- $R_b$ eine Gruppe $-(C_1-C_5)$-Alkylen oder $-CF_2$ darstellt;
- $R_c$ eine Gruppe $-(C_1-C_5)$-Alkyl oder $-CF_3$ darstellt;
- $R_d$ eine Gruppe $-(C_1-C_5)$-Alkyl oder $-CF_3$ darstellt;
- L ein Verbindungsmittel darstellt;
- RM* ausgewählt ist aus RM und RM', wobei RM eine reaktive Funktion ist, die in der Lage ist, eine kovalente Bindung mit einer Fraktion eines Targeting-Mittels, insbesondere mit einer Fraktion eines Antikörpers oder einem funktionellen Fragment davon zu bilden, und wobei RM' eine Fraktion von RM ist, die mindestens eine Schutzgruppe trägt;

in Form von Basen oder Salzen von Basen oder in Form eines Hydrats oder Solvats und wobei vorzugsweise die Gruppe L-RM* ausgewählt ist aus:

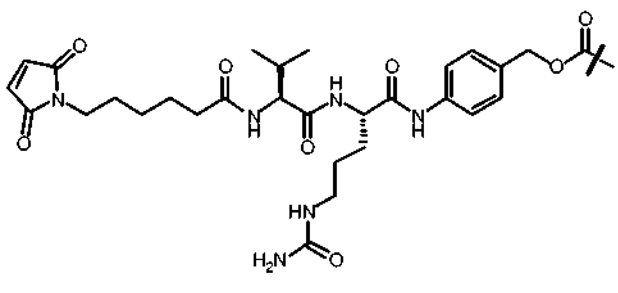

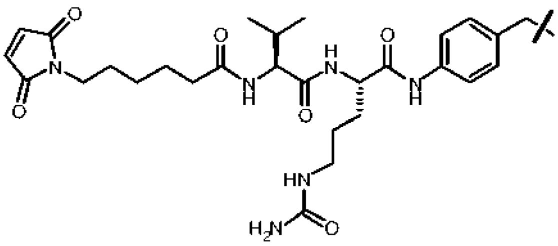

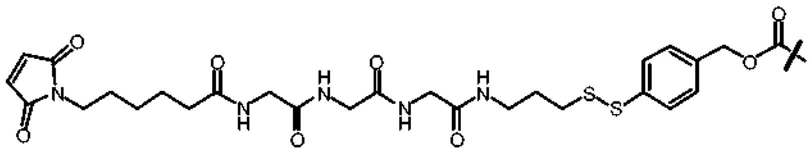

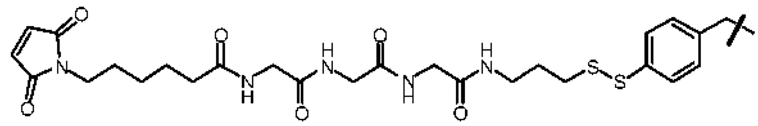

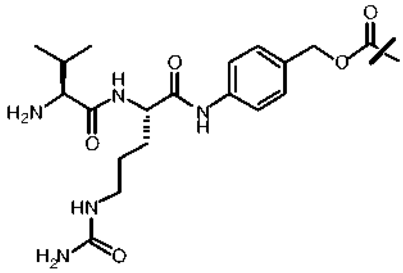

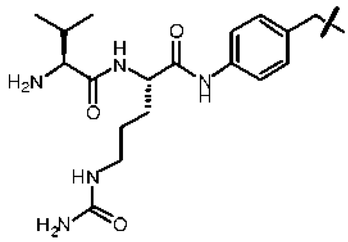

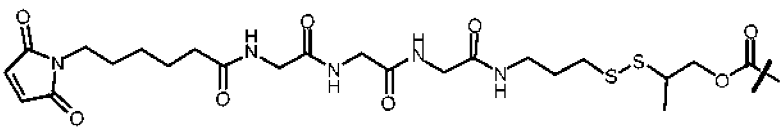

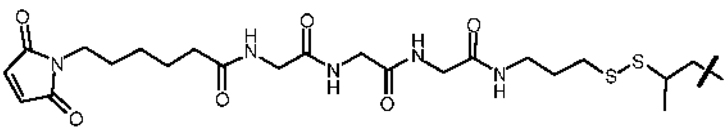

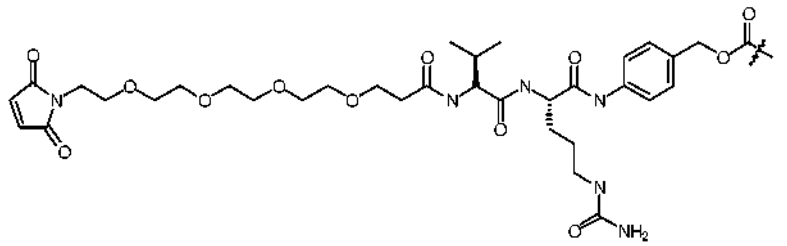

und

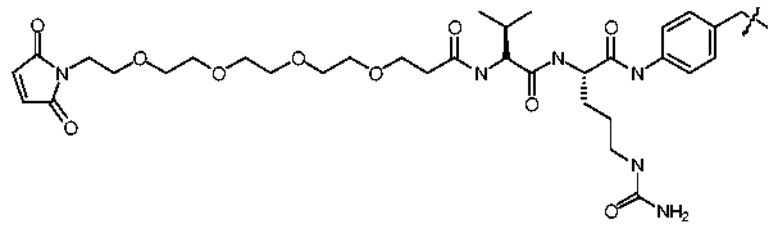

**14.** Verbindung nach Anspruch 13, **dadurch gekennzeichnet, dass** sie ausgewählt ist aus:

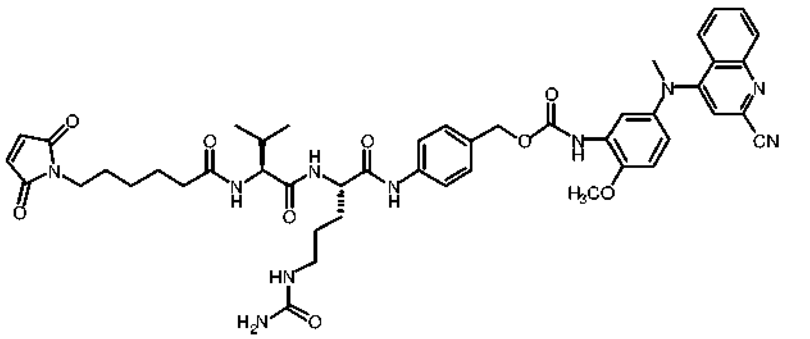

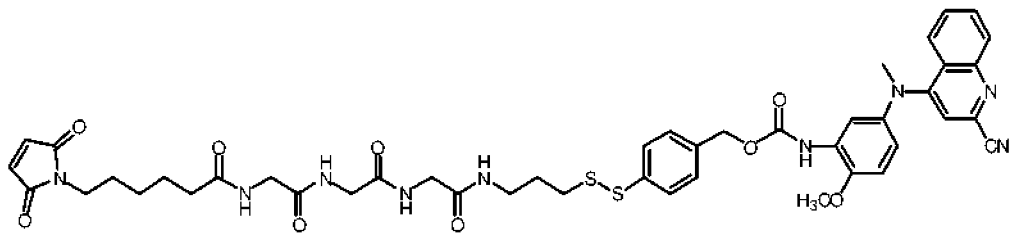

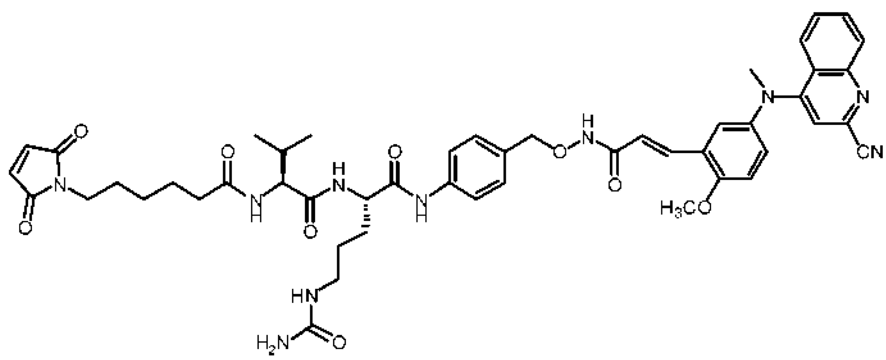

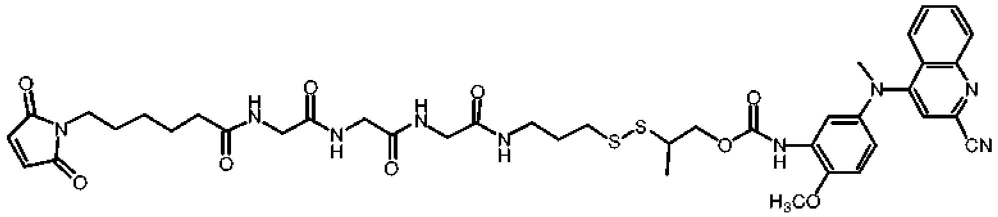

und

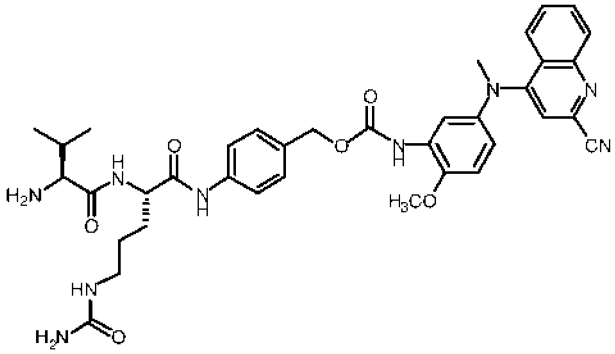

**15.** Antikörper-Medikament-Konjugat von Formel (III):

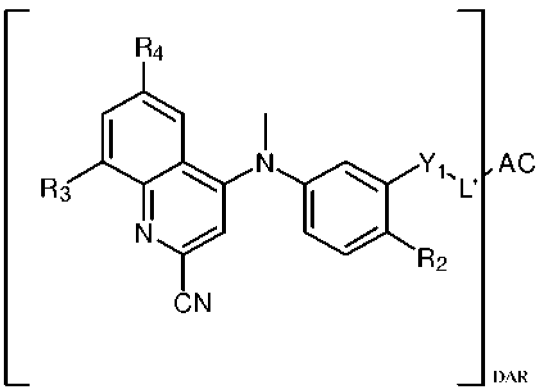

(III)

wobei:

- Y1 -O-, -NH-, -NHCO-, $-NHCOR_a-$, -NHCOO-, $-NHCOOR_b-$, $-(C_2-C_6)$-Alkenylen-CO-NH-O-oder $-(C_2-C_6)$-Alkinylen-CO-NH-O- darstellt;
- $R_2$ eine Gruppe -OCH3, $-OCH_2CH_3$, $-SCH_3$, $-SCH_2CH_3$ oder $-OCHF_2$ darstellt;
- $R_3$ ein Wasserstoffatom oder eine Gruppe $-CH_3$, -CN, -F, -Cl oder $-OR_c$ und vorzugsweise ein Wasserstoffatom darstellt;
- $R_4$ ein Wasserstoffatom oder eine Gruppe $-CH_3$, -CN, -F, -Cl oder $-OR_d$ und vorzugsweise ein Wasserstoffatom darstellt;
- $R_a$ eine Gruppe $-(C_1-C_5)$-Alkylen oder $-CF_2$ darstellt;
- $R_b$ eine Gruppe $-(C_1-C_5)$-Alkylen oder $-CF_2$ darstellt;
- $R_c$ eine Gruppe $-(C_1-C_5)$-Alkyl oder $-CF_3$ darstellt;
- $R_d$ eine Gruppe $-(C_1-C_5)$-Alkyl oder $-CF_3$ darstellt;
- L' ein Verbindungsmittel darstellt;
- AC eine Fraktion eines Targeting-Mittels, insbesondere eine Fraktion eines Antikörpers oder eines funktionellen Fragments davon, darstellt; und

wobei das DAR (Verhältnis von Medikament zu Targeting-Mittel) zwischen 1 und 8, vorzugsweise zwischen 2 und 4, variiert.

**16.** Verfahren zur Herstellung einer Verbindung der Formel (II) nach Anspruch 13, **dadurch gekennzeichnet, dass** es einen Schritt eines Reagierens einer Verbindung von Formel (I) nach Anspruch 1 mit einer Verbindung von Formel X-L"-RM* umfasst, wobei:

- X eine Gruppe darstellt, die in der Lage ist, mit einer Gruppe $R_1$, wie definiert in Anspruch 1 definiert, zu reagieren;
- L" ein Verbindungsmittel darstellt;
- RM* ausgewählt ist aus RM und RM', wobei RM eine reaktive Funktion ist, die in der Lage ist, eine kovalente Bindung mit einer Fraktion eines Targeting-Mittels, insbesondere mit einer Fraktion eines Antikörpers oder einem funktionellen Fragment davon zu bilden, und wobei RM' eine Fraktion von RM ist, die mindestens eine Schutzgruppe trägt;

wobei die Reaktion zwischen der Fraktion $-R_1$ der Verbindung von Formel (I) und der Verbindung von Formel X-L"-RM* in der Bildung einer Fraktion $-Y_1$-L-RM* resultiert.

**17.** Verfahren nach Anspruch 16, wobei RM* RM ist, **dadurch gekennzeichnet, dass** es ferner einen Schritt einer Entschützung einer Fraktion RM' umfasst, was in einer Gruppe RM resultiert.

**18.** Verfahren zur Herstellung einer Verbindung von Formel (III) nach Anspruch 15, **dadurch gekennzeichnet, dass** es einen Schritt umfasst, bei dem eine Verbindung von Formel (II) nach Anspruch 13 mit einer Fraktion eines Targeting-Mittels reagiert wird.

**19.** Pharmazeutische Zusammensetzung, umfassend ein Antikörper-Medikament-Konjugat nach Anspruch 15 in Verbindung mit einem pharmazeutisch annehmbaren Träger.

**20.** Antikörper-Medikament-Konjugat nach Anspruch 15 oder eine pharmazeutische Zusammensetzung nach Anspruch 19 zur Verwendung als Medikament und vorzugsweise zur Verwendung zum Abtöten oder Hemmen des Wachstums von Zellen oder zur Verwendung bei der Behandlung von Krebs.

## Claims

**1.** A compound having the formula (I) :

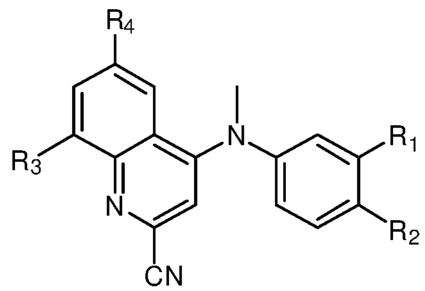

(I)

wherein:

- $R_1$ represents a group: $-NH_2$, $-NHCOR_a$, $-NHCOOR_b$, $-(C_2\text{-}C_6)$alkenylene-CO-NH-OH, - $(C_2\text{-}C_6)$alkynylene-CO-NH-OH or -OH;
- $R_2$ represents a group: $-OCH_3$, $-OCH_2CH_3$, $-SCH_3$, $-SCH_2CH_3$ or $-OCHF_2$;
- $R_3$ represents a hydrogen atom or a group: $-CH_3$, -CN, -F, -Cl or $-OR_c$;
- $R_4$ represents a hydrogen atom or a group: $-CH_3$, -CN, -F, -Cl or $-OR_d$;
- $R_a$ represents a group: $-(C_1\text{-}C_5)$alkyl or $-CF_3$;
- $R_b$ represents a group: $-(C_1\text{-}C_5)$alkyl or $-CF_3$;
- $R_c$ represents a group: $-(C_1\text{-}C_5)$alkyl or $-CF_3$; and
- $R_d$ represents a group: $-(C_1\text{-}C_5)$alkyl or $-CF_3$;

in the following state: base or acid, or salts of acids or salts of bases; or in the form of a hydrate or a solvate.

2. A compound according to claim 1, **characterised in that** $R_1$ represents a group: $-NH_2$, $-NHCOR_a$, $-NHCOOR_b$, or -OH, and/or $R_2$ represents a group: $-OCH_3$, $-OCH_2CH_3$, or $-OCHF_2$, and/or $R_3$ represents a hydrogen atom, and/or $R_4$ represents a hydrogen atom.

3. A compound according to any one of claim 1 or 2, **characterised in that** it has the formula (Ia):

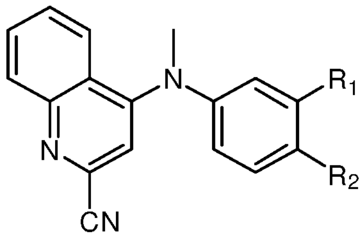

(Ia)

wherein:

- $R_1$ represents a group: $-NH_2$, $-NHCOR_a$, $-NHCOOR_b$, $-(C_2-C_6)$alkenylene-CO-NH-OH, $-(C_2-C_6)$alkynylene-CO-NH-OH or -OH;
- $R_2$ represents a group: $-OCH_3$, $-OCH_2CH_3$, $-SCH_3$, $-SCH_2CH_3$ or $-OCHF_2$;
- $R_a$ represents a group: $-(C_1-C_5)$alkyl or $-CF_3$; and
- $R_b$ represents a group: $-(C_1-C_5)$alkyl or $-CF_3$;

in the following state: base or acid, or salts of acids or salts of bases; or in the form of a hydrate or a solvate.

4. A compound according to any one of claims 1 to 3, **characterised in that** it is selected from:

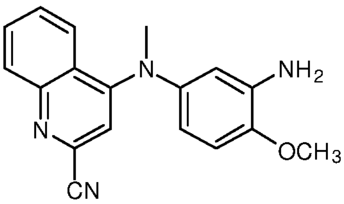

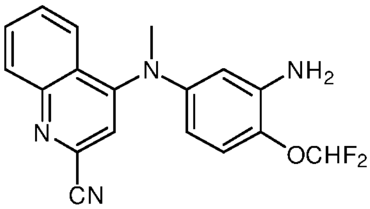

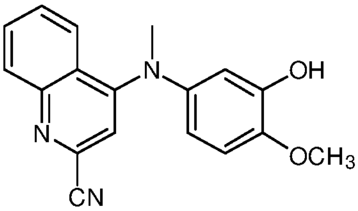

and

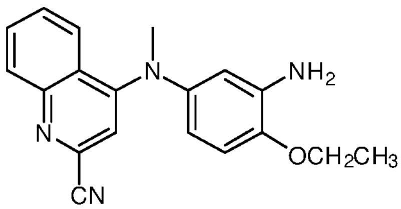

5. A compound according to claim 1, **characterised in that** it has the formula (Ib):

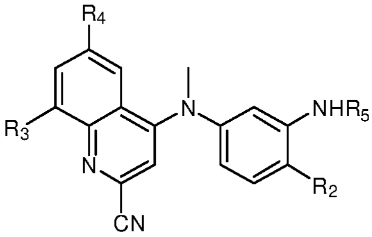

(Ib)

wherein:

- $R_5$ represents a hydrogen atom, a group: $-COR_a$, or $-COOR_b$;
- $R_2$ represents a group: $-OCH_3$, $-OCH_2CH_3$, $-SCH_3$, $-SCH_2CH_3$ or $-OCHF_2$;

- $R_3$ represents a hydrogen atom or a group: $-CH_3$, -CN, -F, -Cl or $-OR_c$, and preferably a hydrogen atom;
- $R_4$ represents a hydrogen atom or a group: $-CH_3$, -CN, -F, -Cl or $-OR_d$, and preferably a hydrogen atom;
- $R_a$ represents a group: $-(C_1-C_5)$alkyl or $-CF_3$;
- $R_b$ represents a group: $-(C_1-C_5)$alkyl or $-CF_3$;
- $R_c$ represents a group: $-(C_1-C_5)$alkyl or $-CF_3$; and
- $R_d$ represents a group: $-(C_1-C_5)$alkyl or $-CF_3$;

in the following state: base or acid, or salts of acids or salts of bases; or in the form of a hydrate or a solvate.

**6.** A compound according to any one of claims 1 to 3, **characterised in that** $R_1$ represents a group: $-(C_2-C_6)$alkenylene-CO-NH-OH or $-(C_2-C_6)$alkynylene-CO-NH-OH, and preferably $R_1$ represents a group: -CH=CH-CO-NH-OH, $-CH=CH-CH_2-CH_2-CO-NH-OH$ or $-C\equiv C-CH_2-CH_2-CO-NH-OH$.

**7.** A compound according to claim 1, **characterised in that** it has the formula (Ic):

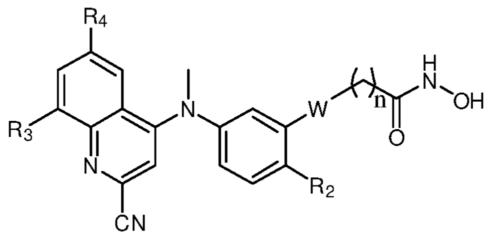

(Ic)

wherein:

- W represents -CH=CH- or -C=C-;
- n is 0, 1, 2, 3 or 4, and preferably n is 0 or 2;
- $R_2$ represents a group: $-OCH_3$, $-OCH_2CH_3$, $-SCH_3$, $-SCH_2CH_3$ or $-OCHF_2$;
- $R_3$ represents a hydrogen atom or a group: $-CH_3$, -CN, -F, -Cl or $-OR_c$, and preferably a hydrogen atom;
- $R_4$ represents a hydrogen atom or a group: $-CH_3$, -CN, -F, -Cl or $-OR_d$, and preferably a hydrogen atom;
- $R_c$ represents a group: $-(C_1-C_5)$alkyl or $-CF_3$; and
- $R_d$ represents a group: $-(C_1-C_5)$alkyl or $-CF_3$;

in the following state: base or acid, or salts of acids or salts of bases; or in the form of a hydrate or a solvate.

**8.** A compound according to claim 7, **characterised in that** it is selected from:

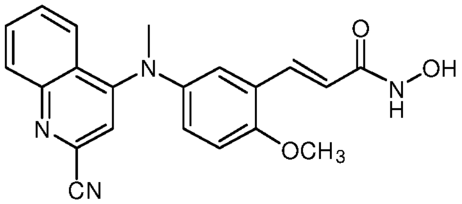

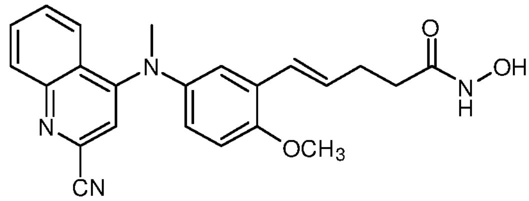

and

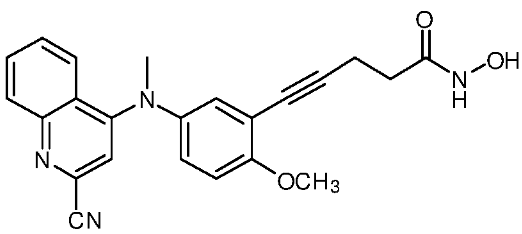

**9.** A method for preparing a compound according to claim 1, wherein $R_1$ represents an $-NH_2$ or -OH group, **charac-**

**terised in that**:

- a compound having the formula (B):

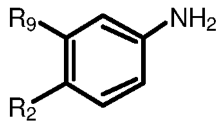

(B)

wherein $R_2$ is as defined according to claim 1and $R_9$ represents a $-NO_2$ or -O-Benzoyl group; is brought into contact with a compound having the formula (D):

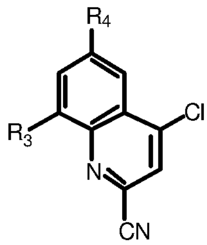

(D)

wherein $R_3$ and $R_4$ are as defined according to claim 1;
so as to form, by an aromatic nucleophilic substitution reaction, or by a coupling reaction in the presence of a catalyst, in particular based on palladium, the compound having the formula (E):

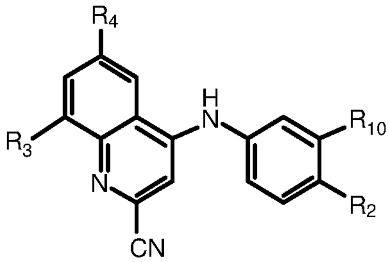

(E)

wherein $R_3$, $R_4$ and $R_2$ are as defined according to claim 1, and $R_{10}$ represents a $-NO_2$ or -O-Benzoyl group;

- the compound having the formula (E) is subjected to a methylation reaction, then to a reduction or deprotection reaction so as to form the compound having the formula (I):

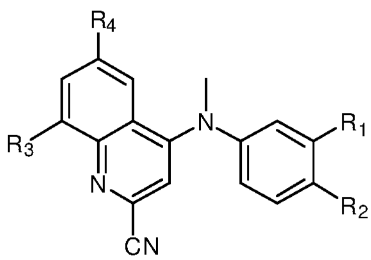

(I)

wherein $R_3$, $R_4$ and $R_2$ are as defined according to claim 1, and $R_1$ represents an $-NH_2$ or -OH group, with $R_a$ and $R_b$ being as defined according to claim 1.

**10.** A method for preparing a compound (Ic) according to claim 7, **characterised in that**:

- the compound having the formula (N):

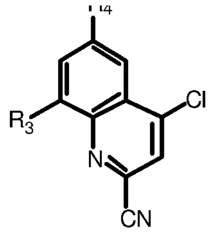

(N)

wherein $R_3$ and $R_4$ are as defined according to claim 7; is brought into contact with a compound having the formula (O):

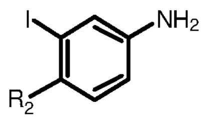

(O)

wherein $R_2$ is as defined according to claim 7, so as to form, by an aromatic nucleophilic substitution reaction, followed by a methylation reaction, the compound having the formula (P):

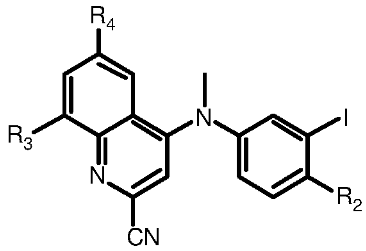

(P)

wherein $R_3$, $R_4$ and $R_2$ are as defined according to claim 7;

- the compound having the formula (P) is subjected to an organometallic coupling reaction with a group: -($C_2$-$C_6$)alkenylene-CO-NH-O-(2-tetrahydropyranyl) or -$C_2$-Cs-alkynylene-CO-NH-O-(2-tetrahydropyranyl), then to a deprotection reaction, so as to form the compound having the formula (Ic):

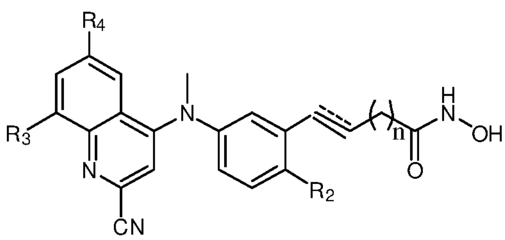

(Ic)

wherein,

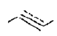

, n, $R_3$, $R_4$ and $R_2$ are as defined according to claim 7.

**11.** An intermediate compound, **characterised in that** it is selected from:

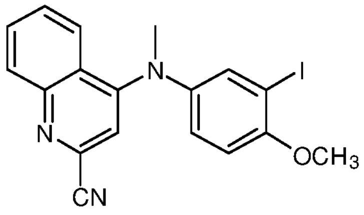

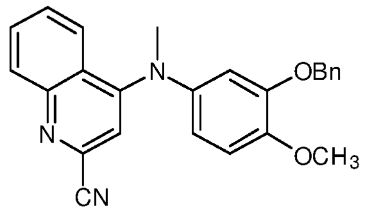

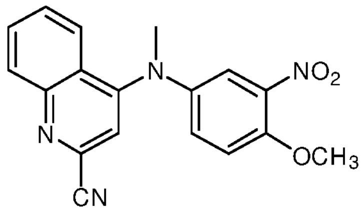

and

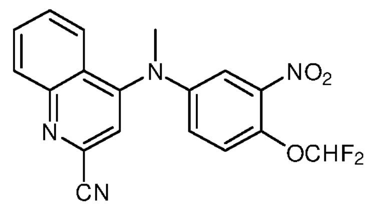

**12.** Use of the compounds according to claim 11, for the preparation of compounds having the formula (I) according to claim 1.

**13.** A compound having the formula (II):

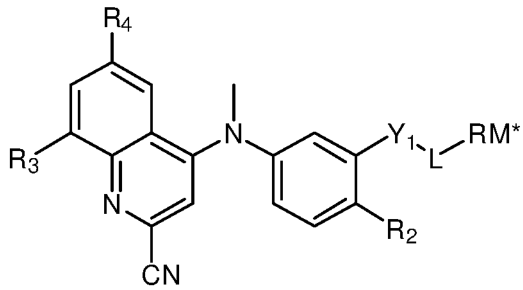

(II)

wherein:

- $Y_1$ represents -O-, -NH-, -NHCO-, -NHCOR$_a$-, -NHCOO-, -NHCOOR$_b$-,
- ($C_2$-$C_6$)alkenylene-CO-NH-O- or -($C_2$-$C_6$)alkenylene-CO-NH-O-;
- $R_2$ represents a group: $-OCH_3$, $-OCH_2CH_3$, $-SCH_3$, $-SCH_2CH_3$ or $-OCHF_2$;
- $R_3$ represents a hydrogen atom or a group: $-CH_3$, -CN, -F, -Cl or -OR$_c$; and preferably a hydrogen atom;
- $R_4$ represents a hydrogen atom or a group: $-CH_3$, -CN, -F, -Cl or -OR$_d$; and preferably a hydrogen atom;
- $R_a$ represents a group: -($C_1$-$C_5$)alkylene- or $-CF_2-$;
- $R_b$ represents a group: -($C_1$-$C_5$)alkylene- or $-CF_2-$;
- $R_c$ represents a group: -($C_1$-$C_5$)alkyl or $-CF_3$;
- $R_d$ represents a group: -($C_1$-$C_5$)alkyl or $-CF_3$;
- L represents a linking agent (linker);
- RM* is selected from RM and RM', wherein RM is a reactive functional group that is able to form a covalent bond with a targeting agent moiety, in particular with an antibody moiety or a functional fragment thereof, and wherein RM' is an RM moiety carrying at least one protecting group;

in the following state: base or salts of bases; or in the form of a hydrate or a solvate,
the L-RM* group being preferably selected from among:

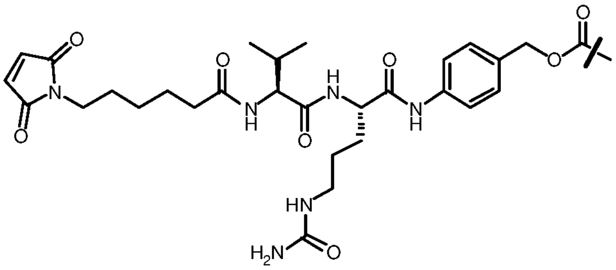

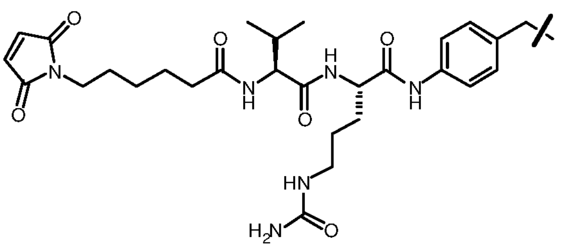

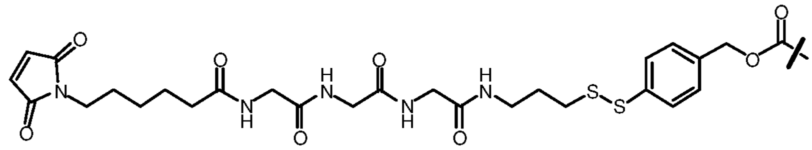

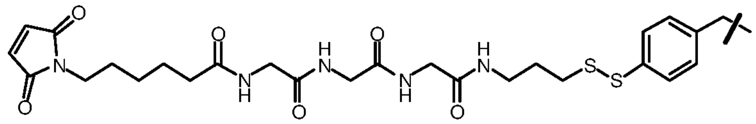

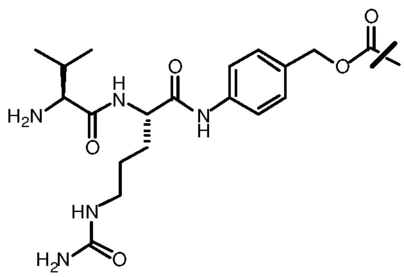

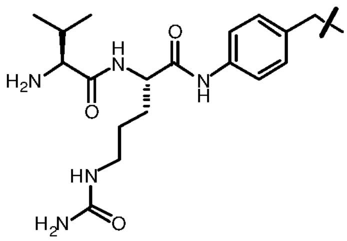

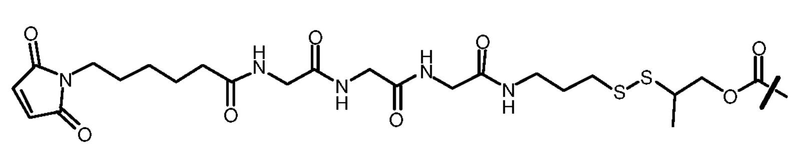

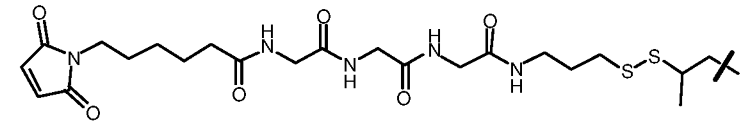

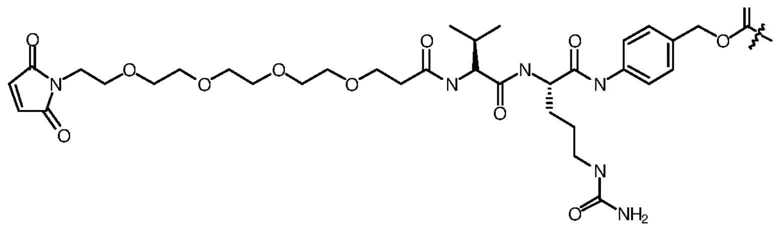

and

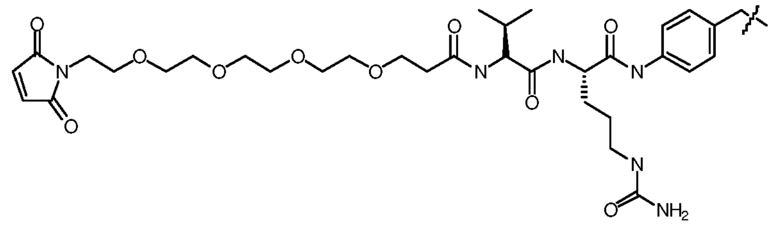

**14.** A compound according to claim 13, **characterised in that** it is selected from among:

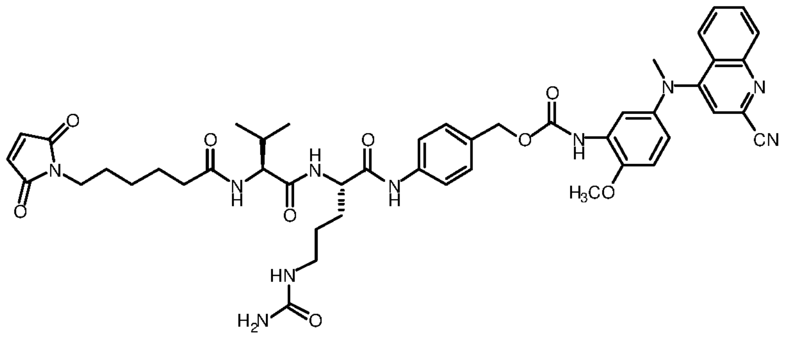

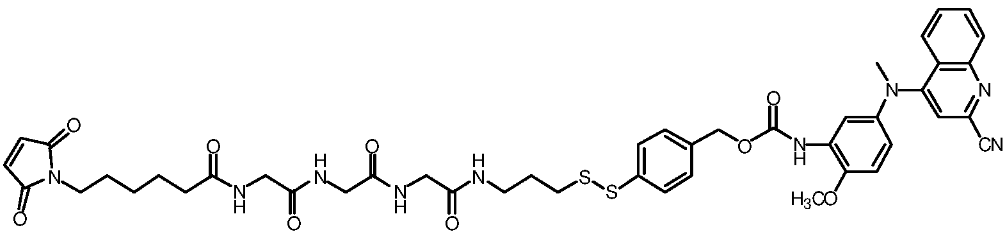

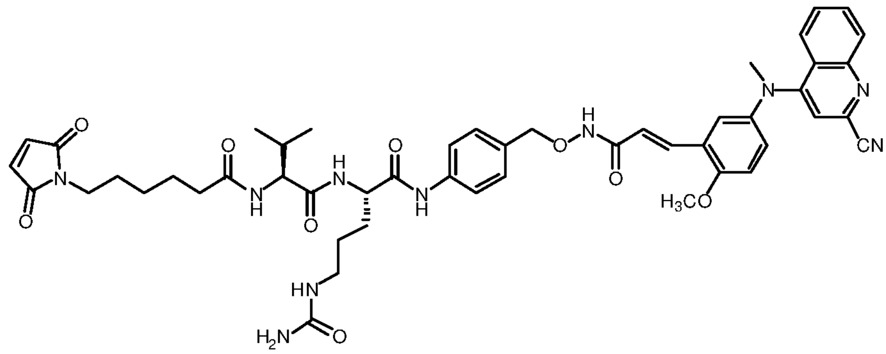

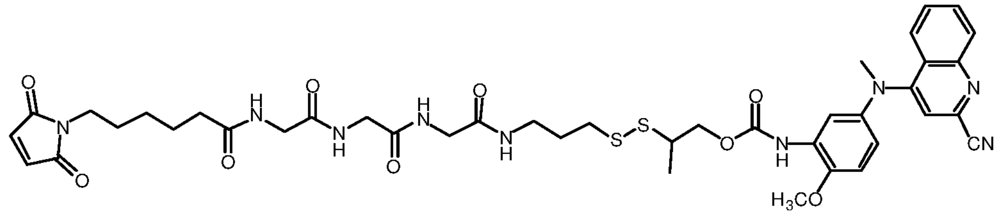

and

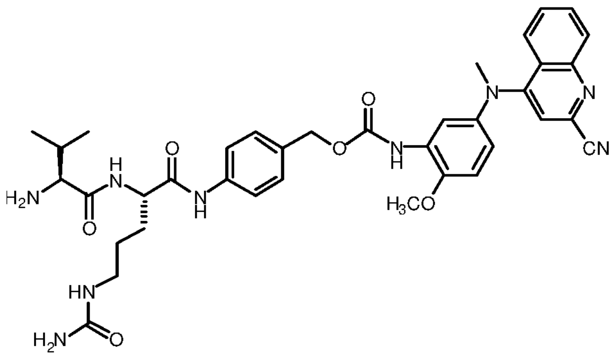

**15.** An antibody-drug conjugate having the formula (III):

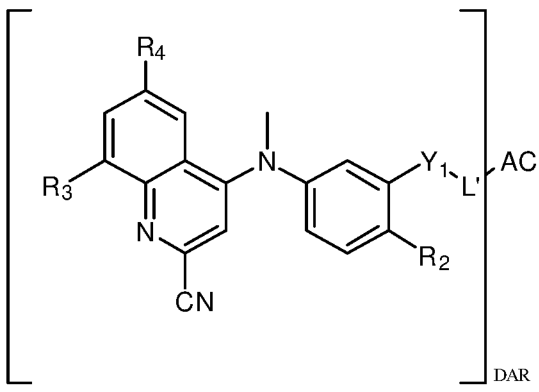

(III)

wherein:

- $Y_1$ represents -O-, -NH-, -NHCO-, $-NHCOR_a-$, -NHCOO-, $-NHCOOR_b-$,
- $(C_2-C_6)$alkenylene-CO-NH-O- or $-(C_2-C_6)$alkenylene-CO-NH-O-;
- $R_2$ represents a group: $-OCH_3$, $-OCH_2CH_3$, $-SCH_3$, $-SCH_2CH_3$ or $-OCHF_2$;
- $R_3$ represents a hydrogen atom or a group: $-CH_3$, -CN, -F, -Cl or $-OR_c$; and preferably a hydrogen atom;

- $R_4$ represents a hydrogen atom or a group: $-CH_3$, -CN, -F, -Cl or $-OR_d$; and preferably a hydrogen atom;
- $R_a$ represents a group: $-(C_1-C_5)$alkylene- or $-CF_2-$;
- $R_b$ represents a group: $-(C_1-C_5)$alkylene- or $-CF_2-$;
- $R_c$ represents a group: $-(C_1-C_5)$alkyl or $-CF_3$;
- $R_d$ represents a group: $-(C_1-C_5)$alkyl or $-CF_3$;
- L represents a linking agent (linker);
- AC represents a targeting agent moiety, in particular an antibody moiety or a functional fragment thereof; and

wherein the DAR (drug-to-antibody [targeting agent] ratio) varies between 1 and 8, and preferably between 2 and 4.

**16.** A method for preparing a compound having the formula (II) according to claim 13, **characterised in that** it includes a reaction step of reacting a compound having the formula (I) according to claim 1, with a compound having the formula X-L"-RM* wherein:

- X represents a group that is capable of reacting with an $R_1$ group as defined according to claim 1;
- L represents a linking agent (linker);
- RM* is selected from RM and RM', wherein RM is a reactive functional group that is able to form a covalent bond with a targeting agent moiety, in particular with an antibody moiety or a functional fragment thereof, and wherein RM' is an RM moiety carrying at least one protecting group;

wherein the reaction between the -R, moiety of the compound having the formula (I) and the compound having the formula X-L"-RM* results in the formation of a $-Y_1$-L-RM* moiety.

**17.** A preparation method according to claim 16, wherein RM* is RM, **characterised in that** it in addition includes a deprotection step of deprotecting an RM' moiety resulting in an RM group.

**18.** A preparation method for preparing a compound having the formula (III) according to claim 15, **characterised in that** it includes a reaction step of reacting a compound having the formula (II) according to claim 13 with a targeting agent moiety.

**19.** A pharmaceutical composition comprising an antibody-drug conjugate according to claim 15, in association with a pharmaceutically acceptable carrier.

**20.** An antibody-drug conjugate according to claim 15 or a pharmaceutical composition according to claim 19, for the use thereof as a medicinal product, and preferable for the use thereof for killing or inhibiting cell growth, or for the use thereof in the treatment of cancer.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1391213 A
- US 20140227180 A1
- US 20110256157 A1
- WO 2011001052 A1
- US 20120225089 A
- EP 1912677 B1
- WO 2014164534 A
- US 8535678 B2
- EP 2018058168 W
- US 2006074187 A


### Littérature non-brevet citée dans la description


- **MOOLTEN et al.** *J Natl Cancer Inst.,* 1972, vol. 49 (4), 1057-62
- **CHARI et al.** *Angew. Chimie. Int. Éd.,* 2014, vol. 53, 3796-3827
- **JACKSON.** *Org. Rés. processus Dev.,* 2016, vol. 20, 852-866
- **LEVY et al.** *Cancer Res.,* 1975, vol. 35 (5), 1182-6
- **LIU et al.** *Proc. 1996). Acad. nat. États-Unis d'Amérique,* vol. 93, 8618-8623
- **LODE et al.** *Cancer Res.,* 1998, vol. 58, 2925-2928
- **UPESLACIS et al.** *Cancer Res.,* 1993, vol. 53, 3336-3342
- **SANDERSON et al.** *Clin. Cancer Res.,* 2005, vol. 11, 843-52
- **GOVIDAN et al.** *Mol. Cancer. Ther.,* 2013, vol. 12, 968-78
- **GOLDENBERG et al.** *Clin. Cancer Res.,* 2007, vol. 13, 5556s-5563s
- **KUNG SUTHERLAND et al.** *Blood,* 2013, vol. 122, 1455-1463
- **CHARI et al.** *Mol. Cancer Ther.,* 2009, vol. 8, B126
- **VERMA et al.** *Bioorg. Méd. Chimie. Lett.,* 2015, vol. 25, 864-8
- **TOLCHER et al.** *J. Clin. Oncol.,* 2000, vol. 18, 4000
- **LAGUZZA et al.** *J. Med. Chimie.,* 1989, vol. 32, 548-555
- **UADIA P.** *Cancer Res.,* 1984, vol. 44, 4263-4266
- **CASI ; NERI.** *J. Control Release.,* 2012, vol. 161 (2), 422-8
- **WU ; SENTER.** *Nat. Biotechnologie,* 2005, vol. 23 (9), 1137-46
- **PETTIT GR.** *J Nat Prod,* 1987, vol. 50, 119-131
- **DARK et al.** *Cancer Res,* 1997, vol. 57, 1829-1834
- **GROSIOS K et al.** *Br J Cancer,* 1999, vol. 81, 1318-1327
- **SOUSSI MA et al.** *Chem. Med. Chem.,* 2015, vol. 10 (8), 1392-402
- **I. KHELIFI et al.** *European Journal of Médicinal Chemistry,* 2017, vol. 127, 1025-1034
- **TOKI et al.** *J. Org. Chimie.,* 2002, vol. 67, 1866-1872
- **BOLU et al.** *Mol. Pharmaceutics,* 2016, vol. 13, 1482-1490
- **R. NANI et al.** *Angew. Chim. Int. Ed. Engl.,* 2015, vol. 54 (46), 13635-13638
- **SHEN et al.** *J. Biol. Chem.,* 1985, vol. 260, 10905-10908
- **DUBOWCHIK et al.** *Bioconjug Chem.,* 2002, vol. 13, 855-69
- **JUN LU et al.** Linker shaving a crucial role in antibody-drug conjugates. *Int. J. Mol. Sci.,* 2016, vol. 17, 561
- **JESSICA R. MCCOMBS ; SHAWN C. OWEN.** Antibody drug conjugates : Design and sélection of linker, payload and conjugation chemistry. *The AAPS Journal,* Mars 2015, vol. 17 (2), 339
- **LAURENT DUCRY ; BERNHARD STRUMP.** Antibody-drug conjugates : linking cytotoxic payloads to monoclonal antibodies. *Bioconjugate Chem.,* 2010, vol. 21, 5-13
- **NARESHKUMAR JAIN et al.** Current ADS linker chemistry. *Pharm. Res.,* 2015, vol. 32, 3526-3540
- Therapeutic Antibodies and Protocols. Springer Science, 2009, vol. 525, 445
- **BADESCU et al.** *Bioconjugate Chem.,* 2014, vol. 25, 460-469
- **STILL et al.** *Can. J. Org. Chem.,* 1984, vol. 62, 586
- **KAZANE et al.** *Proc. Natl. Acad. Sci. U.S.A,* 2012, vol. 109, 3731-3736
- **BAN et al.** *J Am. Chem. Soc.,* 2010, vol. 13, 1523-5

- **ZHOU et al.** *J Am Chem Soc.,* 2013, vol. 135, 12994-7
- **ZEGLIS et al.** *Bioconjug Chem.,* 2013, vol. 24, 1057-67
- **L. CHEN et al.** *Eur. J. Med. Chem.,* 2017, vol. 138, 1114
- **SELON ALAMI et al.** *Eur. J. Med. Chem.,* 2019, vol. 168, 176-188
- **SELON ALAMI et al.** *J. Med. Chem.,* 2019, vol. 62, 1902-1916